(19) Europäisches Patentamt
European Patent Office
Office européen des brevets

(11) Veröffentlichungsnummer: **0 044 808**
**B1**

(12) # EUROPÄISCHE PATENTSCHRIFT

(45) Veröffentlichungstag der Patentschrift: **15.01.86**

(21) Anmeldenummer: **81810282.4**

(22) Anmeldetag: **13.07.81**

(51) Int. Cl.⁴: **A 01 N 47/36,** C 07 D 251/16, C 07 D 251/22, C 07 D 251/44, C 07 D 251/46, C 07 D 239/42, C 07 D 239/46, C 07 D 239/52 // C07C143/78, C07C147/06, C07C147/14, C07C149/443

(54) **N-Phenylsulfonyl-N'-pyrimidinyl- und -triazinylharnstoffe.**

(30) Priorität: **17.07.80 CH 5481/80**
**05.11.80 CH 8216/80**
**17.06.81 CH 3991/81**

(43) Veröffentlichungstag der Anmeldung: **27.01.82 Patentblatt 82/4**

(45) Bekanntmachung des Hinweises auf die Patenterteilung: **15.01.86 Patentblatt 86/3**

(84) Benannte Vertragsstaaten: **AT BE DE FR GB IT NL SE**

(56) Entgegenhaltungen:
**EP-A-0 001 514**
**EP-A-0 001 515**
**EP-A-0 007 687**
**EP-A-0 009 419**
**EP-A-0 023 422**
**EP-A-0 030 140**
**EP-A-0 044 210**

**Herbicides Vol. 1, p. 234-5**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Meyer, Willy, Talstrasse 49, CH- 4125 Riehen (CH)**
Erfinder: **Föry, Werner, Dr., Benkenstrasse 65, CH- 4054 Basel (CH)**

**EP 0 044 808 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

Die vorliegende Erfindung betrifft neue, herbizid wirksame und Pflanzenwuchsregulierende N-Phenylsulfonyl-N'-pyrimidinyl- und -triazinyl-harnstoffe, Verfahren zu ihrer Herstellung, sie als Wirkstoffe enthaltende Mittel, sowie deren Verwendung zum Bekämpfen von Unkräutern, vor allem selektiv in Nutz Pflanzenkulturen oder zum Regulieren und Hemmen des Pflanzenwachstums. Darüber hinaus betrifft die Erfindung auch als Zwischenprodukte hergestellte neue Phenylsulfonamide.

Die erfindungsgemässen N-Phenylsulfonyl-N'-pyrimidinyl- und triazinylharnstoffe entsprechen der allgemeinen Formel I

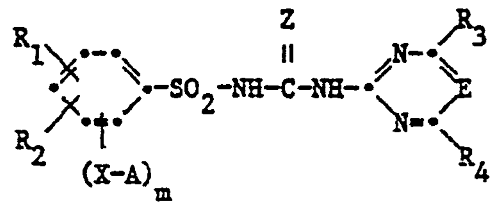

(I)

worin

A einen $C_1$-$C_6$-Halogenalkylrest,

E die Methingruppe oder Sticktoff,

X Sauerstoff, Schwefel, eine Sulfinyl- oder Sulfonylbrücke,

Z Sauerstoff oder Schwefel,

m die Zahl eins oder zwei,

$R_1$ Wasserstoff, Halogen, $C_1$-$C_5$-Alkyl, $C_2$-$C_5$-Alkenyl oder einen Rest -Y-$R_5$,

$R_2$ Wasserstoff, Halogen $C_1$-$C_5$-Alkyl, $C_2$-$C_5$-Halogen-Alkyl, oder einen Rest -Y-$R_5$, -$COOR_6$, -$NO_2$ oder -CO-$NR_7R_8$,

$R_3$ und $R_4$ unabhängig voneinander Wasserstoff, $C_1$-$C_4$ -Alkyl, $C_1$-$C_4$-Alkyl,$C_1$-$C_4$-Alkoxy, $C_1$-$C_4$-Halogenalkyl Halogen oder Alkoxyalkyl mit höchstens 4 Kohlenstoffatomen,

$R_5$ und $R_6$ je $C_1$-$C_5$-Alkyl, $C_2$-$C_5$-Alkenyl oder $C_2$-$C_6$-Alkinyl,

$R_7$ und $R_8$ unabhängig voneinander Wasserstoff, $C_1$-$C_5$-Alkyl, $C_2$-$C_5$-Alkenyl oder $C_2$-$C_6$-Alkinyl und

Y Sauerstoff, Schwefel, eine Sulfinyl- oder Sulfonylbrücke bedeuten, sowie den Salzen dieser Verbindungen, mit der Massgabe, dass m die Zahl zwei bedeutet, wenn gleichzeitig A für -$CHF_2$, -$CF_3$, -$CH_2$-$CF_3$, -$CF_2$-$CHF_2$, -$CF_2$-CHFCl, -$CF_2$-CHFBr oder -$CF_2$-CHF-$CF_3$; $R_4$ für -$OCH_3$, -$CH_3$, -$OC_2H_5$, -$CH_2$-O-$CH_3$ oder -$CH_2$-$CH_2$-$OCH_3$; $R_3$ für $CH_3$ oder $OCH_3$ stehen und der Phenylkern nicht oder in 2-, 3-, 5- oder 6-Stellung zur Sulfonylgruppe nur durch einen weiteren Substituenten aus der Gruppe F, Cl, Br oder -$CH_3$ substituiert ist, oder wenn gleichzeitig X Sauerstoff, A Trifluormethyl, $R_1$ Wasserstoff, $R_2$ $C_2$-$C_5$-Alkenyl in 2-Stellung, $R_3$ Wasserstoff, Chlor, Brom, Methyl, Aethyl, $C_1$-$C_3$-Alkoxy, Trifluormethyl, Methylthio oder Methoxymethyl und $R_4$ Methyl oder Methoxy bedeuten, oder wenn gleichzeitig X Sauerstoff, A Trifluormethyl, $R_1$ Wasserstoff, $R_2$ Chlor in 2-Stellung, $R_3$ Wasserstoff, Chlor, Brom, Methyl, Aethyl, Trifluormethyl, Methoxy, Methoxymethyl oder Methoxyäthyl bedeuten und der Trifluormethoxyrest die 5-Stellung am Phenylring einnimmt.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen Nr. 1514, 1515, dem US Patent Nr. 4 127 405, in der DT-OS 2 715 786 oder in der FR-PS 1 468 747 beschrieben. In den nicht vorveröffentlichten europäischen Patentpublikationen EP-A-23422, EP-A-30140 und EP-A-44210 werden den erfindungsgemässen Substanzen strukturähnliche Verbindungen gleicher Wirkungsrichtung offenbart.

In den Definitionen ist unter Alkyl geradkettiges oder verzweigtes Alkyl zu verstehen; z.B: Methyl, Aethyl, n-Propyl, i-Propyl, die vier isomeren Butyl, n-Amyl, i-Amyl, 2-Amyl, 3-Amyl, n-Hexyl oder i-Hexyl. Unter Alkoxy ist zu verstehen: Methoxy, Aethoxy, n-Propyloxy, i-Propyloxy und die vier isomeren Butyloxyreste, insbesondere aber Methoxy, Aethoxy oder i-Propyloxy.

Beispiele für Alkylthio sind Methylthio, Aethylthio, n-Propylthio, i-Propylthio und n-Butylthio, insbesondere aber Methylthio und Aethylthio.

Beispiele für Alkenylreste sind Vinyl Allyl Isopropenyl 1-Propenyl, 1-butenyl 2-butenyl 3-Butenyl 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl, die durch die Reste -X-A oder -Y-$R_5$ gebildet werden können, sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl, die durch die Reste -X-A oder -Y-$R_5$ gebildet werden können, sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen von $R_1$, $R_2$ sowie in Halogenalkyl oder Halogenalkoxy, Halogenalkylsulfinyl, Halogenalkylsulfonyl und Halogenalkylthio wie sie durch den Rest -X-A gebildet werden können, sind Fluor, Chlor und Brom, vorzugsweise jedoch Fluor und Chlor zu verstehen.

In der Definition von A ist unter einem halogen-substituierten Alkylrest ein solcher zu verstehen, der einen oder mehrere Halogenatome trägt. Vorzugsweise sind mehrere, gegebenenfalls sogar sämtliche Wasserstoffatome der Alkylreste durch Halogenatome ersetzt.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium,

Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomere Butylamine, Dimethyl, Diäthanolamin Dipropylamin, Diisopropylamin, Di-n-butylamin, Pyrrolidin Piperidin Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter den Verbindungen der Formel 1 sind diejenigen beworzugt, in denen

a) Z Sauerstoff bedeutet

b) die Reste $R_3$ und $R_4$ zusammen nicht mehr als 4 Kohlenstoffatome enthalten.

Die Gruppe a) kann aufgeteilt werden in zwei weitere Untergruppen, die aus Verbindungen bestehen, in denen

aa) m die Zahl eins und

ab) m die Zahl zwei bedeutet.

Aus der Gruppe aa) sind die Verbindungen bevorzugt, in denen der Rest -X-A in der 2- oder 3-Position zum Sulfonylrest steht. Hierunter geniessen eine weitere Bevorzugung die Verbindungen, in denen der Rest -X-A in der 2-Position steht.

Aus der Gruppe ab) bilden die Verbindungen eine bevorzugte Gruppe, in denen die beiden Reste -X-A in 2- und 5-Stellung zur Sulfonylgruppe stehen.

Eine weitere Bevorzugung von Verbindungen der oben aufgelisteten Untergruppen der Gruppen aa) und ab) besteht darin, dass die Reste $R_3$ und $R_4$ zusammen höchstens 4 Kohlenstoffatome enthalten. Es bestehen somit die besonders bevorzugten Gruppen von Verbindungen darin, dass in Verbindungen der Formel 1

aab) nur ein Rest -X-A im der 2-Stellung zum Sulfonylrest vorhanden ist und die Reste $R_3$ und $R_4$ zusammen nicht mehr als 4 Kohlenstoffatome enthalten und

abb) zwei Reste -X-A in 2- und 5-Stellung zum Sulfonylrest vorhanden sind und die Reste $R_3$ und $R_4$ zusammen nicht mehr als 4 Kohlenstoffatome enthalten.

Aus der Gruppe abb) geniessen eine weitere Bevorzugung die Verbindungen, in denen A für $-CHF_2$, $-CF_3$ oder $-CH_2-CH_2Cl$ steht.

Aus der Gruppe aab) sind die Verbindungen bevorzugt, in denen $R_1$ Wasserstoff bedeutet, und der Rest $R_2$ in der 5- oder 6-Stellung zur Sulfonylgruppe steht.

Von diesen Verbindungen sind wiederum die bevorzugt, in denen R für Wasserstoff, Fluor, Nitro oder $COOR_6$ steht.

Eine weitere Bevorzugung innerhalb der letztgenannten Gruppe geniessen die Verbindungen in denen X für Sauerstoff oder Schwefel steht.

Ganz besonders bevorzugt sind hiervon wiederum jene, in denen der Substituent $R_2$ Wasserstoff bedeutet,$R_3$ für Chlor, Fluor, Methyl, Chlormethyl, Aethoxy, i-Propyloxy, Aethyl oder Methoxy und $R_4$ für Methyl, Aethyl, Methoxy oder Aethoxy stehen.

Zwei weiterhin aus der letzten Gruppe bevorzugten Untergruppen sind Gruppen von Verbindungen, in denen

α) das Brückenglied X Sauerstoff ist und

β) der Rest A durch $-CF_2G$ verkörpert ist, worin G für Wasserstoff, F, Cl, Br, $CF_3$ oder $CH_3$ steht.

Innerhalb der Gruppe β) sind die Verbindungen herworzuheben, in denen G für Cl, Br oder $CF_3$ steht.

Eine gleiche bevorzugte Stellung wie die in den Gruppen α) und β) nehmen die Verbindungen ein, in denen der Rest A für $-CH_2F$, $-CH_2-CH_2Cl$ oder $-CH_2-CH_2F$ steht.

Von den Verbindungen der Formel 1, in denen Z für Schwefel steht, sind die jenigen bevorzugt, in denen X Sauerstoff oder Schwefel und $R_3$ umd $R_4$ jeweils unabhängig voneinander $C_1$-$C_3$-Alkyl, $C_1$-$C_3$-Alkoxy oder $C_1$-$C_3$-Alkylthio mit zusammen höchstens 4 Kohlenstoffatomen bedeuten umd A für $-CHF_2$, $-CF_3$, $-C_2F_5$ und $-CF_2-CHF_2$ steht, und der Rest -X-A die 2-Stellung besetzt und m die Zahl 1 bedeutet.

Als bevorzugte Einzelverbindungen der vorliegenden Erfindung sind zu nennen:

N-(2-Difluormethoxyphenyl-sulfonyl)-N'-(4-äthyl-6-methoxy-1, 3, 5-triazin-2-yl)-harmstoff,
N-(2=Difluormethoxyphenyl-sulfonyl)-N'-(4, 6-diäthoxy-1, 3, 5-triazin-2-yl)-harnstoff,
N-[2-Difluormethoxyphenyl-sulfonyl)-N'-(4-chlor-6-methyl-pyrimidin-2-yl)-harnstoff,
N-(2-Pentafluoräthoxyphenyl-sulfonyl)-N'-(4-methoxy-6-methyl-1, 3, 5-triazin-2-yl)-harnstoff,
N-(2-Difluormethylthiophenyl-sulfonyl)-N'-(4-chlor-6-methoxy-pyrimidin-2-yl)-harmstoff,
N-[2, 5-Bis-(difluormethoxy)-phenyl-sulfonyl-N'-(4-methoxy-6-methyl-1, 3, 5-triazin-2-yl)-harnstoff,
N-(2-Difluormethoxyphenyl-sulfonyl)-N'-(4-methoxy-6-isopropyloxy-1, 3, 5-triazin-2-yl)-harnstoff,
N-(2-Difluormethylthiophenyl-sulfonyl)-N'-(4-äthyl-6-nethoxy-1, 3, 5-triazin-2-yl)-harnstoff und
N-(2-Difluormethoxyphenyl-sulfonyl)-N'-(4-chlor-6-nethoxy-pyrimidin-2-yl)-harnstoff.

Die Herstellung der Verbindungen der Formel I erfolgt einem inerten, organischen Lösungsmittel.

Nach einem ersten Verfahren werden die Verbindungen der Formel I erhalten, indem man ein Phenylsulfonamid der Formel II

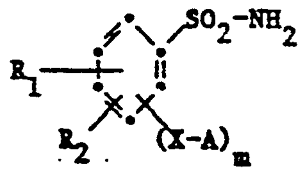

(II) ,

worin A, $R_1$, $R_2$ X und m die unter Formel 1 gegebene Bedeutung haben, in Gegenwart einer Base mit einem N-Pyrimidinyl- oder Triazinyl-carbamat der Formel III

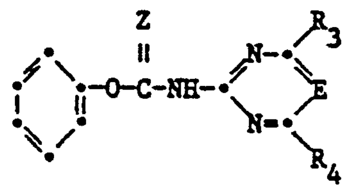

(III) ,

worin E, $R_3$, $R_4$ und Z die unter Formel 1 gegebene Bedeutung haben, umsetzt.

Nach einem zweiten Verfahren gelangt man zu Verbindungen der Formel I, indem man ein Phenylsulfonylisocyanat oder -isothiocyanat der Formel IV

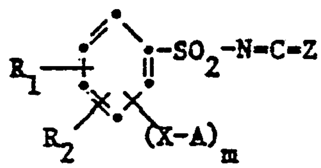

(IV) ,

worin

A, $R_1$, $R_2$, m, X und Z die unter Formel I gegebene Bedeutung haben, gegebenenfalls in Gegenwart einer Base, mit einem Amin der Formel V

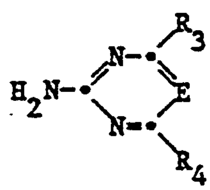

(V) ,

worin E, $R_3$ und $R_4$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

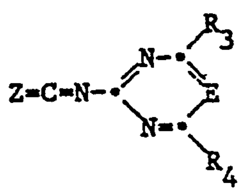

(VI) ,

worin

E, $R_3$, $R_4$ und Z die unter Formel I gegebene Bedeutung haben, umsetzt.

Schliesslich kann man die Verbindungen der Formel I auch erhalten, indem man ein N-Phenylsulfonylcarbamat der Formel VII

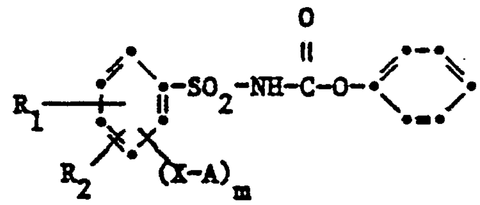 (VII) ,

worin A, $R_1$, $R_2$, m und X die unter Formel I gegebene Bedeutung haben, mit einem Amin der oben angegebene Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Salze übergeführt werden. Dieses geschieht beispielsweise durch Umsetzen mit der äquimolaren Menge Base und Verdampfen des Lösungsmittels.

Die Ausgangsstoffe der Formeln II, IV umd VII sind zum Teil neu und können nach folgenden Methoden hergestellt werden:

Die als Zwischenprodukte verwendeten Sulfonamide der Formel II werden aus den entsprechenden Amilinen durch Diazotierung und Austausch der Diazogruppe mit Schwefeldioxid in Gegenwart eines Katalysators wie Kupfer-1-chlorid in Salzsäure oder Essigsäure und Umsetzen des entstandenen Phenylsulfonylchlorids mit Ammoniumhydroxid-Lösung erhalten.

Die neuen Sulfonamide der Formel II sind speziell für die Synthese der erfindungsgemässen Verbindungen der Formel I entwickelt worden und bilden einen weiteren Aspekt der vorliegenden Erfindung.

Die Phenylsulfonylisocyanate der Formel IV können durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Aehnliche Darstellungen sind in "Newer Methods of Preparative Organic Chemistry" Band VI, 223-241, Academic Press New York und London, beschrieben.

Die Isothiocyanate der Formel IV werden durch Behandlung der Sulfonamide der Formel II mit Schwefelkohlenstoff und Kaliumhydroxyd und anschliessender Unsetzung des Dikaliunsalzes mit Phosgen erhalten. Solche Verfahren sind in Arch. Pharm. 299, 174 (1966) beschrieben.

Die N-Phenylsulfonylcarbamate der Formel VII werden durch Unsetzung der Sulfonamide der Formel II mit Diphenylcarbamat in Gegenwart einerBase erhalten. Aehnliche Verfahren sind in der japanischen Patentschrift 61 169 erwähnt.

Die Ausgangsmaterialien der Formeln III, V und VI sind bekannt oder können nach bekannten Methoden hergestellt werden.

Durch Umsetzung von Aminen der Formel V Oxalylchlorid in chlorierten Kohlenwasserstoffen als Lösungsmittel, lassen sich Isocyanate der Formel VI herstellen. Amine der Formel V sind bekannt und zum Teil im Handel erhältlich oder sie können nach bekannten Methoden hergestellt werden, siehe "The Chemistry of Heterocyclic Compounds" Band XIV, Interscience Publishers New York, London.

Die Umsetzungen zu den Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt.

Die Reaktionszeiten können ebenfalls durch Zugabe einiger Tropfen Base als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglicher Massnahmen.

Die Verbindungen der Formel I haben starke pflanzenwuchsregulierende, insbesondere pflanzenwuchshemmende Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Mais, Baumwolle, Soja und Reis, befähigen. Es werden dabei

teilweise auch Unkräuter geschädigt, welchen bisher nur mit Totalherbiziden beizukommen war.

Die Wirkungsart dieser Wirkstoffe ist unüblich. Sie sind in vielen Fällen translozierbar, d.h. sie werden von der Pflanze aufgenommen und an andere Stellen transportiert, wo sie dann zur Wirkung kommen. Das unübliche daran ist, dass die Wirkstoffe nicht nur den Weg durch die Gefässbündel im Holzteil, von den Wurzeln in die Blätter nehmen, sondern auch durch die Siebröhren im Bastteil von den Blättern zurück in die Wurzel transloziert werden können. So gelingt es beispielsweise durch Oberflächenbehandlung perenierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Erfindung betrifft auch herbizide und pflanzenwachstumsregulierende Mittel, welche einen neuen Wirkstoff der Formel I erhalten, sowie Verfahren zur pre- und post-emergenten Unkrautbekämpfung und zur Henmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokusnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekönnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder-Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen umd einen Fettsäurerest mit 8 - 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phospate wie z.B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der

Alkylkette. Die genannten Verbindungen enthalten üblicherweise pro Propylenglykoleinheit 1 bis 5 Aethylenglykoleinheiten.

Als Beispiele nichtionischer Tenside seien Nonylphenolpolyäthoxyäthanole, Ricinusölpolyglykoläther, Polyproylen-Polyäthylenoxidaddukte, Tributylphenoxypolyhoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkyl-rest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammonium-bromid.

Die in der Formulierungstechnik gebräuchlichen Tenside sind u.a. in folgenden Publikationen beschrieben:

"Mr Cutcheon's Detergents and Emulsifiers Annual" MC Publishing Corp., Ringwood, New Jersey, 1979.
Sisely and Wood, "Encyclopedia of Surface Active Agents" Chemical Publishing Co., Inc. New York, 1964.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

**Lösungen**

Aktiver Wirkstoff: 1 bis 30 %, vorzugsweise 5 bis 20 %
Lösungsmittel: 99 bis 0 %, vorzugsweise 95 bis 0
oberflächenaktives Mittel: 0 bis 99 %, vorzugsweise 0 bis 95 %.

**Emulgierbare Konzentrate**

Aktiver Wirkstoff: 1 bis 20 %, bevorzugt 5 bis 10 %
oberflächenaktives Mittel: 5 bis 30 %, vorzugsweise 10 bis 20 %
flüssiges Trägermittel: 50 bis 94 %, vorzugsweise 70 bis 85 %.

**Stäube**

Aktiver Wirkstoff: 0,1 bis 10 %, vorzugsweise 0,1 bis 1 %
festes Trägermittel: 99,9 bis 90 %, vorzugsweise 99,9 bis 99%

**Suspension-Konzentrate**

Aktiver Wirkstoff: 5 bis 75 %, vorzugsweise 10 bis 50 %
Wasser: 94 bis 25 %, vorzugsweise 90 bis 30 %
oberflächenaktives Mittel: 1 bis 40 %, vorzugsweise 2 bis 30 %.

**Benetzbare Pulver**

Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %.

**Granulate**

Aktiver Wirkstoff: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

**Herstellungsbeispiele**

**Beispiel 1**

a) 2-Difluormethoxyphenyl-sulfonylchlorid.

Zu einer Lösung von 6,4 g Kupfersulfat in 276 ml 36%iger Salzsäure umd 72 ml Wasser lässt man unter Kühlung bei 0° 101,2 g einer 40%igen Natriumhydrogensulfit-Lösung zutropfen. Zu der erhaltenen Lösung lässt mam gleichzeitig 101,2 g einer 40%igen Natriumhydrogensulfit-Lösung und eine auf -10° gekühlte Diazoniunsalz-Lösung, die man durch Zutropfenlassen unter Kühlung von 28,8 g Natriumnitrit in 44 ml Wasser zu einer Lösung von 39 ml Wasser, 84 ml 36%iger Salzsäure und 63,7 g 2-Difluormethoxy-anilin erhält, zutropfen. Dabei erwärmt sich das Reaktionsgemisch unter gleichzeitiger Gasentwicklung auf 30°. Nach Rühren des Reaktionsgemisches für 18 Stunden scheidet sich ein braunes Oel ab. Nach Abtrennen der organischen Phase extrahiert man die wässrige Phase mit Methylenchlorid. Durch Vereinigen und Eindampfen der organischen Phasen erhält man als Rohprodukt 80,3 g 2-Difluormethoxy-phenyl-sulfonylchlorid als braunes Oel.

b) 2-Difluormethoxyphenyl-sulfonamid.

Einer Mischung aus 250 ml Methylenchlorid, 250 ml Wasser und 250 ml 30%iger Ammoniak-Lösung fügt man langsam eine Lösung von 80,3 g rohem 2-Difluormethoxyphenyl-sulfonyl-chlorid in 50 ml Methylenchlorid zu. Nach Abklingen der exothermen Reaktion wird die Reaktionsmischung für 2 Stunden am Rückfluss gekocht. Nach Waschen der organischen Phase mit Wasser, Trocknen über Natriumsulfat, Eindampfen und Umkristallisieren aus Methanol erhält man 40,5 g 2-Difluormethoxyphenyl-sulfon-amid vom Schmelzpunkt 128-130°.

**Beispiel 2**

a) 2-Pentafluoräthoxy-sulfonylchlorid.

Zu einer Lösung von 6,4 g Kupfersulfat in 276 ml 36%iger Salzsäure und 72 ml Wasser lässt man unter Kühlung bei 0° 101,2 g einer 40%igen Natriumdhydrogensulfit-Lösung zutropfen. Zu der erhaltenen Lösung lässt man gleichzeitig 101,2 g einer 40%igen Natriumhydrogensulfit-Lösung und eine auf -10° gekühlte Diazoniumsalz-Lösung, die man durch Zutropfenlassen unter Kühlung von 28,8 g Natriumnitrit in 44 ml Wasser zu einer Lösung von 39 ml Wasser, 84 ml 36%iger Salzsäure und 90,S g 2-Pentafluoräthoxy-anilin erhält, zutropfen. Dabei erwärmt sich das Reaktionsgemisch unter gleichzeitiger Gasentwicklung auf 30°. Nach Rühren des Reaktionsgemisches für 18 Stunden scheidet sich ein braunes Oel ab. Nach Abtrennen der organischen Phase extrahiert man die wässrige Phase mit Methylenchlorid. Durch Vereinigen und Eindampfen der organischen Phasen erhält man als Rohprodukt 2-Pentafluoräthoxy-Phenyl-sulfonylchlorid als braunes Oel.

b) 2-Pentafluoräthoxyphenyl-sulfonamid.

Einer Mischung aus 250 ml Methylenchlorid, 250 ml Wasser und 250 ml 30%iger Ammoniak-Lösung fügt man eine Lösung des im Beispiel 2a erhaltenen rohen 2-Pentafluoräthoxyphenyl-sulfonyl-chlorids in 50 ml Methylenchlorid zu. Nach Abklingen der exothermen Reaktion wird die Reaktionsmischung für 2 Stunden am Rückfluss gekocht. Nach Waschen der organischen Phase mit Wasser, Trocknen über Natriumsulfat, Eindampfen und Umkristallisieren aus Methanol erhält man 65,0 g 2-Pentafluor-äthoxyphenyl-sulfonamid vom Schmelzpunkt 145-146°.

In analoger Weise werden die folgenden neuen, speziell für die Synthese der Verbindungen I entwickelten Zwischenprodukte hergestellt.

Tabelle 1

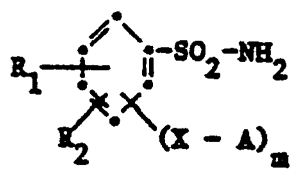

| $R_1$ | $R_2$ | X | A | m | Stellung von X-A | physikalische Daten |
|---|---|---|---|---|---|---|
| H | H | O | $-C_2F_5$ | 1 | 2 | Smp. 145-146° |
| $6-COOCH_2CH_3$ | H | O | $-CHF_2$ | 1 | 2 | |
| $6-COOCH_2CH_3$ | H | S | $-CHF_2$ | 1 | 2 | |
| $6-COOCH_3$ | H | SO | $-CHF_2$ | 1 | 2 | |
| $6-COOCH_2CH_3$ | H | $SO_2$ | $-CHF_2$ | 1 | 2 | |
| 6-Cl | 5-Cl | O | $-CHF_2$ | 1 | 2 | |
| 6-Cl | 5-Cl | S | $-CHF_2$ | 1 | 2 | |
| 6-Cl | 5-Cl | SO | $-CHF_2$ | 1 | 2 | |
| 6-Cl | 5-Cl | $SO_2$ | $-CHF_2$ | 1 | 2 | |
| H | $5-CF_3$ | O | $-CHF_2$ | 1 | 2 | |
| H | $5-CF_3$ | S | $-CHF_2$ | 1 | 2 | |
| H | $5-CF_3$ | SO | $-CHF_2$ | 1 | 2 | |
| H | $5-CF_3$ | $SO_2$ | $-CHF_2$ | 1 | 2 | |
| H | $6-NO_2$ | O | $-CHF_2$ | 1 | 3 | |
| $6-COOCH_2CH_3$ | H | O | $-CF_3$ | 1 | 2 | |
| $6-COOCH_2CH_3$ | H | S | $-CF_3$ | 1 | 2 | |
| $6-COOCH_2CH_3$ | H | SO | $-CF_3$ | 1 | 2 | |
| $6-COOCH_2CH_3$ | H | $SO_2$ | $-CF_3$ | 1 | 2 | |

**Tabelle 1** fortsetzung

| $R_1$ | $R_2$ | X | A | m | Stellung von X-A |
|---|---|---|---|---|---|
| H | 6-$OCH_3$ | O | $CH_2F$ | 1 | 2 |
| 5-$SCH_3$ | H | O | $CH_2F$ | 1 | 2 |
| 5-$NO_2$ | H | O | $CH_2F$ | 1 | 2 |
| 5-$SO_2CH_3$ | H | O | $CH_2F$ | 1 | 2 |
| H | H | S | $C_2F_5$ | 1 | 2 |
| H | H | SO | $C_2F_5$ | 1 | 2 |
| H | H | $SO_2$ | $C_2F_5$ | 1 | 2 |
| H | 5-F | O | $CH_2F$ | 1 | 2 |
| 6-F | H | O | $C_2F_5$ | 1 | 2 |
| H | 5-F | O | $C_2F_5$ | 1 | 2 |
| H | 6-$COOCH_2CH_3$ | O | $CF_3$ | 1 | 3 |
| H | 6-$COOCH_2CH_3$ | S | $CF_3$ | 1 | 3 |
| H | 6-$COOCH_2CH_3$ | SO | $CF_3$ | 1 | 3 |
| H | 6-$COOCH_2CH_3$ | $SO_2$ | $CF_3$ | 1 | 3 |
| H | H | O | $-CF_2-CF_2Cl$ | 1 | 2 |
| H | H | O | $-CF_2-CF_2Br$ | 1 | 2 |
| H | H | O | $-CH_2-CH_2F$ | 1 | 2 |
| H | H | S | $-CH_2-CH_2F$ | 1 | 2 |
| H | H | S | $-CF_2-CF_2Cl$ | 1 | 2 |
| H | H | O | $-CH_2-CCl_3$ | 1 | 2 |
| H | H | O | $-CF_2-CH_3$ | 1 | 2 |
| H | H | S | $-CH_2-CCl_3$ | 1 | 2 |
| H | H | O | $-CF_2Cl$ | 1 | 2 |

| $R_1$ | $R_2$ | X | A | m | Stellung von. X-A | physikalische Daten |
|---|---|---|---|---|---|---|
| H | H | SO | $-CF_2Cl$ | 1 | 2 | |
| H | H | $SO_2$ | $-CF_2Cl$ | 1 | 2 | |
| H | H | O | $-CH_2-CH_2Cl$ | 1 | 2 | Smp.110° |
| H | H | S | $-CH_2-CH_2Cl$ | 1 | 2 | |
| $6-COOCH_3$ | H | O | $CHF_2$ | 1 | 2 | |
| $6-NO_2$ | H | O | $CHF_2$ | 1 | 2 | |

**Beispiel 3:**

a) N-(2-Difluormethoxyphenyl-sulfonyl)-phenyl-carbamat.

Zu einer auf 0-10° gekühlten Suspension von 0,84 g 57,5%iger Natriumhydrid-Dispersion in 5 ml absolutem Dimethylformamid lässt man eine Lösung von 4,5 g 2-Difluormethoxy-phenyl-sulfonamid in 5 ml absolutem Dimethylformamid zutropfen. Zur Vervollständigung der Wasserstoffentwicklung erwärmt man die Reaktionsmischung unter Rühren auf 25°. Nach beendeter Wasserstoffentwicklung und Abkühlen der Mischung auf -30° wird eine Lösung von 4,7 g Diphenylcarbonat in 5 ml absolutem Dinethylformamid zugesetzt und danach die Reaktionsmischung unter Erwärmenlassen bis auf 25° für 18 Stunden gerührt. Nach Eingiessen der Reaktionslösung in ein Gemisch aus 11 ml 2 N Salzsäure und 100 g Eis nimmt man das ausgefallene Produkt mit Aethylacetat auf. Durch Trocknen und Eindampfen der Aethylacetat-Phase umd Umkristallisieren aus Methanol erhält man 5,6 g N-(2-Difluormethoxyphenyl-sulfonyl)-phenyl-carbamat als farblose Kristalle vom Schmelzpunkt 115-117°.

b) N-(2-Difluormethoxy,phenyl-sulfonyl)-N'-(4-äthyl-6-methoxy-1, 3, 5-triazin-2-yl)-harnstoff.

Eine für 5 Stunden am Rückfluss gekochte Lösung von 5,4 g N-(2-Difluor-methoxyphenyl-sulfonyl)-phenyl-carbamat und 2,42 g 2-Amino-4-äthyl-6-methoxy-1, 3, 5-triazin in 60 ml absolutem Dioxan wird in 600 ml Eiswasser gegossen. Das ausgefallene Produkt wird mit Aetylacetat aufgenommen. Das nach Waschen mit Wasser, Trocknen und Eindampfen der Aetylacetat-Phase erhaltene Oel wird erneut in Aetylacetat gelöst und fünfmal mit gesättigter Natriumhydrogencarbonat-Lösung gewaschen. Die vereinigten wässrigen Phasen werden mit verdünnter Salzsäure angesäuert und mit Aetylacetat extrahiert. Nach Trocknen und Eindampfen der Aetylacetat-Phase und Umkristallisieren aus Methanol erhält man 5,1 g N-(Diflourmethoxyphenyl-sulfonyl)-N'-(4-äthyl-6-methoxy-1, 3, 5-triazin-2-yl)-harnstoff vom Schmelzpunkt 114-116° +.

**Beispiel 4:**

a) N-(Difluormethoxyphenyl-sulfonyl)-N-(4, 6-dichlor-1, 3, 5-triazin-2-yl)-harnstoff.

Einer Lösung von 3,9 g 4, 6-Dichlor-2-isocynato-1, 3, 5-triazin in 50 ml absolutem Dioxan werden 4,5 g 2-Difluormethoxyphenyl-sulfonamid zugesetzt. Nach 5 Stunden Kochen am Rückfluss wird die entstandene klare Lösung in 500ml Eiswasser gegossen. Das ausgefallene Produkt wird mit Aetylacetat extrahiert. Durch Trocknen und Einengen der organischen Phase erhält man als Kristallisat vom Schmelzpunkt 105-107° 8,5 g eines 1:1-Adduktes von Dioxan und N-(2-Difluormethoxyphenyl-sulfonyl )-N-(4, 6-dichlor-1, 3, 5-triazin-2-yl)-harnstoff.

b) N-(2-Difluormethoxyphenyl-sulfpnyl)-N'-(4, 6-äthoxy-1, 3, 5-triazin-2-yl)-harnstoff.

Zur Lösung von 5,5 g des nach Beispiel 5a erhaltenen N-(2-Difluor-methoxyphenyl-sulfonyl)-N'-(4, 6-dichlor-1, 3, 5-triazin-2-yl)-harnstoff-Dioxan-Adduktes in 10 ml absolutem Aethanol lässt man innerhalb von 15 Minuten eine Lösung von 1,9 g Natriumäthylat in 10 ml absolutem Aethanol zutropfen. Nach 2 Stunden Kochen am Rückfluss wird die Reaktionsmischung eingedampft und der Rückstand in 200 ml Eiswasser gelöst. Beim Ansäuren der Lösung mit verdünnter Salzsäure fallen 3,2 g N-(2-Difluormethoxyphenyl-sulfonyl)-N -(4,6-diäthoxy-1, 3, 5-triazin 2-yl)-harnstoff als farblose Kristalle vom Schmelzpunkt 117-118° an.

**Beispiel 5:**

a) N-(2-Pentafluorathoxyphenyl-sulfonyl)-N'-methyl-harnstoff.

Das nach Beispiel 2b erhaltene 2-Pentafluoräthoxyphenyl-sulfonamid wird in 400 ml Methylenchlorid suspendiert. Dann werden 16,3 g Methylisocyanat zugegeben umd anschliessend während 15 min 28,2 g Triäthylamin zutropfen gelassen. Es entsteht eine klare Lösung. Durch vollständiges Eindampfen, Lösen des Rückstandes in 5%iger Sodalösung und Wiederansäuern mit 10%iger Salzsäure erhält man 74,6 g N-(2-Pentafluoräthoxyphenyl-sulfonyl)-N'-methyl-harnstoff vom Schmelzpunkt 177-179°.

b) 2-Pentafluoräthoxyphenyl-sulfonylisocyanat.

74,6 g N-(-2-Pentafluoräthoxyphenyl-sulfonyl)-N'-methyl-harnstoff werden in 1300 ml Chlorbenzol suspendiert umd durch azeotropes Abdestillieren von ca. 100 ml Lösungsmittel absolutiert. Dann werden während 3 Stunden 48 g Phosgen bei 120 bis 130° eingeleitet. Durch vollständiges Abdestillieren des Chlorbenzols erhält man 65 g 2-Penta-fluoräthoxyphenyl-sulfonylisocyanat als fast farbloses Oel.

c) N-(2-Pentafluoräthoxyphenyl-sulfonyl)-N'-(4-methoxy-6-methyl-1, 3, 5-triazin-2-yl)-harnstoff.

3,2 g 2-Pentafluoräthoxyphenyl-sulfonylisocyanat und 1,4 g 2-Amino-4-methoxy-6-methyl-1,3,5-triazin werden mit 40 ml absolutem Dioxan während 3 Stunden bei 90-100° verrührt. Dann wird die Lösung auf 20° abgekühlt, filtriert und auf ca. 1/4 des Volumens eingedampft. Aus dem Rückstand kristallisieren, nach Zugabe von 50 ml Aether 3,3 g N-(2-Penta-fluoräthoxyphenyl-sulfonyl)-N'-(4-methoxy-6-methyl-1, 3, 5-triazin-2-yl)-harnstoff vom Schmelzpunkt 157-159°.

**Beispiel 6:**

N-(2-Difluormethoxyphenyl-sulfonyl)-N'-(4-chlor-6-methyl-pyrimidin-2-yl)-harnstoff.

Analog dem Beispiel 5c erhält man aus 2-Difluormethoxyphenylsulfonyl-isocyanat durch Umsetzen mit 2-Amino-4-chlor-6-methyl-Pyrimidin den N-(2-Difluormethoxyphenyl-sulfonyl)-N'-(4-chlor-6-methyl-pyrimidin-2-yl)-harnstoff, Smp. 190-191°.

In analoger Weise werden die in den folgenden Tabellen aufgelisteten Verbindungen der Formel I hergestellt.

**Tabelle 2:**

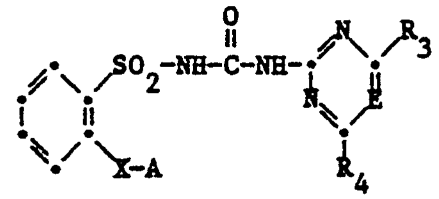

| Nr. | A | E | $R_3$ | $R_4$ | X | Phys.Daten |
|---|---|---|---|---|---|---|
| 1 | $CHF_2$ | N | $OC_2H_5$ | $OC_2H_5$ | O | Smp.117-118° |
| 2 | $CHF_2$ | CH | $OC_2H_5$ | $OC_2H_5$ | O | Smp.146-152° |
| 3 | $CHF_2$ | N | $CH_3$ | n-$OC_4H_9$ | O | sintert ab.110° |
| 4 | $CF_3$ | N | $OC_2H_5$ | $OC_2H_5$ | O | |
| 5 | $CF_3$ | CH | $OC_2H_5$ | $OC_2H_5$ | O | |
| 6 | $CF_3$ | N | $CH_3$ | n-$OC_4H_9$ | O | |
| 7 | $-CF_2-CHF_2$ | N | $OC_2H_5$ | $OC_2H_5$ | O | Smp.103-106° |
| 8 | $-CF_2-CHF_2$ | CH | $OC_2H_5$ | $OC_2H_5$ | O | |
| 9 | $-CF_2-CHF_2$ | N | $CH_3$ | $C_2H_5$ | O | Smp.147-148° |
| 10 | $-CF_2CHFCl$ | N | $OC_2H_5$ | $OC_2H_5$ | O | Smp.129-131° |
| 11 | $-CF_2-CHFCl$ | CH | $OC_2H_5$ | $OC_2H_5$ | O | |

Tabelle 2 (fortsetzung)

| Nr. | A | E | $R_3$ | $R_4$ | X | Phys.Daten |
|---|---|---|---|---|---|---|
| 12 | $-CF_2-CHFCl$ | N | $CH_3$ | $OC_4H_9$ | O | |
| 13 | $-CF_2-CHFBr$ | N | $OC_2H_5$ | $OC_2H_5$ | O | |
| 14 | $-CF_2-CHFBr$ | CH | $OC_2H_5$ | $OC_2H_5$ | O | |
| 15 | $-CF_2-CHFBr$ | N | $CH_3$ | $OC_4H_9$ | O | |
| 16 | $CHF_2$ | N | $CCl_3$ | $CCl_3$ | O | Smp.137-145° |
| 17 | $-C_2F_5$ | N | $OCH_3$ | $OCH_3$ | O | Smp.185-187° |
| 18 | $-CF_2-CClF_2$ | N | $OCH_3$ | $OCH_3$ | O | |
| 19 | $-CHF_2$ | N | $C_2H_5$ | $OCH_3$ | O | Smp.114-116° |
| 20 | $CHF_2$ | N | $C_2H_5$ | $OC_2H_5$ | O | |
| 21 | $CHF_2$ | N | $C_2H_5$ | $n-OC_3H_7$ | O | |
| 22 | $CHF_2$ | N | $C_2H_5$ | $n-OC_4H_9$ | O | |
| 23 | $C_2F_5$ | N | $C_2H_5$ | $OCH_3$ | O | Smp.153-154° |
| 24 | $C_2F_5$ | N | $CH_3$ | $OCH_3$ | O | Smp.145-149° |
| 25 | $C_2F_5$ | N | $CH_3$ | $OC_2H_5$ | O | |
| 26 | $C_2F_5$ | N | $OC_2H_5$ | $OC_2H_5$ | O | |
| 27 | $C_2F_5$ | CH | $CH_3$ | $CH_3$ | O | Smp.173-175° |
| 28 | $C_2F_5$ | CH | $C_2H_5$ | $OCH_3$ | O | |
| 29 | $C_2F_5$ | CH | $CH_3$ | $OCH_3$ | O | Smp.165-170° |
| 30 | $C_2F_5$ | CH | $OCH_3$ | $OCH_3$ | O | |
| 31 | $C_2F_5$ | N | $CH_3$ | $CH_3$ | O | |
| 32 | $CHF_2$ | N | Cl | Cl | O | Smp.105-107° mit 1 Mol Dioxan |
| 33 | $-CF_2-CHFBr$ | N | $CH_3$ | $n-OC_4H_9$ | S | |
| 34 | $-CF_2-CF_2Cl$ | N | $OCH_3$ | $OCH_3$ | S | |
| 35 | $-CF_2-CBrF_2$ | N | $OCH_3$ | $OCH_3$ | S | |
| 36 | $CHF_2$ | N | $CCl_3$ | $CCl_3$ | S | |
| 37 | $CHF_2$ | N | $C_2H_5$ | $OCH_3$ | S | Smp.146-147° |
| 38 | $CHF_2$ | N | $C_2H_5$ | $OCH_3$ | SO | |
| 39 | $CHF_2$ | N | $C_2H_5$ | $OCH_3$ | $SO_2$ | |
| 40 | $C_2F_5$ | N | $OCH_3$ | $OCH_3$ | S | |
| 41 | $CHF_2$ | N | $OC_2H_5$ | $OC_2H_5$ | S | |
| 42 | $CHF_2$ | N | $CH_3$ | $n-OC_4H_9$ | S | |
| 43 | $CHF_2$ | CH | $OC_2H_5$ | $OC_2H_5$ | S | |

**Tabelle 2** (fortsetzung)

| Nr. | A | E | $R_3$ | $R_4$ | X | Phys.Daten |
|---|---|---|---|---|---|---|
| 44 | $CHF_2$ | N | $OC_2H_5$ | $OC_2H_5$ | SO | |
| 45 | $CHF_2$ | CH | $OC_2H_5$ | $OC_2H_5$ | SO | |
| 46 | $CHF_2$ | N | $OC_2H_5$ | $OC_2H_5$ | $SO_2$ | |
| 47 | $CHF_2$ | CH | $OC_2H_5$ | $OC_2H_5$ | $SO_2$ | |
| 48 | $CHF_2$ | N | $CH_3$ | $n-OC_4H_9$ | $SO_2$ | |
| 49 | $CF_3$ | N | $OC_2H_5$ | $OC_2H_5$ | S | |
| 50 | $CF_3$ | CH | $OC_2H_5$ | $OC_2H_5$ | S | |
| 51 | $CF_3$ | N | $CH_3$ | $OC_4H_9$ | S | |
| 52 | $-CF_2-CHF_2$ | N | $OC_2H_5$ | $OC_2H_5$ | S | |
| 53 | $-CF_2-CHF_2$ | CH | $OC_2H_5$ | $OC_2H_5$ | S | |
| 54 | $-CF_2-CHF_2$ | N | $CH_3$ | $n-OC_4H_9$ | S | |
| 55 | $-CF_2-CHFCl$ | N | $OC_2H_5$ | $OC_2H_5$ | S | |
| 56 | $-CF_2-CHFCl$ | CH | $OC_2H_5$ | $OC_2H_5$ | S | |
| 57 | $-CF_2-CHFCl$ | N | $CH_3$ | $n-OC_4H_9$ | S | |
| 58 | $-CF_2-CHFBr$ | N | $OCH_2CH_3$ | $OCH_2CH_3$ | S | |
| 59 | $-CF_2-CHFBr$ | CH | $OC_2H_5$ | $OC_2H_5$ | S | |
| 60 | $C_2F_5$ | CH | $CH_3$ | Cl | O | |
| 61 | $C_2F_5$ | N | $OCH_3$ | Cl | O | Smp.153-155° |
| 62 | $C_2F_5$ | CH | $CH_3$ | F | O | |
| 63 | $C_2F_5$ | N | $OCH_3$ | $OCH_3$ | O | Smp.185-187° |
| 64 | $C_2F_5$ | N | $OCH_3$ | $OC_2H_5$ | O | |
| 65 | $C_2F_5$ | N | $CH_2Cl$ | $OCH_3$ | O | |
| 66 | $C_2F_5$ | N | $-CH(CH_3)_2$ | $OCH_3$ | O | |
| 67 | $C_2F_5$ | N | $C_2H_5$ | Cl | O | |
| 68 | $C_2F_5$ | N | $C_2H_5$ | $CH_3$ | O | |
| 69 | $C_2F_5$ | N | $-CH_2OCH_3$ | $OCH_3$ | O | |
| 70 | $C_2F_5$ | CH | $-CH_2OCH_3$ | $OCH_3$ | O | |
| 71 | $C_2F_5$ | N | $OCH_3$ | $-OCH(CH_3)_2$ | O | |
| 72 | $C_2F_5$ | N | $OCH_3$ | $SCH_3$ | O | |
| 73 | $C_2F_5$ | N | $CH_3$ | $OCH_3$ | S | |
| 74 | $C_2F_5$ | CH | $CH_3$ | $OCH_3$ | S | |
| 75 | $C_2F_5$ | N | $OCH_3$ | $OCH_3$ | S | |
| 76 | $CF_2Br$ | N | $CH_3$ | $OCH_3$ | S | |
| 77 | $CF_2Br$ | N | $OCH_3$ | $OCH_3$ | S | |

Tabelle 2 (fortsetzung)

| Nr. | A | E | $R_3$ | $R_4$ | X |
|---|---|---|---|---|---|
| 78 | $CF_2Br$ | CH | $CH_3$ | $OCH_3$ | S |
| 79 | $CF_2Br$ | CH | $OCH_3$ | $OCH_3$ | S |
| 80 | $CF_2Br$ | CH | $CH_3$ | $CH_3$ | S |
| 81 | $CF_2Br$ | N | $C_2H_5$ | $OCH_3$ | S |
| 82 | $CF_2Br$ | N | $CH_3$ | $OCH_3$ | O |
| 83 | $CF_2Br$ | N | $OCH_3$ | $OCH_3$ | O |
| 84 | $CF_2Br$ | N | $C_2H_5$ | $OCH_3$ | O |
| 85 | $CF_2Br$ | CH | $CH_3$ | $OCH_3$ | O |
| 86 | $CF_2Br$ | CH | $OCH_3$ | $OCH_3$ | O |
| 87 | $CF_3$ | CH | $CH_3$ | Cl | O |
| 88 | $CF_3$ | N | $OCH_3$ | $-OCH(CH_3)_2$ | O |
| 89 | $CF_3$ | N | Cl | $OCH_3$ | O |
| 90 | $CF_3$ | N | $C_2H_5$ | $OCH_3$ | O |
| 91 | $CF_3$ | N | $-CH(CH_3)_2$ | $OCH_3$ | O |
| 92 | $CF_3$ | N | $-OCH(CH_3)_2$ | $OCH_3$ | O |
| 93 | $-CH_2-CF_3$ | CH | $CH_3$ | Cl | O |
| 94 | $-CH_2-CF_3$ | N | $OCH_3$ | Cl | O |
| 95 | $CF_2Cl$ | CH | $-CH(CH_3)_2$ | $OCH_3$ | O |
| 96 | $CF_2Cl$ | CH | $OCH_3$ | $-SC_2H_5$ | O |
| 97 | $CF_2Cl$ | N | $OCH_3$ | $-SC_2H_5$ | O |
| 98 | $CF_2Cl$ | N | $OCH_3$ | $-SCH(CH_3)_2$ | O |
| 99 | $CF_2Cl$ | N | $CCl_3$ | $CH_3$ | O |
| 100 | $CF_2Cl$ | N | $CH_3$ | Br | O |
| 101 | $CF_2Cl$ | N | $CHF_2$ | $OCH_3$ | O |
| 102 | $CF_2Cl$ | N | $CHF_2$ | $CH_3$ | O |
| 103 | $CF_2Cl$ | N | $CH_2CF_3$ | Cl | O |
| 104 | $CF_2Cl$ | N | $CH_2CF_3$ | $OCH_3$ | O |
| 105 | $CF_2Cl$ | N | $CH_2CF_3$ | $CH_3$ | O |
| 106 | $CF_2Cl$ | N | $OC_2H_5$ | $OC_2H_5$ | O |
| 107 | $CF_2Cl$ | CH | $OC_2H_5$ | $OC_2H_5$ | O |
| 108 | $CF_2Cl$ | N | $C_2H_5$ | $OCH_3$ | O |
| 109 | $CF_2Cl$ | N | $C_2H_5$ | $OC_2H_5$ | O |
| 110 | $CF_2Cl$ | N | $CH_3$ | $OCH_3$ | O |

**Tabelle 2** (fortsetzung)

| Nr. | A | E | $R_3$ | $R_4$ | X |
|---|---|---|---|---|---|
| 111 | $CF_2Cl$ | N | $CH_3$ | $OC_2H_5$ | O |
| 112 | $CF_2Cl$ | CH | $CH_3$ | $CH_3$ | O |
| 113 | $CF_2Cl$ | CH | $CH_3$ | $OCH_3$ | O |
| 114 | $CF_2Cl$ | N | $CH_3$ | Br | O |
| 115 | $CF_2Cl$ | N | $CH_3$ | H | O |
| 116 | $CF_2Cl$ | CH | $C_2H_5$ | Cl | O |
| 117 | $CF_2Cl$ | CH | $CH_3$ | Cl | O |
| 118 | $CF_2Cl$ | CH | $CH_3$ | $SCH_3$ | O |
| 119 | $CF_2Cl$ | CH | $CH_3$ | F | O |
| 120 | $CF_2Cl$ | CH | $CH_3$ | Br | O |
| 121 | $CF_2Cl$ | CH | $C_2H_5$ | $OC_2H_5$ | O |
| 122 | $CF_2Cl$ | CH | $C_2H_5$ | $SCH_3$ | O |
| 123 | $CF_2Cl$ | CH | $CF_3$ | $CH_3$ | O |
| 124 | $CF_2Cl$ | CH | $CH_2Cl$ | $CH_3$ | O |
| 125 | $CF_2Cl$ | CH | $CH_2Cl$ | $OCH_3$ | O |
| 126 | $CF_2Cl$ | CH | $OCH_3$ | Cl | O |
| 127 | $CF_2Cl$ | CH | Cl | Cl | O |
| 128 | $CF_2Cl$ | CH | $OCH_3$ | $SCH_3$ | O |
| 129 | $CF_2Cl$ | CH | $OCH_3$ | $-OCH(CH_3)_2$ | O |
| 130 | $CF_2Cl$ | CH | $CH_2F$ | $OCH_3$ | O |
| 131 | $CF_2Cl$ | CH | $CH_2F$ | $CH_3$ | O |
| 132 | $CF_2Cl$ | CH | $CF_3$ | $OCH_3$ | O |
| 133 | $CF_2Cl$ | N | $C_2H_5$ | $-OCH(CH_3)_2$ | O |
| 134 | $CF_2Cl$ | N | $C_2H_5$ | Cl | O |
| 135 | $CF_2Cl$ | N | $C_2H_5$ | $SCH_3$ | O |
| 136 | $CF_2Cl$ | N | $C_2H_5$ | $CH_3$ | O |
| 137 | $CF_2Cl$ | N | $C_2H_5$ | $C_2H_5$ | O |
| 138 | $CF_2Cl$ | N | $OCH_3$ | $-OCH(CH_3)_2$ | O |
| 139 | $CF_2Cl$ | N | $OCH_3$ | $-OCH-CH_3$<br>$CH_2CH_3$ | O |
| 140 | $CF_2Cl$ | N | $CH_3$ | $-CH(CH_3)_2$ | O |
| 141 | $CF_2Cl$ | N | $-CH(CH_3)_2$ | Cl | O |
| 142 | $CF_2Cl$ | N | $-CH(CH_3)_2$ | $OCH_3$ | O |
| 143 | $CF_2Cl$ | N | $-CH(CH_3)_2$ | $OC_2H_5$ | O |

**Tabelle 2** (fortsetzung)

| Nr. | A | E | $R_3$ | $R_4$ | X |
|---|---|---|---|---|---|
| 144 | $CF_2Cl$ | N | $-CH(CH_3)_2$ | $SCH_3$ | O |
| 145 | $CF_2Cl$ | N | $CH_2Cl$ | $CH_3$ | O |
| 146 | $CF_2Cl$ | N | $CH_2Cl$ | $OCH_3$ | O |
| 147 | $CF_2Cl$ | N | $CH_2F$ | $CH_3$ | O |
| 148 | $CF_2Cl$ | N | $CH_2F$ | $OCH_3$ | O |
| 149 | $CF_2Cl$ | N | $CH_2F$ | $OC_2H_5$ | O |
| 150 | $CF_2Cl$ | N | $-CH_2OCH_3$ | $C_2H_5$ | O |
| 156 | $CF_2Cl$ | N | $SCH_3$ | Cl | O |
| 157 | $CF_2Cl$ | N | $SCH_3$ | $OCH_3$ | O |
| 158 | $CF_2Cl$ | N | $SCH_3$ | $OC_2H_5$ | O |
| 159 | $CF_2Cl$ | N | $SCH_3$ | $-OCH(CH_3)_2$ | O |
| 160 | $CF_2Cl$ | N | $-OCH(CH_3)_2$ | Cl | O |
| 161 | $CF_2Cl$ | N | $CF_3$ | $OCH_3$ | O |
| 162 | $CF_2Cl$ | N | $CF_3$ | $CH_3$ | O |
| 163 | $CF_2Cl$ | N | $CF_3$ | $OC_2H_5$ | O |
| 164 | $CF_2Cl$ | N | $CCl_3$ | $OCH_3$ | O |
| 165 | $CF_2Cl$ | N | $CCl_3$ | $SCH_3$ | O |
| 166 | $CF_2Cl$ | N | $CH_3$ | Cl | O |
| 167 | $CF_2Cl$ | N | $-OCH_3$ | Cl | O |
| 168 | $CF_2Cl$ | N | $OCH_3$ | F | O |
| 169 | $CF_2Cl$ | N | $OCH_3$ | Br | O |
| 170 | $CF_2Cl$ | N | $CH_3$ | F | O |

**Tabelle 2** (fortsetzung)

| Nr. | A | E | $R_3$ | $R_4$ | X |
|---|---|---|---|---|---|
| 171 | $CF_2Cl$ | CH | $OCH_3$ | $OCH_3$ | S |
| 172 | $CF_2Cl$ | N | $CH_3$ | $CH_3$ | S |
| 173 | $CF_2Cl$ | N | $OCH_3$ | $OC_2H_5$ | S |
| 174 | $CF_2Cl$ | CH | $-CH_2-OCH_3$ | $OCH_3$ | S |
| 175 | $CF_2Cl$ | N | $OCH_3$ | $OCH_3$ | S |
| 176 | $CF_2Cl$ | CH | $C_2H_5$ | $OCH_3$ | S |
| 177 | $CF_2Cl$ | N | $C_2H_5$ | $CH_3$ | S |
| 178 | $CF_2Cl$ | N | $CH_3$ | $OCH_3$ | SO |
| 179 | $CF_2Cl$ | N | $OCH_3$ | $OCH_3$ | SO |
| 180 | $CF_2Cl$ | CH | $CH_3$ | $OCH_3$ | SO |
| 181 | $CF_2Cl$ | CH | $OCH_3$ | $OCH_3$ | SO |
| 182 | $CF_2Cl$ | N | $CH_3$ | $OCH_3$ | $SO_2$ |
| 183 | $CF_2Cl$ | N | $CH_3$ | $CH_3$ | $SO_2$ |
| 184 | $CF_2Cl$ | N | $OCH_3$ | $OCH_3$ | $SO_2$ |
| 185 | $CF_2Cl$ | CH | $CH_3$ | $OCH_3$ | $SO_2$ |
| 186 | $CF_2Cl$ | CH | $CH_3$ | $CH_3$ | $SO_2$ |
| 187 | $CF_2Cl$ | CH | $OCH_3$ | $OCH_3$ | $SO_2$ |
| 188 | $-CF_2-CF_3$ | N | $OCH_3$ | $-OCH(CH_3)_2$ | O |
| 189 | $-CF_2-CF_3$ | N | Cl | $-OCH(CH_3)_2$ | O |
| 190 | $CF_2Cl$ | N | $SCH_3$ | $OCH_3$ | S |
| 191 | $CF_2Cl$ | N | $CF_3$ | $OCH_3$ | S |
| 192 | $CF_2Cl$ | N | $CH_3$ | Cl | S |
| 193 | $CF_2Cl$ | N | $OCH_3$ | Cl | S |
| 194 | $CF_2Cl$ | N | $C_2H_5$ | $CH_3$ | S |
| 195 | $CF_2Cl$ | N | $OCH_3$ | $-OCH(CH_3)_2$ | S |
| 196 | $CF_2Cl$ | N | $-CH(CH_3)_2$ | $OCH_3$ | S |
| 197 | $CF_2Cl$ | N | $CH_2Cl$ | $OCH_3$ | S |
| 198 | $CF_2Cl$ | N | $CH_2F$ | $OCH_3$ | S |
| 199 | $CF_2Cl$ | CH | $C_2H_5$ | Cl | S |
| 200 | $CF_2Cl$ | CH | $CH_3$ | Cl | S |

**Tabelle 2** (fortsetzung)

| Nr. | A | E | $R_3$ | $R_4$ | X |
|---|---|---|---|---|---|
| 201 | $CF_2Cl$ | CH | $CH_3$ | F | S |
| 202 | $CF_2Cl$ | CH | $OCH_3$ | Cl | S |
| 203 | $CF_2Cl$ | N | $OC_2H_5$ | $OC_2H_5$ | S |
| 204 | $CF_2Cl$ | N | $C_2H_5$ | $OCH_3$ | S |
| 205 | $CF_2Cl$ | N | $CH_3$ | $OCH_3$ | S |
| 206 | $CF_2Cl$ | N | $CH_3$ | $OC_2H_5$ | S |
| 207 | $CF_2Cl$ | CH | $CH_3$ | $CH_3$ | S |
| 208 | $CF_2Cl$ | CH | $CH_3$ | $OCH_3$ | S |
| 209 | $CF_2Cl$ | CH | $OCH_3$ | $OCH_3$ | O |
| 210 | $CF_2Cl$ | N | $CH_3$ | $CH_3$ | O |
| 211 | $CF_2Cl$ | N | $OCH_3$ | $OC_2H_5$ | O |
| 212 | $CF_2Cl$ | CH | $OCH_3$ | $OC_2H_5$ | O |
| 213 | $CF_2Cl$ | CH | $-CH_2-OCH_3$ | $OCH_3$ | O |
| 214 | $CF_2Cl$ | N | $-CH_2-OCH_3$ | $OCH_3$ | O |
| 215 | $CF_2Cl$ | N | $OCH_3$ | $OCH_3$ | O |
| 216 | $CF_2Cl$ | CH | $C_2H_5$ | $OCH_3$ | O |
| 217 | $CF_2Cl$ | N | $-CH_2OCH_3$ | $CH_3$ | O |
| 218 | $CHF_2$ | CH | $-CH(CH_3)_2$ | $OCH_3$ | S |
| 219 | $CHF_2$ | CH | $OCH_3$ | $SC_2H_5$ | S |
| 220 | $CHF_2$ | N | $OCH_3$ | $SC_2H_5$ | S |
| 221 | $CHF_2$ | N | $OCH_3$ | $-SCH(CH_3)_2$ | S |
| 222 | $CHF_2$ | N | $CCl_3$ | $CH_3$ | S |
| 223 | $CHF_2$ | N | $CH_3$ | Br | S |
| 224 | $CHF_2$ | N | $CHF_2$ | $OCH_3$ | S |
| 225 | $CHF_2$ | N | $CHF_2$ | $CH_3$ | S |
| 226 | $CHF_2$ | N | $-CH_2CF_3$ | Cl | S |
| 227 | $CHF_2$ | N | $-CH_2-CF_3$ | $OCH_3$ | S |
| 228 | $CHF_2$ | N | $-CH_2-CF_3$ | $CH_3$ | S |
| 229 | $CHF_2$ | N | $-CH_2-OCH_3$ | $CH_3$ | S |

**Tabelle 2** (fortsetzung)

| Nr. | A | E | $R_3$ | $R_4$ | X | Phys.Daten |
|---|---|---|---|---|---|---|
| 234 | $CHF_2$ | N | $SCH_3$ | Cl | S | Smp.162-163° |
| 235 | $CHF_2$ | N | $SCH_3$ | $OCH_3$ | S | Smp.159-161° |
| 236 | $CHF_2$ | N | $SCH_3$ | $OC_2H_5$ | S | |
| 237 | $CHF_2$ | N | $SCH_3$ | $-OCH(CH_3)_2$ | S | |
| 238 | $CHF_2$ | N | $-OCH(CH_3)_2$ | Cl | S | Smp.147-149° |
| 239 | $CHF_2$ | N | $CF_3$ | $OCH_3$ | S | |
| 240 | $CHF_2$ | N | $CF_3$ | $CH_3$ | S | |
| 241 | $CHF_2$ | N | $CF_3$ | $OC_2H_5$ | S | |
| 242 | $CHF_2$ | N | $CCl_3$ | $OCH_3$ | S | |
| 243 | $CHF_2$ | N | $CCl_3$ | $SCH_3$ | S | |
| 244 | $CHF_2$ | N | $CH_3$ | Cl | S | |
| 245 | $CHF_2$ | N | $OCH_3$ | Cl | S | |
| 246 | $CHF_2$ | N | $OCH_3$ | F | S | |
| 247 | $CHF_2$ | N | $OCH_3$ | Br | S | |
| 248 | $CHF_2$ | N | $CH_3$ | F | S | |
| 249 | $CHF_2$ | N | $CH_3$ | Br | S | |
| 250 | $CHF_2$ | N | $CH_3$ | H | S | |
| 251 | $CHF_2$ | CH | $C_2H_5$ | Cl | S | |
| 252 | $CHF_2$ | CH | $CH_3$ | Cl | S | Smp.194-195° |
| 253 | $CHF_2$ | CH | $CH_3$ | $SCH_3$ | S | |
| 254 | $CHF_2$ | CH | $CH_3$ | F | S | Smp.186-187° |
| 255 | $CHF_2$ | CH | $CH_3$ | Br | S | |
| 256 | $CHF_2$ | CH | $C_2H_5$ | $OC_2H_5$ | S | |
| 257 | $CHF_2$ | CH | $C_2H_5$ | $SCH_3$ | S | |
| 258 | $CHF_2$ | $CH_2$ | $CF_3$ | $CH_3$ | S | |

**Tabelle 2** (fortsetzung)

| Nr. | A | E | $R_3$ | $R_4$ | X | Phys.Daten |
|---|---|---|---|---|---|---|
| 259 | $CHF_2$ | CH | $CH_2Cl$ | $CH_3$ | S | |
| 260 | $CHF_2$ | CH | $CH_2Cl$ | $OCH_3$ | S | |
| 261 | $CHF_2$ | CH | $OCH_3$ | Cl | S | Smp.159-164° |
| 262 | $CHF_2$ | CH | Cl | Cl | S | Smp.201-202° |
| 263 | $CHF_2$ | CH | $OCH_3$ | $SCH_3$ | S | |
| 264 | $CHF_2$ | CH | $OCH_3$ | $-OCH(CH_3)_2$ | S | |
| 265 | $CHF_2$ | CH | $CH_2F$ | $OCH_3$ | S | |
| 266 | $CHF_2$ | CH | $CH_2F$ | $CH_3$ | S | |
| 267 | $CHF_2$ | CH | $CF_3$ | $OCH_3$ | S | |
| 268 | $CHF_2$ | N | $C_2H_5$ | $-OCH(CH_3)_2$ | S | |
| 269 | $CHF_2$ | N | $C_2H_5$ | Cl | S | Smp. 163-164° |
| 270 | $CHF_2$ | N | $C_2H_5$ | $SCH_3$ | S | |
| 271 | $CHF_2$ | N | $C_2H_5$ | $CH_3$ | S | Smp.166-167° |
| 272 | $CHF_2$ | N | $C_2H_5$ | $C_2H_5$ | S | |
| 273 | $CHF_2$ | N | $OCH_3$ | $-OCH(CH_3)_2$ | S | Smp.120-122° |
| 274 | $CHF_2$ | N | $OCH_3$ | $-OCH-CH_3$<br>$CH_2CH_3$ | S | |
| 275 | $CHF_2$ | N | $CH_3$ | $-CH(CH_3)_2$ | S | |
| 276 | $CHF_2$ | N | $-CH(CH_3)_2$ | Cl | S | Smp.169-170° |
| 277 | $CHF_2$ | N | $-CH(CH_3)_2$ | $OCH_3$ | S | Smp.148-150° |
| 278 | $CHF_2$ | N | $-CH(CH_3)_2$ | $OC_2H_5$ | S | |
| 279 | $CHF_2$ | N | $-CH(CH_3)_2$ | $SCH_3$ | S | |
| 280 | $CHF_2$ | N | $CH_2Cl$ | $CH_3$ | S | |
| 281 | $CHF_2$ | N | $CH_2Cl$ | $OCH_3$ | S | |
| 282 | $CHF_2$ | N | $CH_2F$ | $CH_3$ | S | |
| 283 | $CHF_2$ | N | $CH_2F$ | $OCH_3$ | S | |
| 284 | $CHF_2$ | N | $CH_2F$ | $OCH_2H_5$ | S | |
| 285 | $CHF_2$ | N | $-CH_2-OCH_3$ | $C_2H_5$ | S | |
| 287 | $CHF_2$ | CH | $-CH(CH_3)_2$ | $OCH_3$ | O | |

**Tabelle 2** (fortsetzung)

| Nr. | A | E | $R_3$ | $R_4$ | X | Phys.Daten |
|---|---|---|---|---|---|---|
| 288 | $CHF_2$ | CH | $OCH_3$ | $SC_2H_5$ | O | |
| 289 | $CHF_2$ | N | $OCH_3$ | $SC_2H_5$ | O | |
| 290 | $CHF_2$ | N | $OCH_3$ | $-SCH(CH_3)_2$ | O | |
| 291 | $CHF_2$ | N | $CCl_3$ | $CH_3$ | O | |
| 292 | $CHF_2$ | N | $CH_3$ | Br | O | |
| 293 | $CHF_2$ | N | $CHF_2$ | $OCH_3$ | O | |
| 294 | $CHF_2$ | N | $CHF_2$ | $CH_3$ | O | |
| 295 | $CHF_2$ | N | $-CH_2-CF_3$ | Cl | O | |
| 296 | $CHF_2$ | N | $-CH_2-CF_3$ | $OCH_3$ | O | |
| 297 | $CHF_2$ | N | $-CH_2-CF_3$ | $CH_3$ | O | |
| 298 | $CHF_2$ | N | $-CH_2-OCH_3$ | $CH_3$ | O | |
| 299 | $CHF_2$ | N | $CH_3$ | Br | O | |
| 300 | $CHF_2$ | N | $CH_3$ | H | O | |
| 301 | $CHF_2$ | CH | $C_2H_5$ | Cl | O | |
| 302 | $CHF_2$ | CH | $CH_3$ | Cl | O | Smp.190-191° |
| 303 | $CHF_2$ | CH | $CH_3$ | $SCH_3$ | O | Smp.171-172° |
| 304 | $CHF_2$ | CH | $CH_3$ | F | O | Smp.174-176° |
| 305 | $CHF_2$ | CH | $CH_3$ | Br | O | |
| 306 | $CHF_2$ | CH | $C_2H_5$ | $OC_2H_5$ | O | |
| 307 | $CHF_2$ | CH | $C_2H_5$ | $SCH_3$ | O | |
| 308 | $CHF_2$ | CH | $CF_3$ | $CH_3$ | O | |
| 309 | $CHF_2$ | CH | $CH_2Cl$ | $CH_3$ | O | |
| 310 | $CHF_2$ | CH | $CH_2Cl$ | $OCH_3$ | O | |
| 311 | $CHF_2$ | CH | $OCH_3$ | Cl | O | Smp.165-167° |
| 312 | $CHF_2$ | CH | Cl | Cl | O | Smp.183-188° |
| 313 | $CHF_2$ | CH | $OCH_3$ | $SCH_3$ | O | |
| 314 | $CHF_2$ | CH | $OCH_3$ | $-OCH(CH_3)_2$ | O | |
| 315 | $CHF_2$ | CH | $CH_2F$ | $OCH_3$ | O | |
| 316 | $CHF_2$ | CH | $CH_2F$ | $CH_3$ | O | |
| 317 | $CHF_2$ | CH | $CF_3$ | $OCH_3$ | O | |

**Tabelle 2** (fortsetzung)

| Nr. | A | E | $R_3$ | $R_4$ | X | Phys.Daten |
|---|---|---|---|---|---|---|
| 318 | $CHF_2$ | N | $C_2H_5$ | $-OCH(CH_3)_2$ | O | |
| 319 | $CHF_2$ | N | $C_2H_5$ | Cl | O | Smp.135-137° |
| 320 | $CHF_2$ | N | $C_2H_5$ | $SCH_3$ | O | |
| 321 | $CHF_2$ | N | $C_2H_5$ | $CH_3$ | O | Smp.136-138° |
| 322 | $CHF_2$ | N | $C_2H_5$ | $C_2H_5$ | O | |
| 323 | $CHF_2$ | N | $OCH_3$ | $-OCH(CH_3)_2$ | O | Smp.113-115° |
| 324 | $CHF_2$ | N | $OCH_3$ | $-OCH-CH_3$<br>$CH_2CH_3$ | O | |
| 325 | $CHF_2$ | N | $CH_3$ | $-CH(CH_3)_2$ | O | Smp.137-140° |
| 326 | $CHF_2$ | N | $-CH(CH_3)_2$ | Cl | O | Smp.160-162° |
| 327 | $CHF_2$ | N | $-CH(CH_3)_2$ | $OCH_3$ | O | Smp. 75-78° |
| 328 | $CHF_2$ | N | $-CH(CH_3)_2$ | $OC_2H_5$ | O | |
| 329 | $CHF_2$ | N | $-CH(CH_3)_2$ | $SCH_3$ | O | |
| 330 | $CHF_2$ | N | $CH_2Cl$ | $CH_3$ | O | Smp.113-115° |
| 331 | $CHF_2$ | N | $CH_2Cl$ | $OCH_3$ | O | Smp. 94-96° |
| 332 | $CHF_2$ | N | $CH_2F$ | $CH_3$ | O | |
| 333 | $CHF_2$ | N | $CH_2F$ | $OCH_3$ | O | |
| 334 | $CHF_2$ | N | $CH_2F$ | $OC_2H_5$ | O | |
| 335 | $CHF_2$ | N | $-CH_2-OCH_3$ | $C_2H_5$ | O | |
| 341 | $CHF_2$ | N | $SCH_3$ | Cl | O | Smp.147-149° |
| 342 | $CHF_2$ | N | $SCH_3$ | $OCH_3$ | O | Smp.161-162° |
| 343 | $CHF_2$ | N | $SCH_3$ | $OC_2H_5$ | O | |
| 344 | $CHF_2$ | N | $SCH_3$ | $-OCH(CH_3)_2$ | O | Smp.163-164° |
| 345 | $CHF_2$ | N | $-OCH(CH_3)_2$ | Cl | O | Smp.103-104° |

Tabelle 2 (fortsetzung)

| Nr. | A | E | $R_3$ | $R_4$ | X | Phys.Daten |
|---|---|---|---|---|---|---|
| 346 | $CHF_2$ | N | $CF_3$ | $OCH_3$ | O | |
| 347 | $CHF_2$ | N | $CF_3$ | $CH_3$ | O | |
| 348 | $CHF_2$ | N | $CF_3$ | $OC_2H_5$ | O | |
| 349 | $CHF_2$ | N | $CCl_3$ | $OCH_3$ | O | |
| 350 | $CHF_2$ | N | $CCl_3$ | $SCH_3$ | O | |
| 351 | $CHF_2$ | N | $CH_3$ | Cl | O | |
| 352 | $CHF_2$ | N | $OCH_3$ | Cl | O | Smp.167-168° |
| 353 | $CHF_2$ | N | $OCH_3$ | F | O | |
| 354 | $CHF_2$ | N | $OCH_3$ | Br | O | |
| 355 | $CHF_2$ | N | $CH_3$ | F | O | |
| 356 | $CH_2F$ | N | $CH_3$ | $OCH_3$ | O | |
| 357 | $CH_2F$ | N | $OCH_3$ | $OCH_3$ | O | |
| 358 | $CH_2F$ | N | $CH_3$ | $CH_3$ | O | |
| 359 | $CH_2F$ | N | $C_2H_5$ | $OCH_3$ | O | |
| 360 | $CH_2F$ | CH | $CH_3$ | $OCH_3$ | S | |
| 361 | $CH_2F$ | CH | $OCH_3$ | $OCH_3$ | S | |
| 362 | $CH_2F$ | CH | $CH_3$ | $CH_3$ | S | |
| 363 | $CH_2F$ | N | $CH_3$ | $OCH_3$ | O | |
| 364 | $CH_2F$ | CH | $CH_3$ | $OCH_3$ | O | |
| 365 | $CH_2F$ | CH | $OCH_3$ | $OCH_3$ | O | |
| 366 | $CH_2F$ | N | $C_2H_5$ | $OCH_3$ | O | |
| 367 | $CH_2F$ | N | $CH_3$ | $CH_3$ | O | |
| 368 | $-CF_2-CF_2Br$ | N | $CH_3$ | $OCH_3$ | O | |
| 369 | $-CF_2-CF_2Br$ | CH | $CH_3$ | $OCH_3$ | O | |
| 370 | $-CF_2-CF_2Br$ | CH | $OCH_3$ | $OCH_3$ | O | |
| 371 | $-CF_2-CF_2Cl$ | N | $CH_3$ | $OCH_3$ | S | |
| 372 | $-CH_2-CH_2F$ | N | $CH_3$ | $OCH_3$ | O | |
| 373 | $-CH_2-CH_2F$ | N | $CH_3$ | $CH_3$ | O | |
| 374 | $-CH_2-CH_2F$ | N | $OCH_3$ | $OCH_3$ | O | |
| 375 | $-CH_2-CH_2F$ | N | $C_2H_5$ | $OCH_3$ | O | |

**Tabelle 2** (fortsetzung)

| Nr. | A | E | $R_3$ | $R_4$ | X |
|---|---|---|---|---|---|
| 376 | $-CH_2-CH_2F$ | CH | $CH_3$ | $OCH_3$ | O |
| 377 | $-CH_2-CH_2F$ | CH | $OCH_3$ | $OCH_3$ | O |
| 378 | $-CH_2-CH_2F$ | CH | $CH_3$ | $CH_3$ | O |
| 379 | $-CH_2-CH_2F$ | N | $CH_3$ | $OCH_3$ | S |
| 380 | $-CH_2-CH_2F$ | N | $OCH_3$ | $OCH_3$ | S |
| 381 | $-CH_2-CCl_3$ | N | $CH_3$ | $OCH_3$ | O |
| 382 | $-CH_2-CCl_3$ | N | $OCH_3$ | $OCH_3$ | O |
| 383 | $-CH_2-CCl_3$ | CH | $CH_3$ | $OCH_3$ | O |
| 384 | $-CH_2-CCl_3$ | CH | $OCH_3$ | $OCH_3$ | O |
| 385 | $-CH_2-CCl_3$ | N | $C_2H_5$ | $OCH_3$ | O |
| 386 | $-CF_2-CH_3$ | N | $OCH_3$ | $OCH_3$ | O |
| 387 | $-CF_2-CH_3$ | N | $CH_3$ | $OCH_3$ | O |
| 388 | $-CF_2-CH_3$ | N | $CH_3$ | $CH_3$ | O |
| 389 | $-CF_2-CH_3$ | CH | $CH_3$ | $OCH_3$ | O |
| 390 | $-CF_2-CH_3$ | CH | $OCH_3$ | $OCH_3$ | O |
| 391 | $-CH_2-CH_2Cl$ | N | $CH_3$ | $OCH_3$ | S |
| 392 | $-CH_2-CH_2Cl$ | N | $OCH_3$ | $OCH_3$ | S |
| 393 | $-CH_2-CH_2Cl$ | N | $C_2H_5$ | $OCH_3$ | S |
| 394 | $-CH_2-CH_2Cl$ | N | $CH_3$ | $OC_2H_5$ | S |
| 395 | $-CH_2-CH_2Cl$ | CH | $C_2H_5$ | $OCH_3$ | S |
| 396 | $-CH_2-CH_2Cl$ | N | $CH_3$ | $OC_2H_5$ | O |
| 397 | $-CH_2-CH_2Cl$ | N | $CH_3$ | $OC_2H_5$ | S |
| 398 | $-CH_2-CH_2Cl$ | CH | $OCH_3$ | $OCH_3$ | S |
| 399 | $-CH_2-CH_2Cl$ | CH | $CH_3$ | $OCH_3$ | S |
| 400 | $-CH_2-CH_2Cl$ | N | $CF_3$ | $OCH_3$ | O |
| 401 | $-CH_2-CH_2Cl$ | N | $CH_2F$ | $OCH_3$ | O |
| 402 | $-CH_2-CH_2Cl$ | CH | $CH_2CH_3$ | CL | O |
| 403 | $-CH_2-CH_2Cl$ | CH | $OCH_3$ | Cl | O |
| 404 | $-CH_2-CH_2Cl$ | CH | $-CH_2OCH_3$ | $OCH_3$ | O |
| 405 | $-CH_2-CH_2Cl$ | N | $-CH_2OCH_3$ | $OCH_3$ | O |

Tabell 2 (fortsetzung)

| Nr. | A | E | $R_3$ | $R_4$ | X | Phys.Daten |
|---|---|---|---|---|---|---|
| 406 | $-CH_2-CH_2Cl$ | N | $CH_3$ | $OCH_3$ | O | Smp.147–175° |
| 407 | $-CH_2-CH_2Cl$ | N | $OCH_3$ | $OCH_3$ | O | |
| 408 | $-CH_2-CH_2Cl$ | N | $CH_3$ | $CH_3$ | O | |
| 409 | $-CH_2-CH_2Cl$ | N | $C_2H_5$ | $OCH_3$ | O | |
| 410 | $-CH_2-CH_2Cl$ | N | $C_2H_5$ | $CH_3$ | O | |
| 411 | $-CH_2-CH_2Cl$ | N | $CH_3$ | Cl | O | |
| 412 | $-CH_2-CH_2Cl$ | N | $CH_2Cl$ | $OCH_3$ | O | |
| 413 | $-CH_2-CH_2Cl$ | N | $OCH_3$ | $-OCH(CH_3)_2$ | O | |
| 414 | $-CH_2-CH_2Cl$ | N | $OCH_3$ | $SCH_3$ | O | |
| 415 | $-CH_2-CH_2Cl$ | N | $-CH(CH_3)_2$ | $OCH_3$ | O | |
| 416 | $-CH_2-CH_2Cl$ | N | $OC_2H_5$ | $OCH_3$ | O | |
| 417 | $-CH_2-CH_2Cl$ | N | $OC_2H_5$ | $OC_2H_5$ | O | |
| 418 | $-CH_2-CH_2Cl$ | N | $OCH_3$ | CL | O | |
| 419 | $-CH_2-CH_2Cl$ | CH | $CH_3$ | $OCH_3$ | O | |
| 420 | $-CH_2-CH_2Cl$ | CH | $OCH_3$ | $OCH_3$ | O | |
| 421 | $-CH_2-CH_2Cl$ | CH | $CH_3$ | $CH_3$ | O | |
| 422 | $-CH_2-CH_2Cl$ | CH | $CH_3$ | Cl | O | |
| 423 | $-CH_2-CH_2Cl$ | CH | $CH_3$ | F | O | |
| 424 | $-CH_2-CH_2Cl$ | CH | $C_2H_5$ | $OCH_3$ | O | |
| 425 | $-CH_2-CHCl-CH_2Cl$ | N | $CH_3$ | $OCH_3$ | O | |
| 426 | $-CH_2-CHCl-CH_2Cl$ | N | $OCH_3$ | $OCH_3$ | O | |
| 427 | $-CH_2-CHCl-CH_2Cl$ | N | $CH_3$ | $CH_3$ | O | |
| 428 | $-CH_2-CHCl-CH_2Cl$ | N | $C_2H_5$ | $OCH_3$ | O | |
| 429 | $-CH_2-CHCl-CH_2Cl$ | CH | $CH_3$ | $OCH_3$ | O | |
| 430 | $-CH_2-CHCl-CH_2Cl$ | CH | $OCH_3$ | $OCH_3$ | O | |
| 431 | $-CH_2-CHCl-CH_2Cl$ | CH | $CH_3$ | $CH_3$ | O | |
| 432 | $-CH_2-CHCl-CH_2Cl$ | N | $CH_3$ | $OCH_3$ | S | |
| 433 | $-CH_2-CHCl-CH_2Cl$ | N | $OCH_3$ | $OCH_3$ | S | |
| 434 | $-CH_2-CHCl-CH_2Cl$ | CH | $CH_3$ | $OCH_3$ | S | |
| 435 | $-CH_2-CHCl-CH_2Cl$ | CH | $OCH_3$ | $OCH_3$ | S | |

**Tabelle 2** (fortsetzung)

| Nr. | A | E | $R_4$ | $R_4$ | X | Phys. Daten |
|---|---|---|---|---|---|---|
| 436 | $-CH_3-CHCl-CH_2Cl$ | N | $C_2H_5$ | $CH_3$ | O | |
| 437 | $-CH_2-CHCl-CH_2Cl$ | N | $OCH_3$ | Cl | O | |
| 438 | $-CH_2-CHCl-CH_2Cl$ | CH | $CH_3$ | Cl | O | |
| 439 | $CH_2F$ | N | $CH_3$ | $OC_2H_5$ | O | |
| 440 | $CH_2F$ | CH | $CH_3$ | Cl | O | |
| 441 | $CH_2F$ | N | $OCH_3$ | Cl | O | |
| 442 | $-CH_2-CHCl-CH_2Cl$ | CH | $C_2H_5$ | Cl | O | |
| 443 | $-CH_2-CHBr-CH_2Br$ | N | $CH_3$ | $OCH_3$ | O | |
| 444 | $-CH_2-CHCl-CHCl$<br>$CH_3$ | N | $CH_3$ | $OCH_3$ | O | |
| 445 | $-CH_2-CHCl-CHCl$<br>$CH_3$ | N | $OCH_3$ | $OCH_3$ | O | |
| 446 | $-CH_2CHCl-CHCl$<br>$CH_3$ | N | $CH_3$ | $CH_3$ | O | |
| 447 | $-CH_2-CHCl-CHCl$<br>$CH_3$ | CH | $CH_3$ | $OCH_3$ | O | |
| 448 | $-CH_2-CHCl-CHCl$<br>$CH_3$ | CH | $CH_3$ | Cl | O | |
| 449 | $-CH_2-CHCl-CHCl$<br>$CH_3$ | CH | $OCH_3$ | $OCH_3$ | O | |
| 450 | $-CH_2-CHCl-CHCl$<br>$CH_3$ | N | $C_2H_5$ | $OCH_3$ | O | |
| 451 | $-CF_2-CHF_2$ | N | $C_2H_5$ | $OCH_3$ | O | Smp. 158-160° |
| 452 | $-CF_2-CHF_2$ | CH | $CH_3$ | Cl | O | |
| 453 | $-CF_2-CHF_2$ | CH | $OCH_3$ | Cl | O | Smp. 164-165° |
| 454 | $-CF_2-CHFCl$ | N | $C_2H_5$ | $OCH_3$ | O | Smp. 144-146° |
| 455 | $-CF_2-CHFCl$ | CH | $CH_3$ | Cl | O | Smp. 177-179° |
| 456 | $-CF_2-CHFCl$ | CH | $OCH_3$ | Cl | O | Smp. 164-165° |
| 457 | $-CF_2-CHFCl$ | N | $CH(CH_3)_2$ | $OCH_3$ | O | Smp. 128-130° |

Tabelle 3

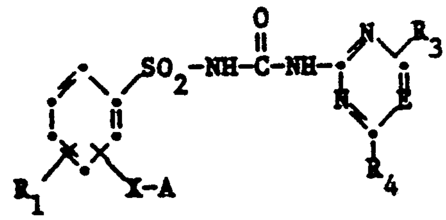

| Nr. | A | Stellung von -X-A | $R_1$ | $R_3$ | $R_4$ | X | E | phys. Daten |
|---|---|---|---|---|---|---|---|---|
| 501 | $CHF_2$ | 2 | 5-F | $C_2H_5$ | $OCH_3$ | O | N | Smp.140-141° |
| 502 | $CHF_2$ | 2 | 5-F | $CH_3$ | Cl | O | CH | |
| 503 | $CHF_2$ | 2 | 5-F | $OCH_3$ | $SCH_3$ | O | N | |
| 504 | $CHF_2$ | 2 | 5-F | $OCH_3$ | $-OCH(CH_3)_2$ | O | N | |
| 505 | $CHF_2$ | 2 | 5-F | $OCH_3$ | Cl | O | N | |
| 506 | $CHF_2$ | 2 | 5-F | $C_2H_5$ | $OCH_3$ | S | N | |
| 507 | $CHF_2$ | 2 | 5-F | $CH_3$ | Cl | S | N | |
| 508 | $CHF_2$ | 2 | 5-F | $CH_3$ | Cl | S | CH | |
| 509 | $CHF_2$ | 2 | 5-F | $C_2H_5$ | $CH_3$ | O | N | |
| 510 | $CHF_2$ | 2 | 5-F | $C_2H_5$ | $CH_3$ | O | CH | |
| 511 | $CHF_2$ | 2 | 5-F | $OCH_3$ | Cl | O | CH | Smp.160-161° |
| 512 | $CHF_2$ | 2 | 5-F | $OC_2H_5$ | Cl | O | N | |
| 513 | $CHF_2$ | 2 | 5-F | $CH_3$ | F | O | N | |
| 514 | $CHF_2$ | 2 | 5-F | $CH_3$ | Br | O | N | |
| 515 | $CHF_2$ | 2 | 5-F | $CH_3$ | Br | O | CH | |
| 516 | $CHF_2$ | 2 | 5-F | $CH_3$ | F | O | CH | |
| 517 | $CHF_2$ | 2 | 5-F | $C_2H_5$ | Cl | O | CH | |
| 518 | $CHF_2$ | 2 | 5-F | $CH_2F$ | $OCH_3$ | O | N | |
| 519 | $C_2F_5$ | 2 | 5-F | $CH_3$ | $OCH_3$ | O | N | |
| 520 | $C_2F_5$ | 2 | 5-F | $CH_3$ | $OCH_3$ | O | N | |
| 521 | $C_2F_5$ | 2 | 5-F | $CH_3$ | $OCH_3$ | O | CH | |
| 522 | $C_2F_5$ | 2 | 5-F | $OCH_3$ | $OCH_3$ | O | CH | |
| 523 | $C_2F_5$ | 2 | 5-F | $CH_3$ | Cl | O | CH | |
| 525 | $C_2F_5$ | 2 | 6-F | $CH_3$ | $OCH_3$ | O | N | |
| 526 | $C_2F_5$ | 2 | 6-F | $CH_3$ | $OCH_3$ | O | CH | |
| 527 | $C_2F_5$ | 2 | 6-F | $C_2H_5$ | $OCH_3$ | O | N | |
| 528 | $C_2F_5$ | 2 | 6-F | $CH_3$ | $OCH_3$ | S | N | |

**Tabelle 3** (fortsetzung)

| Nr. | A | Stellung von -X-A | $R_1$ | $R_3$ | $R_4$ | X | E |
|---|---|---|---|---|---|---|---|
| 529 | $C_2F_5$ | 2 | 6-F | $CH_3$ | $OCH_3$ | S | CH |
| 530 | $C_2F_5$ | 2 | 6-F | $C_2H_5$ | $OCH_3$ | S | N |
| 531 | $C_2F_5$ | 2 | 6-$COOCH_3$ | $CH_3$ | $OCH_3$ | O | N |
| 532 | $C_2F_5$ | 2 | 6-$COOCH_3$ | $OCH_3$ | $OCH_3$ | O | N |
| 533 | $C_2F_5$ | 2 | 6-$COOCH_3$ | $CH_3$ | $OCH_3$ | O | CH |
| 534 | $C_2F_5$ | 2 | 6-F | $C_2H_5$ | $OCH_3$ | O | N |
| 535 | $CHF_2$ | 2 | 6-F | $C_2H_5$ | $OCH_3$ | O | N |
| 536 | $CHF_2$ | 2 | 6-F | $CH_3$ | Cl | O | CH |
| 537 | $CHF_2$ | 2 | 6-F | $CH_3$ | Br | O | CH |
| 538 | $CHF_2$ | 2 | 6-F | $OCH_3$ | $-OCH(CH_3)_2$ | O | N |
| 539 | $CHF_2$ | 2 | 6-F | $OCH_3$ | Cl | O | N |
| 540 | $CHF_2$ | 2 | 6-F | $-CH(CH_3)_2$ | $OCH_3$ | O | N |
| 541 | $CHF_2$ | 2 | 6-F | $C_2H_5$ | $OCH_3$ | O | CH |
| 542 | $CHF_2$ | 2 | 6-F | $C_2H_5$ | Cl | O | CH |
| 543 | $CHF_2$ | 2 | 6-F | $CH_3$ | $SCH_3$ | O | N |
| 544 | $CHF_2$ | 2 | 6-F | $OCH_3$ | $SCH_3$ | O | N |
| 545 | $CHF_2$ | 2 | 6-F | $-OCH(CH_3)_2$ | Cl | O | N |
| 546 | $CHF_2$ | 2 | 6-F | $C_2H_5$ | $CH_3$ | O | N |
| 547 | $CHF_2$ | 2 | 6-F | $-CH(CH_3)_2$ | $CH_3$ | O | N |
| 548 | $CHF_2$ | 2 | 6-F | $CH_2F$ | $OCH_3$ | O | N |
| 549 | $CHF_2$ | 4 | 6-F | $C_2H_5$ | $OCH_3$ | S | N |
| 550 | $CHF_2$ | 2 | 6-F | $CH_3$ | Cl | S | CH |
| 551 | $CHF_2$ | 2 | 6-F | $CH_3$ | Br | S | CH |
| 552 | $CHF_2$ | 2 | 6-F | $OCH_3$ | Cl | S | CH |
| 553 | $CHF_2$ | 2 | 6-F | $C_2H_5$ | $CH_3$ | S | CH |
| 554 | $CHF_2$ | 2 | 5 Cl | $C_2H_5$ | $OCH_3$ | O | N |
| 555 | $CHF_2$ | 2 | 5 Cl | $-CH(CH_3)_2$ | $OCH_3$ | O | N |
| 556 | $CHF_2$ | 2 | 5 Cl | $CH_3$ | Cl | O | CH |
| 557 | $CHF_2$ | 2 | 5 Cl | $OCH_3$ | Cl | O | CH |
| 558 | $CHF_2$ | 2 | 5 Cl | $-OCH(CH_3)_2$ | $OCH_3$ | O | N |
| 559 | $CHF_2$ | 2 | 5 Cl | $-OCH_3$ | $SCH_3$ | O | CH |

Tabelle 3 (fortsetzung)

| Nr. | A | Stellung von-X-A | $R_1$ | $R_3$ | $R_4$ | X | E | phys. Daten |
|---|---|---|---|---|---|---|---|---|
| 560 | $CHF_2$ | 2 | 5-Cl | $C_2H_5$ | $OCH_3$ | O | N | Smp. 130-132° |
| 561 | $CHF_2$ | 2 | 5-Cl | $-CH(CH_3)_2$ | $OCH_3$ | O | N | Smp. 149-151° |
| 562 | $CHF_2$ | 2 | 5-Cl | $CH_3$ | Cl | O | CH | Smp.182° (Zers.) |
| 563 | $CHF_2$ | 2 | 5-Cl | $OCH_3$ | Cl | O | CH | Smp.170-172° |
| 564 | $CHF_2$ | 2 | 5-Cl | $-OCH(CH_3)_2$ | $OCH_3$ | O | N | Smp.128-135° |
| 565 | $CHF_2$ | 2 | 5-Cl | $OCH_3$ | $SCH_3$ | O | CH | |

Tabelle 4

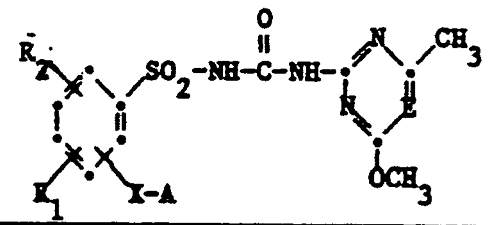

| Nr. | A | Stellung von -X-A | $R_1$ | $R_2$ | X | E | Phys. Daten |
|---|---|---|---|---|---|---|---|
| 601 | $CHF_2$ | 3 | $6-NO_2$ | 3-Br | S | N | |
| 602 | $CF_3$ | 3 | $6-COOC_2H_5$ | 3-Br | O | N | |
| 603 | $CF_3$ | 3 | $6-COOC_2H_5$ | 3-Br | S | N | |
| 604 | $CF_3$ | 3 | $6-CH_3$ | 2-Cl | $SO_2$ | CH | |
| 605 | $CHF_2$ | 3 | $6-CH_3$ | 2-Cl | $SO_2$ | CH | |
| 606 | $CHF_2$ | 3 | $2-NO_2$ | 2-Cl | O | N | |
| 607 | $CHF_2$ | 3 | $2-NO_2$ | 2-Cl | O | CH | |
| 608 | $CHF_2$ | 3 | $2-COOC_2H_5$ | 2-Cl | O | N | |
| 609 | $CHF_2$ | 3 | $2-COOC_2H_5$ | 2-Cl | S | N | |
| 610 | $CHF_2$ | 3 | 6-Cl | 2-Cl | O | CH | |
| 611 | $CHF_2$ | 3 | 6-Cl | 2-Cl | S | CH | |
| 612 | $CHF_2$ | 3 | 6-Cl | 2-Cl | SO | N | |
| 613 | $CHF_2$ | 3 | 6-Cl | 2-Cl | $SO_2$ | N | |
| 614 | $CHF_2$ | 3 | $2-CF_3$ | 2-Cl | O | N | |
| 615 | $CHF_2$ | 3 | $5-NO_2$ | 2-Cl | O | N | Smp. 170-172°C |
| 616 | $CHF_2$ | 3 | $6-OCH_3$ | 2-Cl | O | CH | |
| 617 | $CHF_2$ | 3 | $6-OCH_3$ | 2-Cl | O | N | |
| 618 | $CHF_2$ | 3 | $6-SCH_3$ | 2-Cl | O | CH | |
| 619 | $CHF_2$ | 3 | $6-SCH_3$ | 2-Cl | O | N | |
| 620 | $CHF_2$ | 3 | $2-SO_2CH_3$ | 2-Cl | O | CH | |
| 621 | $CHF_2$ | 3 | $2-SO_2CH_3$ | 2-Cl | O | N | |

Tabelle 5

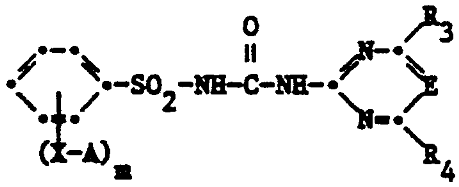

| Nr. | A | X | m | Stellungen von X-A | E | $R_3$ | $R_4$ | Phys. Daten |
|---|---|---|---|---|---|---|---|---|
| 701 | $CHF_2$ | O | 2 | 2,5 | N | $CH_3$ | $OCH_3$ | Smp.113° |
| 702 | $CHF_2$ | S | 2 | 2,5 | N | $CH_3$ | $OCH_3$ | |
| 703 | $CHF_2$ | O | 2 | 2,6 | N | $CH_3$ | $OCH_3$ | |
| 704 | $CHF_2$ | O | 2 | 2,5 | CH | $CH_3$ | $OCH_3$ | |
| 705 | $CHF_2$ | O | 2 | 2,5 | CH | Cl | $CH_3$ | |
| 706 | $CHF_2$ | O | 2 | 2,5 | CH | Cl | $OCH_3$ | |
| 707 | $CHF_2$ | O | 2 | 2,5 | N | $C_2H_5$ | $OCH_3$ | |
| 708 | $CHF_2$ | O | 2 | 2,5 | N | $OCH_3$ | $OCH_3$ | |
| 709 | $-CF_2-CF_2H$ | O | 2 | 2,5 | N | $CH_3$ | $OCH_3$ | |
| 710 | $CF_3$ | O | 2 | 2,5 | N | $CH_3$ | $OCH_3$ | |

Tabelle 6

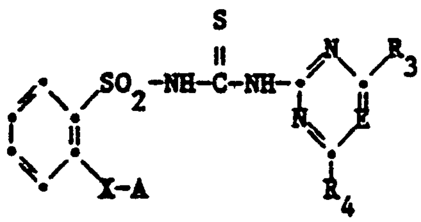

| Nr. | A | E | $R_3$ | $R_4$ | X |
|---|---|---|---|---|---|
| 801 | $CHF_2$ | N | $C_2H_5$ | $OCH_3$ | O |
| 802 | $CHF_2$ | CH | $C_2H_5$ | $OCH_3$ | O |
| 803 | $CHF_2$ | N | $OCH_3$ | $-OCH(CH_3)_2$ | O |
| 804 | $CHF_2$ | N | $-CH(CH_3)_2$ | $OCH_3$ | O |
| 805 | $CHF_2$ | N | $C_2H_5$ | $OCH_3$ | S |
| 806 | $CHF_2$ | CH | $C_2H_5$ | $OCH_3$ | S |
| 807 | $CHF_2$ | N | $C_2H_5$ | $-OCH(CH_3)_2$ | S |
| 808 | $CHF_2$ | N | $-CH(CH_3)_2$ | $OCH_3$ | S |
| 809 | $C_2F_5$ | N | $CH_3$ | $OCH_3$ | O |
| 810 | $C_2F_5$ | N | $CH_3$ | $OCH_3$ | S |
| 811 | $-CF_2-CHF_2$ | N | $C_2H_5$ | $OCH_3$ | O |
| 812 | $-CF_2-CHF_2$ | N | $-OCH(CH_3)_2$ | $OCH_3$ | O |

**Formulierungsbeispiele**

**Beispiel 7:**

(% = Gewichtsprozent)

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einergeeignete Mühle gut vermahlen. Man erhält Spritzpulver, die sich mit Wasser zu Suspensionen jeden gewünschten Konzentration verdünnen lassen.

| b) Emulsions-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeden gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mitdem Träger vermischt und auf einer geeigmetem Mühle vermahlen wird.

d) Extruder Granulat

| | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200 | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (7-8 Mol AeO) | 3 % |
| Wasser | 91 % |

**Biologische Beispiele**

**Beispiel 8:**

Nachweis der Herbizidwirkung vo den Auflaufen der Pflanzen
Im Gewächshaus werden Pflanzensamen in Blumentöpfe von 12-15 cm Durchmesser gesät. Unmittelbar danach wird die Erdoberfläche mit einer wässrigen Dispersion oder Lösung der Wirkstoffe behandelt. Es werden Konzentrationen von 4 kg Wirkstoffnengen pro Hektar angewendet. Die Töpfe werden dann im Gewächshaus bei einer Temperatur von 22-25°C und 50-70% relativer Luftfeuchtigkeit gehalten. Nach 3 Wochen wird der Versuch ausgewertet und die Wirkung nach folgendem Masstab beurteilt:

1: Pflanzen nicht gekeimt oder total abgestorben,
2-3: sehr starke Wirkung,
4-6: mittlere Wirkung,
7-8: geringe Wirkung,
9: keine Wirkung (wie unbehandelte Kontrolle).

**Pre-emergente Wirkung**

Aufwandmenge: 4 kg Wirksubstanz/Hektar

| Verb. Nr. | Avena | Setaria | Sinapis | Stellaria |
|---|---|---|---|---|
| 1 | 2 | 1 | 2 | 1 |
| 2 | 6 | 4 | 2 | 2 |
| 19 | 2 | 1 | 2 | 1 |
| 261 | 6 | 3 | 2 | 2 |
| 302 | 4 | 1 | 2 | 2 |
| 303 | 3 | 1 | 2 | 2 |
| 312 | 7 | 3 | 2 | 2 |
| 323 | 3 | 2 | 2 | 2 |
| 342 | 5 | 4 | 2 | 2 |
| 344 | 4 | 2 | 2 | 2 |
| 345 | 3 | 1 | 2 | 2 |
| 352 | 4 | 2 | 2 | 2 |
| 701 | 2 | 1 | 2 | 1 |

**Beispiel 9:**

Nachweis der Selektivität bei Vorauflaufanwendung
In der gleichen Versuchsanordnung wie im Beispiel 9 werden eine grössere Anzahl von Pflanzensamen mit verschiedenen Aufwandmengen an Wirksubstanz behandelt. Die Auswertung erfolgte nach dem gleichen Maßstab.

**Versuchergebnisse** (pre-emergente)

| Wirkung<br>Aufwandmenge<br>kg AS/ha | Verb.Nr. 19 | | | | Verb. Nr. 24 | | | |
|---|---|---|---|---|---|---|---|---|
| Testpflanze | 0.25 | 0.12 | 0.06 | 0.03 | 0.25 | 0.12 | 0.06 | 0.03 |
| Weizen | 5 | 6 | 7 | 9 | 9 | 9 | 9 | 9 |
| Mais | 3 | 3 | 7 | 8 | 9 | 9 | 9 | 9 |
| Avena fatua | 5 | 6 | 6 | 8 | 9 | 9 | 9 | 9 |
| Alopecurus myos. | 2 | 2 | 2 | 3 | 4 | 9 | 9 | 9 |
| Echinochloa c.g. | 2 | 2 | 3 | 3 | 6 | 6 | 9 | 9 |
| Rottboellia ex. | 3 | 3 | 4 | 4 | 7 | 9 | 9 | 9 |
| Cyperus escul. | 2 | 3 | 3 | 5 | 3 | 4 | 9 | 9 |
| Soja | 2 | 2 | 3 | 3 | 4 | 4 | 8 | 9 |
| Baumwolle | 1 | 2 | 2 | 2 | 2 | 2 | 6 | 6 |
| Abutilon | 1 | 1 | 1 | 1 | 1 | 1 | 2 | 2 |
| Xanthium Sp. | 2 | 2 | 2 | 3 | 2 | 2 | 9 | 9 |
| Amaranthus ret. | - | - | - | - | 3 | 3 | 3 | 3 |
| Chenopodium Sp. | 2 | 2 | 2 | 2 | 2 | 2 | 2 | 2 |
| Solanum nigrum | - | - | - | - | 3 | 3 | 3 | 3 |
| Ipomosa | 2 | 2 | 2 | 2 | 2 | 2 | 4 | 7 |
| Sinapis | 1 | 1 | 1 | 1 | 1 | 2 | 6 | 6 |
| Stellaria | - | - | - | - | 2 | 2 | 2 | 2 |
| Chrysanth.leuc. | - | - | - | - | 2 | 2 | 2 | 2 |
| Galium aparine | 2 | 2 | 3 | 3 | 5 | 6 | 6 | 9 |
| Viola tricolor | 1 | 1 | 1 | 1 | 2 | 2 | 2 | 2 |
| Veronica Sp. | - | - | - | - | 1 | 1 | 1 | 1 |

| Wirkung Aufwandmenge kg AS/ha | Verb. Nr. 261 | | | | Verb. Nr. 701 | | | |
|---|---|---|---|---|---|---|---|---|
| Testpflanze | 0.25 | 0.12 | 0.06 | 0.03 | 0.25 | 0.12 | 0.06 | 0.03 |
| Weizen | 7 | 8 | 9 | 9 | 2 | 3 | 6 | 9 |
| Mais | 9 | 9 | 9 | 9 | 1 | 2 | 3 | 5 |
| Avena fatua | 7 | 8 | 8 | 9 | 2 | 2 | 3 | 4 |
| Alopecurus myos. | 4 | 4 | 5 | 7 | 2 | 2 | 2 | 2 |
| Echinochloa c.g. | 2 | 3 | 3 | 3 | 2 | 2 | 2 | 2 |
| Rottboellia ex. | 4 | 4 | 7 | 7 | 2 | 3 | 3 | 3 |
| Cyperus escul. | 1 | 2 | 3 | 3 | 3 | 7 | 7 | 7 |
| Soja | 6 | 9 | 9 | 9 | 2 | 2 | 3 | 3 |
| Baumwolle | 4 | 4 | 4 | 9 | 2 | 4 | 6 | 7 |
| Abutilon | 1 | 2 | 4 | 4 | 3 | 3 | 4 | 4 |
| Xanthium Sp. | 4 | 6 | 6 | 8 | 1 | 1 | 2 | 2 |
| Chenopodium Sp. | 2 | 2 | 3 | 3 | 1 | 1 | 2 | 2 |
| Ipomoea | 2 | 2 | 3 | 4 | 2 | 2 | 2 | 3 |
| Sinapis | 1 | 1 | 2 | 3 | 2 | 2 | 2 | 3 |
| Galium aparine | 2 | 3 | 3 | 3 | 2 | 2 | 4 | 6 |
| Viola tricolor | 2 | 2 | 2 | 2 | 1 | 2 | 2 | 3 |

**Beispiel 10:**

Nachweis der Herbizidwirkung nach dem Auflaufen der Pflanzen (Kontaktwirkung)

Eine Anzahl Unkräuter und Kulturpflanzen, sowohl monocotyle wie dicotyle, wurden nach dem Auflaufen, im 4- bis 6-Blattstadium mit einer wässrigen Wirkstoffdispersion in Dosierungen von 4 kg AS/ha gespritzt und dann bei 24° bis 26°C und 45-60% relativer Luftfeuchtigkeit gehalten. 15 Tage nach der Behandlung wird der Versuch ausgewertet und die Wirkung nach dem gleichen Maßstab wie im Beispiel 9 bewertet.

**Post-emergente Wirkung**

Aufwandmange: 4 kg Wirksubstanz/Hektar

| Verb. Nr. | Avena | Setaria | Lolium | Solanum | Sinapis | Stellaria | Phaseolus |
|---|---|---|---|---|---|---|---|
| 1 | 2 | 4 | 3 | 2 | 2 | 3 | 3 |
| 2 | 7 | 7 | 3 | 2 | 2 | 3 | 5 |
| 19 | 3 | 4 | 2 | 2 | 2 | 4 | 2 |
| 261 | 7 | 6 | 4 | 1 | 2 | 3 | 4 |
| 302 | 3 | 3 | 3 | 2 | 3 | 4 | 3 |
| 303 | 4 | 2 | 4 | 3 | 2 | 2 | 4 |
| 312 | 7 | 6 | 4 | 6 | 3 | 7 | 7 |
| 323 | 3 | 4 | 4 | 1 | 2 | 2 | 3 |
| 342 | 6 | 5 | 3 | 3 | 1 | 2 | 3 |
| 344 | 4 | 4 | 4 | 2 | 2 | 2 | 4 |
| 345 | 5 | 4 | 3 | 3 | 2 | 4 | 5 |
| 352 | 6 | 5 | 4 | 3 | 2 | 4 | 4 |
| 701 | 2 | 3 | 3 | 2 | 2 | 3 | 3 |

**Beispiel 11:**

Nachweis der Selektivität bei Nachauflaufanwendung

In der gleichen Versuchsanordnung wie im Beispiel 10 werden eine grössere Anzahl von Pflanzen mit verschiedenen Aufwandmengen an Wirksubstanz behandelt. Die Auswertung erfolgte nach dem in Beispiel 9 angegebenen Maßstab.

**Versuchsergebnisse** (postemergent)

| Wirkung<br>Aufwandmenge<br>kg AS/ha | Verb. Nr. 24 | | | | Verb. Nr. 701 | | | |
|---|---|---|---|---|---|---|---|---|
| Testpflanze | 0.25 | 0.12 | 0.06 | 0.03 | 0.25 | 0.12 | 0.06 | 0.03 |
| Gerste | 9 | 9 | 9 | 9 | - | - | - | - |
| Weizen | 9 | 9 | 9 | 9 | 4 | 6 | 9 | 9 |
| Mais | 9 | 9 | 9 | 9 | 2 | 4 | 4 | 8 |
| Reis trocken | 4 | 4 | 6 | 9 | 7 | 7 | 7 | 9 |
| Avena fatua | - | - | - | - | 2 | 3 | 4 | 6 |
| Alopecurus myos. | 7 | 9 | 9 | 9 | 2 | 3 | 3 | 3 |
| Echinochloa c.g. | - | - | - | - | 2 | 2 | 3 | 3 |
| Sorghum halep. | 3 | 3 | 9 | 9 | - | - | - | - |
| Rottboellia ex. | - | - | - | - | 4 | 4 | 7 | 9 |
| Soja | 1 | 2 | 3 | 3 | 4 | 4 | 6 | 6 |
| Baumwolle | 7 | 7 | 7 | 7 | 9 | 9 | 9 | 9 |
| Xanthium Sp. | 1 | 1 | 1 | 2 | 1 | 1 | 2 | 3 |
| Amarantus ret. | 2 | 2 | 3 | 3 | - | - | - | - |
| Chenopodium Sp. | 3 | 3 | 4 | 4 | 3 | 3 | 4 | 4 |
| Sinapis | 3 | 3 | 3 | 3 | 2 | 2 | 2 | 2 |
| Stellaria | 4 | 4 | 4 | 4 | - | - | - | - |
| Chrysanth.leuc. | 3 | 3 | 3 | 3 | - | - | - | - |
| Galium aparine | - | - | - | - | 2 | 2 | 4 | 4 |
| Viola tricolor | - | - | - | - | 2 | 2 | 2 | 2 |

**Beispiel 12:**

Nachweis der Keimung an Lagerkartoffeln.

Eine Anzahl im Handel erhältliche Kartoffeln der Sorte "Urgenta" ohne Keime werden gewaschen und abgetrocknet. Danach werden die Kartoffeln für jeweils eine Minute in Wirkstoffemulsionen verschiedener Konzentration getaucht, in Kunststoffschalen auf Filterpapier ausgelegt und bei Temperaturen von 14° und 21°C im Dunkeln bei 50% relativer Luftfeuchtigkeit gehalten. Die Auswertung erfolgte 34 Tage nach der Applikation. Gleichzeitig wird der Gewichtsverlust der Knollen und das Gewicht der Keime im Vergleich zur unbehandelten Kontrolle ermittelt.

Einige der Verbindungen der Formel I zeigten in diesem Versuch eine vollständige Verhinderung der Keimbildung. Gleichzeitig betrug der Gewichtsverlust der Kartoffeln weniger als 10 % des Gewichtsverlustes der Kontrollkartoffeln.

**Beispiel 13:**

Nachweis der Wuchshemmung bei tropischen Bodenbedeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet.

In diesem Versuch zeigten die mit einigen der Wirkstoffe der Formel 1 behandelten Pflanzen eine deutliche Reduktion des Neuzuwachses (weniger als 20 %), ohne dass dabei die Versuchspflanzen geschädigt werden.

**Patentansprüche**

für die Vertragsstaaten BE DE FR GB IT NL SE

1. N-Phenylsulfonyl-N'-pyrimidinyl- und triazinyl-harnstoffe der allgemeinen Formel I

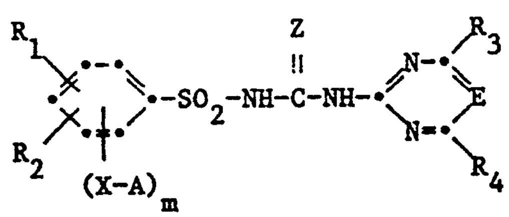

(I)

worin

A einen $C_1$-$C_6$-Halogenalkylrest

E die Methingruppe oder Stickstoff,

X Sauerstoff, Schwefel, eine Sulfinyl- oder Sulfonylbrücke,

Z Sauerstoff oder Schwefel,

m die Zahl ein oder zwei,

$R_1$ Wasserstoff, Halogen, C-C-Alkyl, C-C-Alkenyl oder einen Rest -Y-R5,

$R_2$ Wasserstoff, Halogen, $C_1$-$C_5$-Alkyl, $C_2$-$C_5$-Alkenyl, $C_1$-$C_4$-Halogen-alkyl, oder einen Rest -Y-$R_5$, -$COOR_6$, -$NO_2$ oder -CO-$NR_7R_8$,

$R_3$ und $R_4$ unabhängig voneinander Wasserstoff $C_1$-$C_4$-Alkyl, $C_1$-$C_4$-Alkoxy, $C_1$-$C_4$-Alkylthio, $C_1$-$C_4$-Halogenalkyl, Halogen oder Alkoxy-alkyl mit höchstens 4 Kohlenstoffatomen,

$R_5$ und $R_6$ je $C_1$-$C_5$-Alkyl, $C_2$-$C_5$-Alkenyl oder $C_2$-$C_5$-Alkinyl,

$R_7$ und $R_8$ unabhängig voneinander Wasserstoff, $C_1$-$C_5$-Alkyl, $C_2$-$C_5$-Alkenyl oder $C_2$-$C_6$-Alkinyl und

Y Sauerstoff, Schwefel, eine Sulfinyl- oder Sulfonylbrücke bedeuten, sowie die Salze dieser Verbindungen, mit der Massgabe, dass

m die Zahl zwei bedeutet, wenn gleichzeitig A für -$CHF_2$, -$CF_3$, -$CH_2$-$CF_3$, -$CF_2$-$CHF_2$, -$CF_2$-CHFCl, -$CF_2$-CHFBr oder -$CF_2$-CHF-$CF_3$; $R_3$ für -$OCH_3$ oder -$CH_3$; $R_4$ für -$OCH_3$, -$CH_3$, -$OC_2H_5$, -$CH_2$-O-$CH_3$ oder -$CH_2$-$CH_2$-$OCH_3$ stehen und der Phenylkern nicht oder in 2-, 3-, 5- oder 6-Stellung zur Sulfonylgruppe nur durch einen weiteren Substituenten aus der Gruppe F, Cl, Br oder -$CH_3$ substituiert ist oder wenn gleichzeitig X Sauerstoff, A Trifluormethyl, $R_1$ Wasserstoff, $R_2$ $C_2$-$C_5$-Alkenyl in 2-Stellung, $R_3$ Wasserstoff, Chlor, Brom, Methyl, Aethyl, $C_1$-

$C_3$-Alkoxy, Trifluormethyl, Methylthio oder Methoxymethyl und $R_4$ Methyl oder Methoxy bedeuten, oder wenn gleichzeitig X Sauerstoff, A Trifluormethyl, $R_1$ Wasserstoff, $R_2$ Chlor in 2-Stellung, $R_3$ Wasserstoff, Chlor, Brom, Methyl, Aethyl, Trifluormethyl, Methoxy, Methoxymethyl oder Methoxyäthyl bedeuten und der Trifluormethoxyrest die 5-Stellung am Phenylring einnimmt.

2. Verbindungen gemäss Anspruch 1, dsdurch gekennzeichnet, dass Z Sauerstoff bedeutet.

3. Verbindungen gemäss Anspruch 1, dadurch gekennzeichnet, dass die Reste $R_3$ und $R_4$ zusammen nicht mehr als 4 Kohlenstoffatome enthalten.

4. Verbindungen gemäss Anspruch 1, dadurch gekennzeichnet, dass Z Sauerstoff und m die Zahl eins bedeuten.

5. Verbindungen gemäss Anspruch 1, dadurch gekennzeichnet, dass Z Sauerstoff und m die Zahl zwei bedeutet.

6. Verbindungen gemäss Anspruch 4, dadurch gekennzeichnet dass der Rest -X-A in 2- oder 3-Position zur Sulfonylgruppe steht.

7. Verbindungen genäss Anspruch 6, dadurch gekennzeichnet, dass der Rest -X-A in der 2-Position steht.

8. Verbindungen gemäss Anspruch 5, dadurch gekennzeichnet, dass die beiden Reste -X-A in 2- und in 5-Stellung zur Sulfonylgruppe stehen.

9. Verbindungen gemäss Anspruch 2, dadurch gekennzeichnet, dass nur ein Rest -X-A in der 2-Stellung zum Sulfonylrest vorhanden ist und die Reste $R_3$ und $R_4$ zusammen nicht mehr als 4 Kohlenstoffatome enthalten.

10. Verbindungen gemäss Anspruch 2, dadurch gekennzeichnet, dass zwei Reste -X-A in 2- und 5-Stellung zum Sulfonylrest vorhanden sind und die Reste $R_3$ und $R_4$ zusammen nicht mehr als 4 Kohlenstoffatome enthalten.

11. Verbindungen gemäss Anspruch 10, dadurch gekennzeichnet, dass A für $-CHF_2$, $-CF_3$ oder $-CH_2-CH_2Cl$ steht.

12. Verbindungen gemäss Anspruch 9, dadurch gekennzeichnet, dass $R_1$ Wasserstoff bedeutet, und der Rest $R_2$ in der 5- oder 6-Stellung zur Sulfonylgruppe steht.

13. Verbindungen gemäss Anspruch 12, dadurch gekennzeichnet, dass $R_2$ für Wasserstoff, Fluor, Nitro oder $COOR_6$ steht.

14. Verbindungen gemäss Anspruch 13, dadurch gekennzeichnet, dass X für Sauerstoff oder Schwefel steht.

15. Verbindungen gemäss Anspruch 14, dadurch gekennzeichnet, dass $R_2$ Wasserstoff, $R_3$ Chlor, Fluor, Methyl, Chlormethyl, Aethoxy, i-Propyloxy, Aethyl oder Methoxy und $R_4$ Methyl, Aethyl, Methoxy oder Aethoxy bedeuten.

16. Verbindungen gemäss Anspruch 15, dadurch gekemnzeichnet, dass X Sauerstoff ist.

17. Verbindungen gemäss Anspruch 15, dadurch gekennzeichnet, dass der Rest A durch $-CF_2G$ verkörpert ist, worin G für Wasserstoff, F, Cl, Br, $CF_3$ oder $CH_3$ steht.

1 18. Verbindungen gemäss Anspruch 16, dadurch gekennzeichnet, dass G für Chlor, Brom oder $CF_3$ steht.

19. Verbindungen gemäss Anspruch 15, dadurch gekennzeichnet, dass A für $-CH_2F$, $-CH_2-CH_2F$ und $-CH_2-CH_2Cl$ steht.

20. Verbindungen gemäss Anspruch 1, dadurch gekennzeichnet, dass Z Schwefel, X Sauerstoff oder Schwefel und $R_3$ und $R_4$ jeweils unabhängig voneinander $C_1$-$C_3$-Alkyl, $C_1$-$C_3$-Alkoxy oder $C_1$-$C_3$-Alkylthio mit zusammen höchstens 4 Kohlenstoffatomen bedeuten, A für $-CHF_2$, $-CF_3$, $-C_2F_5$ und $-CF_3$, $-C_2F_5$ und $-CF_2-CHF_2$ steht, der Rest -X-A die 2-Stellung besetzt und m die Zahl 1 bedeutet.

21. N-(2-Difluormethoxyphenyl-sulfonyl)-N'-(4-äthyl-6-methoxy-1, 3, 5-triazin-2-yl)-harnstoff gemäss Anspruch 1.

22. N-(2-Difluormethoxyphenyl-sulfonyl)-N'-(4,6-diäthoxy-1, 3, 5-triazin-2-yl)-harnstoff gemäss Anspruch 1.

23. N-(2-Difluormethoxyphenyl-sulfonyl)-N'-(4-chlor-6-methyl-pyrimidin-2-yl)-harnstoff gemäss Anspruch 1.

24. N-(2-Pentafluoräthoxyphenyl-sulfonyl)-N'-(4-methoxy-6-methyl-1,3, 5-triazin-2-yl)-harnstoff gemäss Anspruch 1.

25. N-(2-Difluormethylthiophenyl-sulfonyl)-N'-(4-chlor-6-methoxy-pyrimidin-2-yl)-harnstoff gemäss Anspruch 1.

26. N-[2,5-Bis-(difluormethoxy)-phenyl-sulfonyl]-N'-(4-methoxy-6-methyl-1, 3, 5-triazin-2-yl)-harnstoff gemäss Anspruch 1.

27. N-(2-Difluormethoxyphenyl-sulfonyl)-N'-(4-methoxy-6-isopropyloxy-1, 3, 5-triazin-2-yl)-harnstoff gemäss Anspruch 1.

28. N-(2-Difluormethylthiophenyl-sulfonyl)-N'-(4-äthyl-6-methoxy-1, 3, 5-triazin-2-yl)-harnstoff gemäss Anspruch 1.

29. N-(2-Difluormethoxyphenyl:sulfonyl)-N'-(4-chlor-6-methoxy-pyrimidin-2-yl)-harnstoff gemäss Anspruch 1.

30. Verfahren zur Herstellung der Verbindungen der Formel I, Anspruch 1, dadurch gekennzeichnet, dass man ein Phenylsulfonamid det Formel II

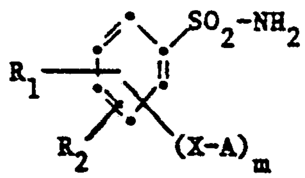

(II)

worin A, $R_1$, $R_2$, X und m die unter Formel I gegebene Bedeutung haben, in Gegenwart einer Base mit einem N-Pyrimidinyl- oder Triazinylcarbamat der Formel

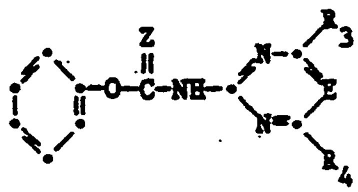

(III)

worin E, $R_3$, $R_4$ und Z die unter Formel I gegebene Bedeutung haben, umsetzt und gegebenenfalls in ihre Salze überführt.

31. Verfahren zur Herstellung der Verbindungen der Formel I, Anspruch 1, dadurch gekennzeichnet, dass man ein Phenylsulfonylisocyanat oder -isothiocyanat der Formel IV

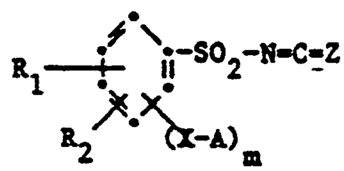

(IV)

worin A, $R_1$, $R_2$, m, X und 2 die unter Formel I gegebene Bedeutung haben, gegebenenfalls in Gegenwart einer Base, mit einem Amin der Formel V

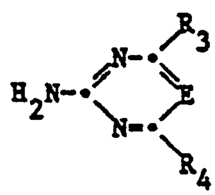

(V)

worin E, $R_3$ und $R_4$ die unter Formel I gegebene Bedeutung haben, umsetzt und gegebenenfalls in ihre Salze überführt.

32. Verfahren zur Herstellung der Verbindungen der Formel I, Anspruch 1, dadurch gekennzeichnet, dass man Sulfonamide der oben angegebenen Formel II gegebenenfalls in Gegenwart einer Base mit einem Isocyanat oder Isothiocyanat der Formel VI

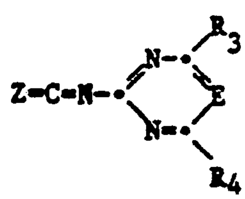

(VI)

worin E, $R_3$, $R_4$ und Z die unter Formel I gegebene Bedeutung haben, umsetzt und gegebenenfalls in ihre Salze überführt.

33. Verfahren zur Herstellung der Verbindungen der Formel I, Anspruch 1, dadurch gekennzeichnet, dass man ein N-Phenylsulfonylcarbamat der formel VII

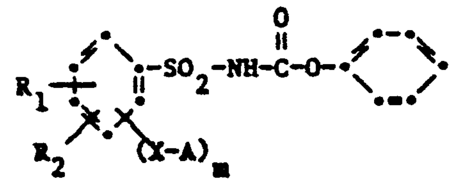

(VII)

worin A, $R_1$, $R_2$, m und X die unter Formel 1 gegebene Bedeutung haben, mit einem Amin der oben angegebenen Formel V umsetzt und gegebenenfalls in ihre Salze überführt.

34. Verfahren zur Herstellung von basischen Additionssalzen der Formel I gemäss einem der Ansprüche 30 bis 33, dadurch gekennzeichnet, dass man einen Sulfonylharnstoff der Formel 1 mit einem Amin, einem Alkalimetall- oder Erdalkalimetallhydroxid oder einer quaternären Ammoniumbase umsetzt.

35. Phenylsulfonamide der Formel II

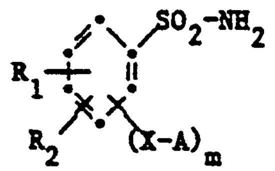

worin A einen $C_1$-$C_6$-Halogenalkylrest, mit Ausnahme der Reste $CHF_2$, $-CF_3$, $-CH_2-CF_3$, $-CF_2-CF_2H$, $-CF_2-CClFH$, $-CF_2-CClBrH$ und $-CF_2-CFH-CF_3$, bedeutet und X, $R_1$ und $R_2$ die unter Formel I gegebenen Bedeutungen haben, wobei A dann auch für $-CHF_2$ oder $-CF_3$ stehen kann, wenn nicht gleichzeitig $R_1$ Wasserstoff und $R_2$ Wasserstoff, $C_2$-$C_5$-Alkenyl, Fluor, Chlor, Brom oder Methyl bedeuten.

36. Verfahren zur Herstellung der Phenylsulfonanide gemäss Anspruch 35, dadurch gekennzeichnet, dass man durch Diazotierung und Austausch der Diazogruppe mit Schwefeldioxid in Gegenwart eines Katalysators aus einem entsprechenden Anilin das Phenylsulfonylchlorid herstellt und dieses durch Umsetzen mit Ammoniumhydroxid-Lösung in das Sulfonanid überführt.

37. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

38. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

39. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

40. Die Verwendung der N-Phenylsulfonyl-N'-triazinyl- oder -pyrimidinyl-harnstoffe der Formel 1, oder sie enthaltender Mittel, gemäss Anspruch 38, zur selektiven pre- oder postemergenten Bekämpfung von Unkräutern in Nutzpflanzenkulturen.

41. Die Verwendung der N-Phenylsulfonyl-N'-triazinyl- oder -pyrimidinyl-harnstoffe der Formel I, oder sie enthaltender Mittel, gemäss Anspruch 39, zur Unterdrückung des Pflanzenwachstums über das 2-Blatt-stadium hinaus, dadurch gekennzeichnet, dass die Wirkstoffe pre-emergent angewendet werden.

42. N-(2-pentafluoräthoxy-phenylsulfonyl)-N'-(4,6-dimethoxy-1, 3, 5-triazin-2-yl)-harnstoff gemäss Anspruch 1.

43. N-[2-(2-Chloräthoxy)-phenylsulfonyl]-N'-(4-methoxy-6-methyl-1, 3, 5-triazin-2-yl)-harnstoff gemäss Anspruch 1.

44. N[2 (2 Chloräthoxy)-phenylsulfonyl]-N'-(4,6-dimethoxy-1, 3, 5-triazin-2-yl)-harnstoff gemäss Anspruch 1.

45. N-(2-pentafjuoräthoxy-phenylsulfonyl)-N'-(4-methoxy-6-methyl pyrimidin-2-yl)-harnstoff gemäss Anspruch 1.

46. N(2-pentafluoräthoxy-phenylsulfonyl)-N'-(4, 6-dimethyl-pyrimidin-2-yl)-harnstoff gemäss Anspruch 1.

**Patentansprüche**

für den Vertragsstaat AT

1. Ein herbizides und den pflanzenwuchs hemmendes Mittel, dadurch gekennzeichnet, dass es neben Träger- und/oder anderen Zuschlagstoffen als Wirkstoff mindestens einen N-Phenylsulfonyl-N'-pyrimidinylund triazinyl-harnstoff der allgemeinen Formel I

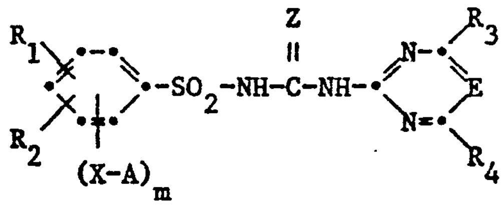

(I)

oder ein Salz davon enthält, worin
A einen $C_1$-$C_6$-Halogenalkylrest
E die Methingruppe oder Stickstoff,
X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$-$C_5$-Alkyl, $C_2$-$C_5$-Alkenyl oder einen Rest -Y-$R_5$,
$R_2$ Wasserstoff, Halogen, $C_1$-$C_5$-Alkyl, $C_2$-$C_5$-Alkenyl, $C_1$-$C_4$-Halogen-alkyl, oder einen Rest -Y-$R_5$, -$COOR_6$, -$NO_2$ oder -CO-$NR_7R_8$,
$R_3$ und $R_4$ unabhängig voneinander Wasserstoff, $C_1$-$C_4$-Alkyl, $C_1$-$C_4$-Alkoxy, $C_1$-$C_4$-Alkylthio, $C_1$-$C_4$-Halogenalkyl, Halogen oder Alkoxyalkyl mit höchstens 4 Kohlenstoffatomen,
$R_5$ und $R_6$ je $C_1$-$C_5$-Alkyl, $C_2$-$C_5$-Alkenyl oder $C_2$-$C_6$-Alkinyl,
$R_7$ und $R_8$ unabhängig voneinander Wasserstoff, $C_1$-$C_5$-Alkyl, $C_2$-$C_5$-Alkenyl oder $C_2$-$C_6$-Alkinyl und
Y Sauerstoff, Schwefel, eine Sulfinyl- oder Sulfonylbrücke bedeuten, sowie die Salze dieser Verbindungen, mit der Massgabe, dass
m die Zahl zwei bedeutet, wenn gleichzeitig A für -$CHF_2$, -$CF_3$, -$CH_2$-$CF_3$, -$CF_2$-$CHF_2$, -$CF_2$-CHFCl, -$CF_2$-CHFBr oder -$CF_2$-CHF-$CF_3$; $R_3$ für -$OCH_3$ oder -$CH_3$; $R_4$ für -$OCH_3$, -$CH_3$, -$OC_2H_5$, -$CH_2$-O-$CH_3$ oder -$CH_2$-$CH_2$-$OCH_3$ stehen und der Phenylkern nicht oder in 2-, 3-, 5- oder 6-Stellung zur Sulfonylgruppe nur durch einen weiteren Substituenten aus der Gruppe F, Cl, Br oder -$CH_3$ substituiert ist oder wenn gleichzeitig X Sauerstoff, A Trifluormethyl, $R_1$ Wasserstoff, $R_2$ $C_2$-$C_5$-Alkenyl in 2-Stellung, $R_3$ Wasserstoff, Chlor, Brom, Methyl, Aethyl, $C_1$-$C_3$-Alkoxy, Trifluormethyl Methylthio oder Methoxymethyl und $R_4$ Methyl oder Methoxy bedeuten, oder wenn gleichzeitig X Sauerstoff, A Trifluormethyl, $R_1$ Wasserstoff, $R_2$ Chlor in 2-Stellung, $R_3$ Wasserstoff, Chlor, Brom, Methyl, Aethyl, Trifluornethyl, Methoxy, Methoxymethyl oder Methoxyäthyl bedeuten und der Trifluormethoxyrest die 5-Stellung am Phenylring einnimmt.

2. Mittel gemäss Anspruch 1, dadurch gekennzeichnet, dass Z Sauerstoff bedeutet.

3. Mittel gemäss Anspruch 1, dadurch gekennzeichnet, dass die Reste $R_3$ und $R_4$ zusammen nicht mehr als 4 Kohlenstoffatome enthalten.

4. Mittel gemäss Anspruch 1, dadurch gekennzeichnet, dass Z Sauerstoff und m die Zahl eins bedeuten.

5. Mittel gemäss Anspruch 1, dadurch gekennzeichnet, dass Z Sauerstoff und m die Zahl zwei bedeutet.

6. Mittel gemäss Anspruch 4, dadurch gekennzeichnet, dass der Rest -X-A in 2- oder 3-position zur Sulfonylgruppe steht.

7. Mittel gemäss Anspruch 6, dadurch gekennzeichnet, dass der Rest -X-A in der 2-Position steht.

8. Mittel gemäss Anspruch 5, dadurch gekennzeichnet, dass die beiden Reste -X-A in 2- und in 5-Stellung zur Sulfonylgruppe stehen.

9. Mittel gemäss Anspruch 2, dadurch gekennzeichnet, dass nur ein Rest -X-A in der 2-Stellung zum Sulfonylrest vorhanden ist und die Reste $R_3$ und $R_4$ zusammen nicht mehr als 4 Kohlenstoffatome enthalten.

10.Mittel gemäss Anspruch 2, dadurch gekennzeichnet, dass zwei Reste -X-A in 2- und 5-Stellung zum Sulfonylrest vorhanden sind und die Reste $R_3$ und $R_4$ zusammen nicht mehr als 4 Kohlenstoffatome enthalten.

11. Mittel gemäss Anspruch 10, dadurch gekennzeichnet, dass A für -$CHF_2$, -$CF_3$ oder -$CH_2$-$CH_2Cl$ steht.

12. Mittel gemäss Anspruch 9, dadurch gekennzeichnet, dass $R_1$ Wasserstoff bedeutet, und der Rest $R_2$ in der 5- oder 6-Stellung zur Sulfonylgruppe steht.

13. Mittel gemäss Anspruch 12, dadurch gekennzeichnet, dass $R_2$ für Wasserstoff, Fluor, Nitro oder $COOR_6$ steht.

14. Mittel gemäss Anspruch 13, dadurch gekennzeichnet, dass X für Sauerstoff oder Schwefel steht.

15. Mittel gemäss Anspruch 14, dadurch gekennzeichnet, dass $R_2$ Wasserstoff, $R_3$ Chlor, Fluor, Methyl, Chlormethyl, Aethoxy, i-Propyloxy, Aethyl oder Methoxy und $R_4$ Methyl, Aethyl, Methoxy oder Aethoxy bedeuten.

16. Mittel gemäss Anspruch 15, dadurch gekennzeichnet, dass X Sauerstoff ist.

17. Mittel gemäss Anspruch 15, dadurch gekennzeichnet, dass der Rest A durch -$CF_2G$ verkörpert ist, worin G füi Wasserstoff, F, Cl, Br, $CF_3$ oder $CH_3$ steht.

18. Mittel gemäss Anspruch 16, dadurch gekennzeichnet, dass G für Chlor, Brom oder $CF_3$ steht.

19. Mittel gemäss Anspruch 15, dadurch gekennzeichnet, dass A für -$CH_2F$, -$CH_2$-$CH_2F$ und -$CH_2$-$CH_2Cl$ steht.

20.Mitte1 gemäss Anspruch 1, dadurch gekennzeichnet, dass Z Schwefel, X Sauerstoff oder Schwefel und $R_3$ und $R_4$ jeweils unabhängig voneinander $C_1$-$C_3$-Alkyl, $C_1$-$C_3$-Alkoxy oder $C_1$-$C_3$-Alkylthio mit zusammen höchstens 4 Kohlenstoffatomen bedeuten, A für -$CHF_2$, -$CF_3$, -$C_2F_5$ und -$CF_3$, -$C_2F_5$ und -$CF_2$-$CHF_2$ steht, der Rest -X-A die 2-Stellung besetzt und m die Zahl 1 bedeutet.

21. Mittel gemäss Anspruch 1, dadurch gekennzeichnet, dass es als Wirkstoff N-(2-Difluormethoxyphenyl-sulfonyl)-N'-(4-äthyl-6-methoxy-1, 3, 5-triazin-2-yl)-harnstoff enthält.

22. Mittel gemäss Anspruch 1, dadurch gekennzeichnet, dass es als Wirkstoff N-(2-Difluormethoxyphenyl-sulfonyl)-N'-(4, 6-diäthoxy-1, 3, 5-triazin-2-yl)-harnstoff enthält.

23. Mittel gemäss Anspruch 1, dadurch gekennzeichnet, dass es als Wirkstoff N-(2-Difluormethoxyphenyl-sulfonyl)-N'-(4-chlor-6-methyl-pyrimidin-2-yl)-harnstoff enthält.

24. Mittel gemäss Anspruch 1, dadurch gekennzeichnet, dass es als Wirkstoff N-(2-Pentafluoräthoxyphenyl-sulfonyl)-N'-(4-methoxy-6-methyl-1, 3, 5-triazin-2-yl)-harnstoff enthält.

25. Mittel gemäss Anspruch 1, dadurch gekennzeichnet, dass es als Wirkstoff N-(2-Difluormethylthiophenyl-sulfonyl)-N'-(4-chlor-6-methoxy-pyrimidin-2-yl)-harnstoff enthält.

26. Mittel gemäss Anspruch 1, dadurch gekennzeichnet, dass es als Wirkstoff N-[2, 5-Bis-(difluormethoxy)-phenyl-sulfonyl]-N'-(4-methoxy-6-methyl-1, 3, 5-triazin-2-yl)-harnstoff enthält.

27. Mittel gemäss Anspruch 1, dadurch gekennzeichnet, dass es als Wirkstoff N-(2-Difluormethoxyphenyl-sulfonyl)-N'-(4-methoxy-6-isopropyloxy-1, 3, 5-triazin-2-yl)-harnstoff enthält.

28. Mittel gemäss Anspruch 1, dadurch gekennzeichnet, dass es als Wirkstoff N-(2-Difluormethylthiophenyl-sulfonyl)-N'-(4-äthyl-6-methoxy-1, 3, 5-triazin-2-yl)-harnstoff enthält.

29. Mittel gemäss Anspruch 1, dadurch gekennzeichnet, dass es als Wirkstoff N-(2-Difluormethoxyphenyl-sulfonyl)-N'-(4-chlor-6-methoxy-pyrimidin-2-yl)-harnstoff enthält.

30. Verfahren zur Herstellung der Verbindungen der Formel I, Anspruch 1, dadurch gekennzeichnet, dass man entweder

a) ein Phenylsulfonamid der Formel II

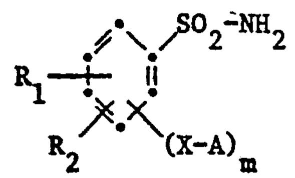

(II)

worin A, $R_1$, $R_2$, X und m die unter Formel I gegebene Bedeutung haben, in Gegenwart einer Base mit einem N-Pyrimidinyl- oder Triazinylcarbamat der Formel

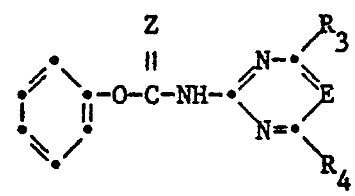

(III)

worin E, $R_3$, $R_4$ und Z die unter Formel I gegebene Bedeutung haben, umsetzt oder

b) ein Phenylsulfonylisocyanat oder -isothiocyanat der Formel IV

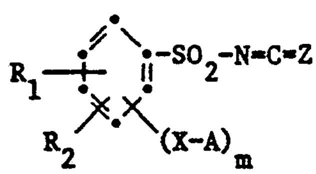

(IV)

worin A, $R_1$, $R_2$, m, X und Z die unter Formel I gegebene Bedeutung haben, gegebenenfalls in Gegenwart einer Base, mit einem Amin der Formel V

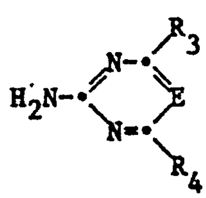

(V)

worin E, $R_3$ und $R_4$ die unter Formel I gegebene Bedeutung haben, umsetzt oder

c) man Sulfonamide der oben angegebenen Formel II gegebenenfalls in Gegenwart einer Base mit einem Isocyanat oder Isothiocyanat der Formel VI

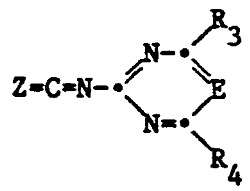

(VI)

worin E, $R_3$, $R_4$ und Z die unter Formel I gegebene Bedeutung haben, umsetzt oder
d) ein N-Phenylsulfonylcarbamat der Formel VII

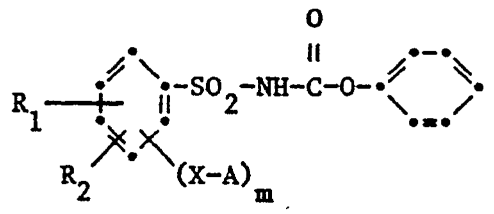

(VII)

worin A, $R_1$, $R_2$, m und X die unter Formel I gegebene Bedeutung haben, mit einem Amin der oben angegebenen Formel V umsetzt und gegebenenfalls in ihre Salze überführt indem man einen Sulfonylharnstoff der Formel I mit einem Amin, einem Alkalimetall- oder Erdalkalimetallhydroxid oder einer quaternären Ammoniumbase umsetzt.

31. Verfahren zur Herstellung der Phenylsulfonamide der Formel II

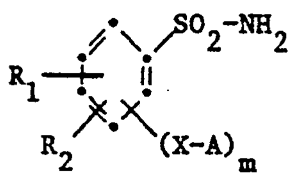

worin A einen $C_1$-$C_6$-Halogenalkylrest, mit Ausnahme der Reste $CHF_2$, $-CF_3$, $-CH_2-CF_3$, $-CF_2-CF_2H$, $-CF_2-CClFH$, $-CF_2-CClBrH$ und $-CF_2-CFH-CF_3$, bedeutet und X, $R_1$ und $R_2$ die unter Formel I gegebenen Bedeutungen haben, wobei A dann auch für $-CHF_2$ oder $-CF_3$ stehen kann, wenn nicht gleichzeitig $R_1$ Wasserstoff und $R_2$ Wasserstoff, $C_2$-$C_5$-Alkenyl, Fluor, Chlor, Brom oder Methyl bedeuten, dadurch gekennzeichnet, dass man aus einem entsprechenden Anilin durch Diazotierung und Austausch der Diazogruppe mit Schwefeldioxid in Gegenwart eines Katalysators das Phenylsulfonylchlorid herstellt und dieses durch Umsetzen mit Ammoniumhydroxid-Lösung in das Sulfonamid überführt.

32. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

33. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

34. Die Verwendung der N-Phenylsulfonyl-N'-triazinyl- oder -pyrimidinyl-harnstoffe der Formel I, oder sie enthaltender Mittel, gemäss Anspruch 32, zur selektiven pre- oder postemergenten Bekämpfung von Unkräutern in Nutzpflanzenkulturen.

35. Die Verwendung der N-phenylsulfonyl-N'-triazinyl- oder -pyrimidinyl-harnstoffe der Formel 1, oder sie enthaltender Mittel, gemäss Anspruch 33, zur Unterdrückung des Pflanzenwachstums über das 2-Blatt-stadium hinaus, dadurch gekennzeichnet, dass die Wirkstoffe preemergent angewendet werden.

36. Mittel gemäss Anspruch 1, dadurch gekennzeichnet, dass es als Wirkstoff N-(2-Pentafluoräthoxy-phenylsulfonyl)-N'-(4, 6-dimethoxy-1, 3, 5-triazin-2-yl)-harnstoff enthält.

37. Mittel gemäss Anspruch 1, dadurch gekennzeichnet, dass es als Wirkstoff N-[2-(2-Chloräthoxy)-phenylsulfonyl]-N'-(4-methoxy-6-methyl-1, 3, 5-triazin-2-yl)-harnstoff enthält.

38. Mittel gemäss Anspruch 1, dadurch gekennzeichnet, dass es als Wirkstoff N-[2-(2-Chloräthoxy)-phenylsulfonyl]-N'-(4, 6-dimethoxy-1, 3, 5-triazin-2-yl)-harnstoff enthält.

39. Mittel gemäss Anspruch 1, dadurch gekennzeichnet, dass es als Wirkstoff N-(2-Pentafluoräthoxy-phenylsulfonyl)-N'-(4-methoxy-6-methyl pyrimidin-2-yl)-harnstoff enthält.

40. Mittel gemäss Anspruch 1, dadurch gekennzeichnet, dass es als Wirkstoff N-(2-Pentafluoräthoxy-phenylsulfonyl)-N'-(4, 6-dimethyl-pyrimidin-2-yl)-harnstoff enthält.

**Claims**

for the Contracting States: DE, GB, FR, IT, NL, BE, SE

1. An N-phenylsulfonyl-N'-pyrimidinylurea or N-phenylsulfonyl-N'-triazinylurea of the general formula I

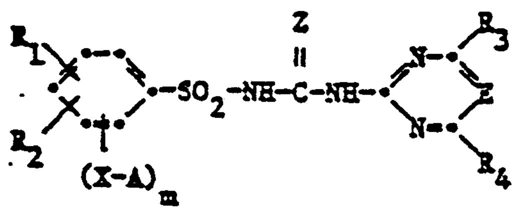

(I)

wherein
A is a $C_1$-$C_6$haloalkyl radical,
E is the methine group or nitrogen,
X is oxygen sulfur a sulfinyl or sulfonyl bridge,
Z is oxygen or sulfur,
m is 1 or 2,
$R_1$ is hydrogen, halogen, $C_1$-$C_5$alkyl, $C_2$-$C_5$alkenyl or a radical -Y-$R_5$,
$R_2$ is hydrogen, halogen, $C_1$-$C_5$alkyl, $C_2$-$C_5$alkenyl, $C_1$-$C_4$-haloalkyl, or a radical -Y-$R_5$, -$COOR_6$, -$NO_2$ or -CO-$NR_7$-$R_8$,
$R_3$ and $R_4$, each independently of the other, are hydrogen, $C_1$-$C_4$alkyl, $C_1$-$C_4$alkoxy, $C_1$-$C_4$alkylthio, $C_1$-$C_4$haloalkyl, halogen or alkoxyalkyl of at most 4 carbon atoms,
$R_5$ and $R_6$, each independently of the other, are $C_1$-$C_5$alkyl, $C_2$-$C_5$alkenyl or $C_2$-$C_5$alkynyl,
$R_7$ and $R_8$, each independently of the other, are hydrogen, $C_1$-$C_5$alkyl, $C_2$-$C_5$alkenyl or $C_2$-$C_6$ alkynyl, and
Y is oxygen, sulfur, a sulinyl or sulfonyl bridge, or a salt thereof, with the proviso that m is 2 when at the same time A is -$CHF_2$, -$CF_3$, -$CH_2$-$CF_3$, -$CF_2$-$CHF_2$, -$CF_2$-CHFCl, -$CF_2$-CHFBr or -$CF_2$-CHF-$CF_3$; $R_3$ is $OCH_3$ or $CH_3$; $R_4$ is $OCH_3$, -$CH_3$, -$OC_2H_5$, -$CH_2$-O-$CH_3$ or -$CH_2$-$CH_2$-$OCH_3$, and the phenyl nucleus is not substituted or is substituted in the 2-, 3-, 5- or 6-position to the sulfonyl group only by one further substituent selected from the group consisting of F, Cl, Br or -$CH_3$, or when at the same time X is oxygen, A is trifluoromethyl, $R_1$ is hydrogen, $R_2$ is $C_2$-$C_5$alkenyl in the 2-position, $R_3$ is hydrogen, chlorine, bromine, methyl, ethyl, $C_1$-$C_3$alkoxy, trifluoromethyl, methylthio or methoxymethyl and $R_4$ is methyl or methoxy, or when at the same time X is oxygen, A is trifluoromethyl, $R_1$ is hydrogen, $R_2$ is chlorine in the 2-position, $R_3$ is hydrogen, chlorine, bromine, methyl, ethyl, trifluoromethyl, methoxy, methoxymethyl or methoxyethyl and the trifluoromethoxy radical is in the 5-position at the phenyl ring.

2. A compound according to claim 1, wherein Z is oxygen.

3. A compound according to claim 1 wherein $R_3$ and $R_4$ together contain not more than 4 carbon atoms.

4. A compound according to claim 1, wherein Z is oxygen and m is 1.

5. A compound according to claim 1, wherein Z is oxygen and m is 2.

6. A compound according to claim 4, wherein the radical -X-A is in the 2- or 3-position to the sulfonyl group.

7. A compound according to claim 6, wherein the radical -X-A is in the 2-position.

8. A compound according to claim 5, wherein both radicals -X-A are in the 2- and 5-position to the sulfonyl group.

9. A compound according to claim 2, wherein only one radical -X-A is in the 2-position to the sulfonyl radical and $R_3$ and $R_4$ together contain not more than 4 carbon atoms.

10. A compound according to claim 2, wherein two radicals -X-A are in the 2- and 5-position to the sulfonyl radical and $R_3$ and $R_4$ together contain not more than 4 carbon atoms.

11. A compound according to claim 10, wherein A is -$CHF_2$, $CF_3$ or -$CH_2$-$CH_2Cl$.

12. A compound according to claim 9, wherein $R_1$ is hydrogen and $R_2$ is in the 5- or 6-position to the sulfonyl group.

13. A compound according to claim 12, wherein $R_2$ is hydrogen, fluorine, nitro or $COOR_6$.

14. A compound according to claim 13, wherein X is oxygen or sulfur.

15. A compound according to claim 14, wherein $R_2$ is hydrogen, $R_3$ is chlorine, fluorine, methyl, chloromethyl, ethoxy, isopropyloxy, ethyl or methoxy, and $R_4$ is methyl, ethyl, methoxy or ethoxy.

16. A compound according to claim 15 wherein X is oxygen.

17. A compound according to claim 15, wherein A is -$CF_2$G,wherein G is hydrogen, F, Cl, Br, $CF_3$ or $CH_3$.

18. A compound according to claim 16, wherein G is chlorine, bromine or $CF_3$.

19. A compound according to claim 15, wherein A is -$CH_2F$, -$CH_2$-$CH_2F$, and -$CH_2$-$CH_2Cl$.

20. A compound according to claim 1, wherein Z is sulfur, X is oxygen or sulfur, each of $R_3$ and $R_4$ independently is $C_1$-$C_3$-alkyl, $C_1$-$C_3$alkoxy or $C_1$-$C_3$alkylthio, containing together at most 4 carbon atoms, A is -$CHF_2$, -$CF_3$, -$C_2F_5$, and -$CF_2$-$CHF_2$, the radical -X-A is in the 2-position and m is 1.

21. N-(2-Difluoromethoxyphenylsulfonyl)-N'-(4-ethyl-6-methoxy-1, 3, 5-triazin-2-yl)urea according to claim 1.

22. N-(2-Difluoromethoxyphenylsulfonyl)-N'-(4, 6-diethoxy-1, 3, 5-triazin-2-yl)urea according to claim 1.

23. N-(2-Difluoromethoxyphenylsulfonyl)-N'-(4-chloro-6-methylpyrimidin-2-yl)urea according to claim 1.

24. N-(2-Pentafluoroethoxyphenylsulfonyl)-N'-(4-methoxy-6-methyl-1, 3, 5-triazin-2-yl)urea according to claim 1.

25. N-(2-Difluoromethylthiophenylsulfonyl)-N' -(4-chloro-6-methoxypyrimidin-2- yl)urea according to claim 1.

26. N-[2, 5-Bis-(difluoromethoxy)phenylsulfonyl]-N'-(4-methoxy-6-methyl-1, 3, 5-triazin-2-yl)urea according to claim 1.

27. N-(2-Difluoromethoxyphenylsulfonyl)-N'-(4-methoxy-6-isopropyloxy-1, 3, 5-triazin-2-yl)urea according to claim 1.

28. N-(2-Difluoromethylthiophenylsulfonyl)-N'-(4-ethyl-6-methoxy-1, 3, 5-triazin-2-yl)urea according to claim 1.

29. N-(2-Difluoromethoxyphenylsulfonyl)-N'-(4-chloro-6-methoxypyrimidin-2-yl)urea according to claim 1.

30 A process for the preparation of a compound of formula I according to claim 1, which process comprises reacting a phenylsulfonamide of formula II

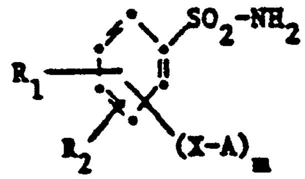

(II)

wherein A, $R_1$, $R_2$, X and m are as defined for formula I, with an N-pyrimidinylcarbamate or N-triazinylcarbamate of the formula

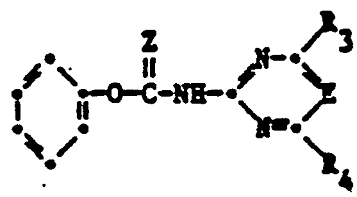

(III)

wherein E, $R_3$, $R_4$ and Z are as defined for formula I, in the presence of a base, and, if desired, converting the resultant compound of formula I into a salt thereof.

31. A process for the preparation of a compound of formula I according to claim 1, which process comprises reacting a phenylsulfonylisocyanate or phenylsulfonylisothiocyanate of formula IV

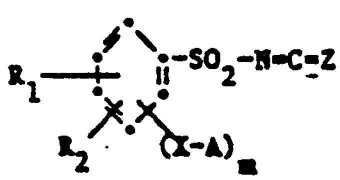

(IV)

wherein A, $R_1$, $R_2$, m, X and Z are as defined for formula I, with an amine of formula V

wherein E, $R_3$ and $R_4$ are as defined for formula I, in the absence or presence of a base, and, if desired, converting the resultant compound of formula I into a salt thereof.

32. A process for the preparation of a compound of formula I according to claim 1, which process comprises reacting a sulfonamide of the above formula II with an isocyanate or isothiocyanate of formula VI

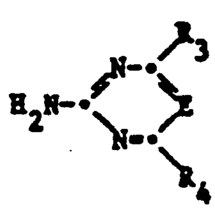

(V)

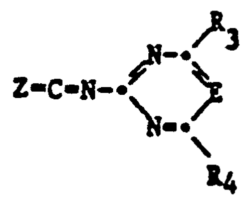

(VI)

wherein E, $R_3$, $R_4$ and Z are as defined for formula I, in the absence or presence of a base, and, if desired, converting the resultant compound of formula I into a salt thereof.

33. A process for the preparation of a compound of formula I according to claim 1, which process comprises reacting arr N-phenylsulfonylcarbamate of formula VII

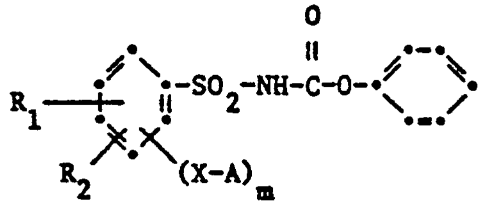

(VII)

wherein A, $R_1$, $R_2$, m and X are as defined for formula I, with an amine of the above formula V and, if desired, converting the resultant compound of formula I into a salt thereof.

34. A process for the preparation of a basic addition salt of formula I according to any one of claims 30 to 33, which process comprises reacting a sulfonylurea of formula I with an amine, an alkali metal hydroxide, an alkaline earth metal hydroxide or a quaternary ammonium base.

35. A phenylsulfonamide of formula II

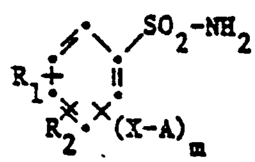

wherein A is a $C_1$-$C_6$haloalkyl radical, excepting $CHF_2$, $-CF_3$ $-CH_2-CF_3$, $-CF_2-CF_2H$, $-CF_2-CClFH$, $-CF_2-CClBrH$ and $-CF_2-CFH-CF_3$, and X, $R_1$ and $R_2$ are as defined for formula I, with the proviso that A can also be $-CHF_2$ or $-CF_3$ unless at the same time $R_1$ is hydrogen and $R_2$ is hydrogen, $C_2$-$C_5$alkenyl, fluorine, chlorine, bromine or methyl.

36. A process for the preparation of a phenylsulfonamide according to claim 35, which process comprises preparing a phenylsulfonyl chloride from a corresponding aniline by diazotisation and replacement of the diazo group with sulfur dioxide, in the presence of a catalyst, and converting said phenylsulfonyl chloride into the sulfonamide by reaction with ammonium hydroxide solution.

37. A herbicidal and plant growth regulating composition which contains, as active ingredient, at least one N-phenylsulfonyl-N'-triazinylurea or N-phenylsulfonyl-N'-pyrimidinylurea of formula I according to claim 1, together with suitable carriers and/or other adjuvants.

38. A method of controlling undesired plant growth, which method comprises applying thereto or to the locus thereof a herbicidally effective amount of an N-phenylsulfonyl-N'-triazinylurea or N-phenylsulfonyl-N'-pyrimidinylurea of formula I according to claim 1, or of a composition containing such a compound.

39. A method of suppressing plant growth, which method comprises applying thereto or to the locus thereof an effective amount of an N-phenylsulfonyl-N'-triazinylurea or N-phenylsulfonyl-N'-pyrimidinylurea of formula I according to claim 1 or of a composition containing such a compound.

40. A method according to claim 38 of selectively controlling weeds in crops of useful plants, which method comprises applying the N-phenylsulfonyl-N'-triazinylurea or N-phenylsulfonyl-N'-pyrimidinylurea of formula I according to claim 1, or a composition containing such a compound, preemergence or postemergence.

41. A method according to claim 39 of suppressing plant growth beyond the two-leaf-stage, which method comprises applying the N-phenylsulfonyl-N'-triazinylurea or N-phenylsulfonyl-N'-pyrimidinylurea of formula I according to claim 1 or a composition containing such a compound, preemergence.

42. N-(2-Pentafluoroethoxyphenylsulfonyl)-N'-(4, 6-dimethoxy-1, 3, 5-triazin-2-yl)urea according to claim 1.

43. N-[2-(2-Chloroethoxy)phenylsulfonyl]-N'-(4-methoxy-6-methyl-1, 3, 5-triazin-2-yl)urea according to claim 1.

44. N-[2-(2-Chloroethoxy)phenylsulfonyl]-N'-(4, 6-dimethoxy-1, 3, 5-triazin-2-yl)urea according to claim 1.

45. N-(2-Pentafluoroethoxyphenylsulfonyl)-N'-(4-methoxy-6-methylpyrimidin-2-yl)urea according to claim 1.

46. N-(2-Pentafluoroethoxyphenylsulfonyl)-N'-(4, 6-dimethylpyrimidin-2-yl)urea according to claim 1.

**Claims**

for the Contracting State: AT

1. A herbicidal and plant growth regulating composition which contains, as active ingredient, at least one N-phenylsulfonyl-N'-pyrimidinylurea or N-phenylsulfonyl-N'triazinylurea of the general formula I

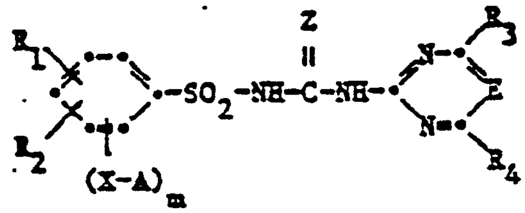

(I)

wherein
A is a $C_1$-$C_6$haloalkyl radical,
E is the methine group or nitrogen,
X is oxygen, sulfur, a sulfinyl or sulfonyl bridge,
Z is oxygen or sulfur,
m is 1 or 2,
$R_1$ is hydrogen, halogen, $C_1$-$C_5$alkyl, $C_2$-$C_5$alkenyl or a radical -Y-$R_5$,
$R_2$ is hydrogen, halogen, $C_1$-$C_5$alkyl, $C_2$-$C_5$alkenyl, $C_1$-$C_4$-haloalkyl, or a radical -Y-$R_5$, -$COOR_6$, -$NO_2$ or -CO-$NR_7$-$R_8$,
$R_3$ and $R_4$, each independently of the other, are hydrogen, $C_1$-$C_4$alkyl, $C_1$-$C_4$alkoxy, $C_1$-$C_4$alkylthio, $C_1$-$C_4$haloalkyl, halogen or alkoxyalkyl of at most 4 carbon atoms,
$R_5$ and $R_6$, each independently of the other, are $C_1$-$C_5$alkyl, $C_2$-$C_5$alkenyl or $C_2$-$C_6$alkynyl,
$R_7$ and $R_8$, each independently of the other, are hydrogen, $C_1$-$C_5$alkyl, $C_2$-$C_5$alkenyl or $C_2$-$C_6$alkynyl, and
Y is oxygen, sulfur, a sulfinyl or sulfonyl bridge, or a salt thereof, with the proviso that m is 2 when at the same time A is -$CHF_2$, -$CF_3$, -$CH_2$-$CF_3$, -$CF_2$-$CHF_2$, -$CF_2$-CHFCl, -$CF_2$-CHFBr or -$CF_2$-CHF-$CF_3$; $R_3$ is $OCH_3$ or $CH_3$; $R_4$ is $OCH_3$, -$CH_3$, -$OC_2H_5$, -$CH_2$-O-$CH_3$ or -$CH_2$-$CH_2$-$OCH_3$, and the phenyl nucleus is not substituted or is substituted in the 2-, 3-, 5- or 6-position to the sulfonyl group only by one further substituent selected from the group consisting of F, Cl, Br or -$CH_3$, or when at the same time X is oxygen, A is trifluoromethyl, $R_1$ is hydrogen, $R_2$ is $C_2$-$C_5$alkenyl in the 2-position, $R_3$ is hydrogen, chlorine, bromine, methyl, ethyl, $C_1$-$C_3$alkoxy, trifluoromethyl, methylthio or methoxymethyl and $R_4$ is methyl or methoxy, or when at the same time X is oxygen, A is trifluoromethyl, $R_1$ is hydrogen, $R_2$ is chlorine in the 2-position, $R_3$ is hydrogen, chlorine, bromine, methyl, ethyl, trifluoromethyl, methoxy, methoxymethyl or methoxyethyl and the trifluoromethoxy radical is in the 5-position at the phenyl ring, together with carriers and/or other adjuvants.

2. A composition according to claim 1 wherein Z is oxygen.

3. A composition according to claim 1 wherein $R_3$ and $R_4$ together contain not more than 4 carbon atoms.

4. A composition according to claim 1, wherein Z is oxygen and m is 1.

5. A composition according to claim 1, wherein Z is oxygen and m is 2.

6. A composition according to claim 4, wherein the radical -X-A is in the 2- or 3-position to the sulfonyl group.

7. A composition according to claim 6 wherein the radical -X-A is in the 2-position.

8. A composition according to claim 5, wherein both radicals -X-A are in the 2- and 5-position to the sulfonyl group.

9. A composition according to claim 2, wherein only one radical -X-A is in the 2-position to the sulfonyl radical and $R_3$ and $R_4$ together contain not more than 4 carbon atoms.

10. A composition according to claim 2, wherein two radicals -X-A are in the 2- and 5-position to the sulfonyl radical and $R_3$ and $R_4$ together contain not more than 4 carbon atoms.

11. A composition according to claim 10, wherein A is -$CHF_2$, $CF_3$ or -$CH_2$-$CH_2$Cl.

12. A composition according to claim 9, wherein $R_1$ is hydrogen and $R_2$ is in the 5- or 6-position to the sulfonyl group.

13. A composition according to claim 12, wherein $R_2$ is hydrogen, fluorine, nitro or $COOR_6$.

14. A composition according to claim 13, wherein X is oxygen or sulfur.

15. A composition according to claim 14, wherein $R_2$ is hydrogen, $R_3$ is chlorine, fluorine, methyl, chloromethyl, ethoxy, isopropyloxy, ethyl or methoxy, and $R_4$ is methyl, ethyl, methoxy or ethoxy.

16. A composition according to claim 15, wherein X is oxygen.

17. A composition according to claim 15, wherein A is -$CF_2$G wherein C is hydrogen, F, Cl, Br, $CF_3$ or $CH_3$.

18. A composition according to claim 16, wherein C is chlorine, bromine or $CF_3$.

19. A composition according to claim 15 wherein A is -$CH_2$F -$CH_2$-$CH_2$F and -$CH_2$-$CH_2$Cl.

20. A composition according to claim 1, wherein Z is sulfur, X is oxygen or sulfur, each of $R_3$ and $R_4$ independently is $C_1$-$C_3$-alkyl, $C_1$-$C_3$alkoxy or $C_1$-$C_3$alkylthio,containing together at most 4 carbon atoms, A is -$CHF_2$, -$CF_3$, -$C_2F_5$ and -$CF_2$-$CHF_2$, the radical -X-A is in the 2-position, and m is 1.

21. A composition according to claim 1, which contains, as active ingredient, N-(2-difluoromethoxyphenylsulfonyl)-N'-(4-ethyl-6-methoxy-1, 3, 5-triazin-2-yl)urea.

22. A composition according to claim 1, which contains, as active ingredient, N-(2-difluoromethoxyphenylsulfonyl)-N'-(4, 6-diethoxy-1, 3, 5-tria,in-2-yl)urea.

23. A composition according to claim 1, which contains, as active ingredient, N-(2-difluoromethoxyphenylsulfonyl)-N'-(4-chloro-6-methylpyrmidin-2-yl)urea.

24. A composition according to claim 1, which contains, as active ingredient, N-(2-pentafluoroethoxyphenylsulfonyl)-N'-(4-methoxy-6-methyl-1, 3,-5-triazin-2-yl)urea.

25. A composition according to claim 1, which contains, as active ingredient, N-(2-difluoromethylthiophenylsulfonyl)-N'-(4-chloro-6-methoxypyrimidin-2-yl)urea.

26. A composition according to claim 1, which contains, as active ingredient, N-(2, 5-bis-(difluoromethoxy)phenyl-sulfonyl)-N'-(4-methoxy-6-methyl-1, 3, 5-triazin-2-yl)urea.

27. A composition according to claim 1, which contains, as active ingredient, N-(2-difluoromethoxyphenylsulfonyl)-N'-(4-methoxy-6-isopropyloxy-1, 3, 5-triazin-2-yl)urea.

28. A composition according to claim 1, which contains, as active ingredient, N-(2-difluoromethylthiophenylsulfonyl)-N'-(4-ethyl-6-methoxy-1, 3, 5-triazin-2-yl)urea.

29. A composition according to claim 1, which contains, as active ingredient, N-(2-difluoromethoxyphenylsulfonyl)-N'-(4-chloro-6-methoxypyrimidin-2-yl)urea.

30. A process for the preparation of a compound of formula I according to claim 1, which process comprises either

a) reacting a phenylsulfonamide of formula II

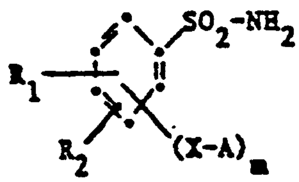

(II)

wherein A, $R_1$, $R_2$, X and m are as defined for formula I, with an N-pyrimidinylcarbamate or N-triazinylcarbamate of the formula

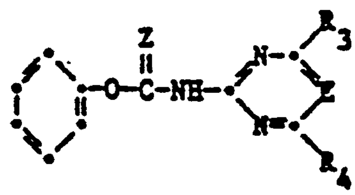

(III)

wherein E, $R_3$, $R_4$, and Z are as defined for formula I, in the presence of a base, or

b) reacting a phenylsulfonylisocyanate or phenylsulfonylisothiocyanate of formula IV

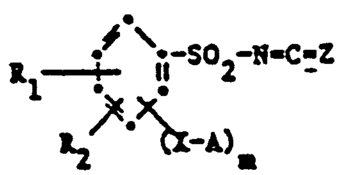

(IV)

wherein A, $R_1$, $R_2$, m, X, and Z are as defined for formula I, with an amine of formula V

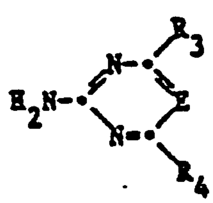

(V)

wherein E, $R_3$, and $R_4$ are as defined for formula I, in the absence or presence of a base, or

c) reacting a sulfonamide of the above formula II with an isocyanate or isothiocyanate of formula VI

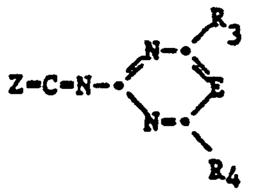

(VI)

wherein E, $R_3$, $R_4$, and Z are as defined for formula I, in the absence or presence of a base, or d) reacting an N-phenylsulfonylcarbamate of formula VII

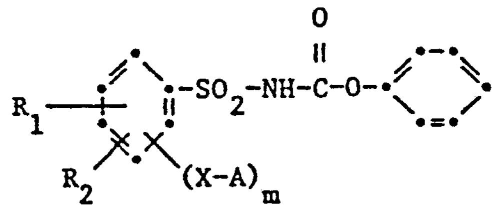

(VII)

wherein A $R_1$ $R_2$, m, and X are as defined for formula I, with an amine of the above formula V and, if desired, converting the resultant sulfonylurea of formula I into a salt thereof by reaction with an amine, an alkali metal hydroxide, an alkaline earth metal hydroxide or a quaternary ammonium base.

31. A process for the preparation of a phenylsulfonamide of formula II

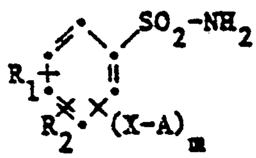

wherein A is a $C_1$-$C_6$haloalkyl radical, excepting $CHF_2$, $-CF_3$ $-CH_2-CF_3$, $-CF_2-CF_2H$, $-CF_2-CClFH$, $-CF_2-CClBrH$ and $-CF_2-CFH-CF_3$, and X, $R_1$ and $R_2$ are as defined for formula I, with the proviso that A can also be $-CHF_2$ or $-CF_3$ unless at the same time $R_1$ is hydrogen and $R_2$ is hydrogen, $C_2$-$C_5$alkenyl, fluorine, chlorine, bromine or methyl, which process comprises preparing a phenylsulfonyl chloride from a corresponding aniline by diazotisation and replacement of the diazo group with sulfur dioxide, in the presence of a catalyst, and converting said phenylsulfonyl chloride into the sulfonamide by reaction with ammonium hydroxide solution.

32. A method of controlling undesired plant growth, which method comprises applying thereto or to the locus thereof a herbicidally effective amount of an N-phenylsulfonyl-N'-triazinylurea or N-phenylsulfonyl-N'-pyrimidinylurea of formula I according to claim 1, or of a composition containing such a compound.

33. A method of suppressing plant growth, which method comprises applying thereto or to the locus thereof an effective amount of an N-phenylsulfonyl-N'-triazinylurea or N-phenylsulfonyl-N'-pyrimidinylurea of formula I according to claim 1, or of a composition containing such a compound.

34. A method according to claim 32 of selectively controlling weeds in crops of useful plants, which method comprises applying the N-phenylsulfonyl-N'-triazinylurea or N-phenylsulfonyl-N'-pyrimidinylurea of formula I according to claim 1, or a composition containing such a compound, preemergence or postemergence.

35. A method according to claim 33 of suppressing plant growth beyond the two-leaf-stage, which method comprises applying the N-phenylsulfonyl-N'-triazinylurea or N-phenylsulfonyl-N'-pyrimidinylurea of formula I according to claim 1,or a composition containing such a compound, preemergence.

36. A composition according to claim 1, which contains, as active ingredient, N-(2-pentafluoroethoxyphenylsulfonyl)-N'-(4, 6-dimethoxy-1, 3, 5-triazin-2-yl)urea.

37. A composition according to claim 1, which contains, as active ingredient, N-[2-(2-chloroethoxy)phenylsulfonyl]-N'-(4-methoxy-6-methyl-1, 3, 5-triazin-2-yl)urea.

38. A composition according to claim 1, which contains, as active ingredient, N-[2-(2-chloroethoxy)phenylsulfonyl]-N'-(4, 6-dimethoxy-1, 3, 5-triazin-2-yl)urea.

39. A composition according to claim 1, which contains, as active ingredient, N-(2-pentafluoroethoxyphenylsulfonyl)-N'-(4-methoxy-6-methylpyrimidin-2-yl)urea.

40. A composition according to claim 1, which contains, as active ingredient, N-(2-pentafluoroethoxyphenylsulfonyl)-N'-(4, 6-dimethylpyrimidin-2-yl)urea.

**Revendications**

pour les Etats contractants: DE, GB, FR, IT, NL, BE, SE

1 - N-phénylsulfonyl-N'-pyrimidinyl- et triazinyl-urées de formule générale I

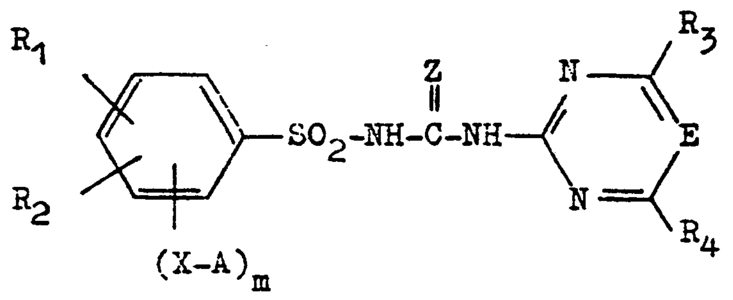

(I)

où

A représente un radical halogénalcoyle en $C_1$ à $C_6$,
E représente le groupe méthine ou un azote,
X représente un oxygène, un soufre, un pont sulfinyle ou sulfonyle,
Z représente un oxygène ou un soufre,
m représente le nombre 1 ou 2,
$R_1$ représente un hydrogène, un alcoyle en $C_1$ à $C_5$, un alcényle en $C_2$ à $C_5$ ou un radical $-Y-R_5$,
$R_2$ représente un hydrogène, un halogène, un alcoyle en $C_1$ à $C_5$, un alcényle en $C_2$ à $C_5$, un halogénalcoyle en Cl à C4 ou un radical $-Y-R_5$, $-COOR_6$, $-NO_2$ ou $-CO-NR_7R_8$,
$R_3$ et $R_4$ représentent indépendamment l'un de l'autre un hydrogène, un alcoyle en $C_1$ à $C_4$, un alcoxy en $C_1$ à $C_4$, un alcoylthio en $C_1$ à $C_4$, un halogénalcoyle en $C_1$ à $C_4$, un halogène ou un alcoxyalcoyle avec au plus 4 atomes de carbone,
$R_5$ et $R_6$ représentent chacun un alcoyle en $C_1$ à $C_5$, un alcényle en $C_2$ à $C_5$ ou un alcynyle en $C_2$ à $C_5$,
$R_7$ et $R_8$ représentent indépendamment l'un de l'autre un hydrogène, un alcoyle en $C_1$ à $C_5$, un alcényle en $C_2$ à $C_5$ ou un alcynyle en $C_2$ à $C_6$ et
Y représente un oxygène, un soufre, un pont sulfinyle ou sulfonyle,
ainsi que les sels de ces composés, avec la précision que
m représente le nombre 2, lorsque A représente simultanément $-CHF_2$, $-CF_3$, $-CH_2-CF_3$, $-CF_2-CHF_2$, $-CF_2-CHFCl$, $-CF_2-CHFBr$ ou $-CF_2-CHF-CF_3$; $R_3$ représente $-OCH_3$ ou $-CH_3$;
$R_4$ représente $-OCH_3$, $-CH_3$, $-OC_2H_5$, $-CH_2-O-CH_3$ ou $-CH_2-CH_2-OCH_3$
et le noyau phényle n'est pas substitué ou est seulement substitué en position 2, 3, 5 ou 6 par rapport au groupe sulfonyle par un autre substituant du groupe F, Cl, Br ou $-CH_3$ ou lorsque simultanément X représente un oxygène. A un trifluorométhyle, $R_1$ un hydrogène, $R_2$ un alcényle en $C_2$ à $C_5$ en position 2, $R_3$ un hydrogène, un chlore, um brome, un méthyle, un éthyle, un alcoxy en $C_1$ à $C_3$, un trifluorométhyle, un méthylthio ou un méthoxyméthyle et $R_4$ représente un méthyle ou un méthoxy, ou lorsque simultanément X représente un oxygène, A un trifluorométhyle, $R_1$ un hydrogène, $R_2$ un chlore en position 2, $R_3$ un hydrogène, un chlore, un brome, un méthyle, un éthyle, un trifluorométhyle, un méthoxy, un méthoxyméthyle ou un méthoxyéthyle et le radical trifluorométhoxy prend la position 5 sur le noyau phényle.

2 - Composés selon la revendication 1, caractérisés en ce que Z représente un oxygène.

3 - Composés selon la revendication 1, caractérisés en ce que les radicaux $R_3$ et $R_4$ ensemble ne contiennent pas plus de 4 atomes de carbone.

4 - Composés selon la revendication 1, caractérisés en ce que Z représente un oxygène et m le nombre 1.

5 - Composés selon la revendication 1, caractérisés en ce que Z représente un oxygène et m le nombre 2.

6 - Composés selon la revendication 4, caractérisés en ce que le radical -X-A est en position 2 ou 3 par rapport au groupe sulfonyle.

7 - Composés selon la revendication 6, caractérisés en ce que le radical -X-A est en position 2.

8 - Composés selon la revendication 5, caractérisés en ce que les deux radicaux -X-A sont en position 2 et 5 par rapport au groupe sulfonyle.

9 - Composés selon la revendication 2, caractérisés en ce que seul un radical -X-A est présent en position 2 par rapport au radical sulfonyle et en ce que les radicaux $R_3$ et $R_4$ ensemble ne contiennent pas plus de 4 atomes de carbone.

10 - Composés selon la revendication 2, caractérisés en ce que deux radicaux -X-A sont en position 2 et 5 par rapport au radical sulfonyle et en ce que les radicaux $R_3$ et $R_4$ ensemble ne contiennent pas plus de 4 atomes de carbone.

11 - Composés selon la revendication 10, caractérisés en ce que X représente $-CHF_2$, $-CF_3$ ou $-CH_2-CH_2Cl$.

12 - Composés selon la revendication 9, caractérisés en ce que $R_1$ représente un hydrogène, et le radical $R_2$ est en position 5 ou 6 par rapport au groupe sulfonyle.

13 - Composés selon la revendication 12, caractérisés en ce que $R_2$ représente un hydrogène, un fluor, un nitro ou $COOR_6$.

14 - Composés selon la revendication 13, caractérisés en ce que X représente un oxygène ou un soufre.

15 - Composés selon la revendication 14, caractérisés en ce que $R_2$ représente un hydrogène, $R_3$ représente un chlore, un fluor, un méthyle, un chlorométhyle, un éthoxy, un i-propyloxy, un éthyle ou un méthoxy et $R_4$ représente un méthyle, un éthyle, un méthoxy ou un éthoxy.

16 - Composés selon la revendication 15, caractèrisés en ce que X est un oxygène.

17 - Composés selon la revendication 15, caractérisés en ce que le radical A est incarné par $-CF_2G$, où G représente un hydrogène, F, Cl, Br, $CF_3$ ou $CH_3$.

18 - Composés selon la revendication 16, caractérisés en ce que G représente un chlore, un brome ou $CF_3$.

19 - Composés selon la revendication 15, caractérisés en ce que A représente $-CH_2F$, $-CH_2-CH_2F$ et $-CH_2-CH_2Cl$.

20 - Composés selon la revendication 1, caractérisés en ce que Z représente un soufre, X un oxygène ou un soufre et $R_3$ et $R_4$ représentent chacun indépendamment l'un de l'autre un alcoyle en $C_1$ à $C_3$, un alcoxy en $C_1$ à $C_3$ ou un alcoylthio en $C_1$ à $C_3$ avec ensemble au plus 4 atomes de carbone, A représente $-CHF_2$, $CF_3$, $-C_2F_5$ et $-CF_3$, $-C_2F_5$ et $-CF_2-CHF_2$, le radical -X-A a la position 2 et m représente le nombre 1.

21 - N-(2-Difluorométhoxyphényl-sulfonyl)-N'-(4-éthyl-6-méthoxy-1, 3, 5-triazin-2-yl)-urée selon la revendication 1.

22 - N-(2-difluorométhoxyphényl-sulfonyl)-N'-(4, 6-diéthoxy-1, 3, 5-triazin-2-yl)-urée selon la revendication 1.

23 - N-(2-difluorométhoxyphènyl-sulfonyl)-N'-(4-chloro-6-méthyl-pyrimidin-2-yl)-urée selon la révendication 1.

24 - N-(2-pentafluoréthoxyphényl-sulfonyl)-N'-(4-méthoxy-6-méthyl-1, 3, 5-triazin-2-yl)-urée selon la revendication 1.

25 - N-(2-Difluorométhylthiophényl-sulfonyl)-N'-(4-chloro-6-méthoxy-pyrimidin-2-yl)-urée selon la revendication 1.

26 - N-[2, 5-Bis-(difluorométhoxy)-phényl-sulfonyl)-N'-(4-méthoxy-6-méthyl-1, 3, 5-triazin-2-yl)-urée selon la revendication 1.

27 - N-(2-Difluorométhoxyphényl-sulfonyl)-N'-(4-méthoxy-6-isopropyloxy-1, 3, 5-triazin-2-yl)-urée selon la revendication 1.

28 - N-(2-(Difluorométhylthiophényl-sulfonyl)-N'-(4-éthyl-6-méthoxy-1, 3, 5-triazin—2-yl)-urée selon la revendication 1.

29 - N-(2-Difluorométhoxyphényl-sulfonyl)-N'-(4-chloro-6-méthoxypyrimidin-2-yl)-urée selon la revendication 1.

30 - Procédé de préparation des composés de formule 1 de la revendication 1, caractérisé en ce qu'on fait réagir un phénylsulfonamide de formule II

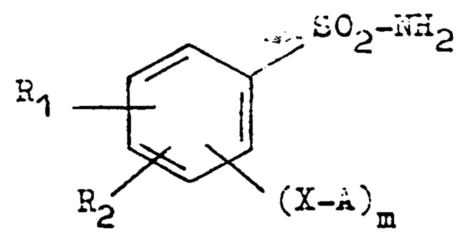

(II)

où A, $R_1$, $R_2$, X et m ont la signification donnée pour la formule I, en présence d'une base avec un N-pyrimidinyl- ou triazinyl-carbamate de formule

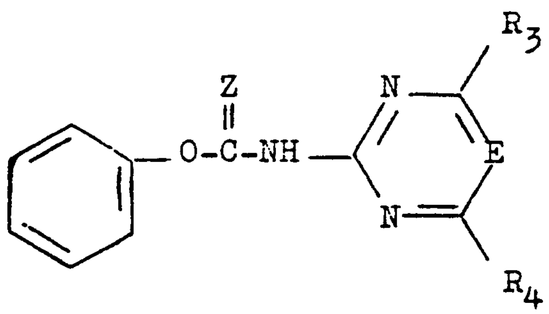

(III)

où E, $R_3$, $R_4$ et Z ont la signification donnée pour la formule I,
et éventuellement en ce qu'on le transforme en ses sels.

31 - Procédé de préparation des composés de formule I de la revendication 1, caractérisé en ce qu'on fait réagir un phénylsulfonylisocyanate ou -isothiocyanate de formule IV

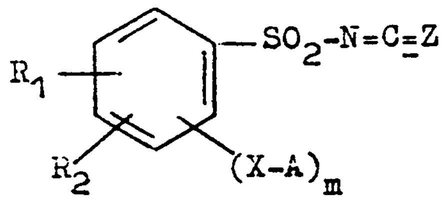

(IV)

où X, $R_1$, $R_2$, m, X et Z ont la signification donnée pour la formule I, éventuellement en présence d'une base, avec une amine de formule V

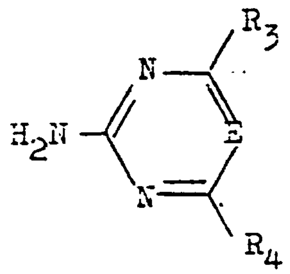

(V)

où E, $R_3$ et $R_4$ ont la signification donnée pour la formule I,
et éventuellememt en ce qu'on le transforme en ses sels.

32 - Procédé de préparation des composés de formule I de la revendication 1, caractérisé en ce qu'on fait réagir des sulfonamides de formule II indiquée ci-dessus éventuellement en présence d'une base avec un isocyanate ou isothiocyanate de formule VI

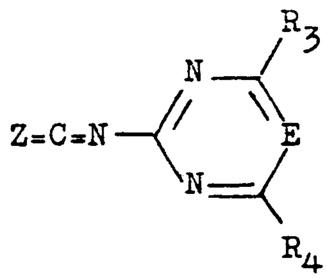

(VI)

où E, $R_3$, $R_4$ et Z ont la signification donnée pour la formule I et éventuellement en ce qu'on les transforme en leurs sels.

33 - Procédé de préparation des composés de formule I de la revendication 1, caractérisé en ce qu'on fait réagir un N-phénylsulfonylcarbamate de formule VII

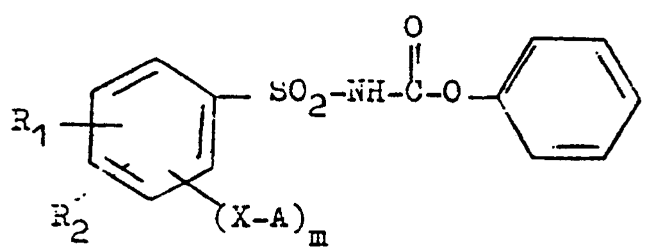

(VII)

où A, $R_1$, $R_2$, m et X ont la signification donnée pour la formule I, avec une amine de formule V donnée cidessus et éventuellement en ce qu'on le transforme en ses sels.

34 - Procédé de préparation de sels d'addition basiques de formule I selon l'une des revendications 30 à 33, caractérisé en ce qu'on fait réagir une sulfonylurée de formule I avec une amine, un hydroxyde de métal alcalin ou alcalino-terreux ou une base d'ammonium quaternaire.

35 - Phénylsulfonamides de formule II

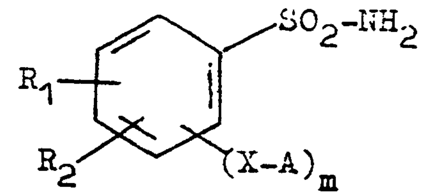

où A représente un radical halogénoalcoyle en $C_1$ à $C_6$, à l'exception des radicaux $CHF_2$, $-CF_3$, $-CH_2-CF_3$, $-CF_2-CF_2H$, $-CF_2-CClFH$, $-CF_2-CClBrH$ et $-CF_2-CFH-CF_3$, et X, $R_1$ et $R_2$ ont les significations données pour la formule I, où A peut également représenter $-CHF_2$ ou $-CF_3$ lorsqu'on n'a pas simultanément $R_1$ représentant un hydrogène et $R_2$ un hydrogène, un alcényle en $C_2$ à $C_5$, un fluor, un chlore, un brome ou un méthyle.

36 - Procédé de préparation des phénylsulfonamides selon la revendication 35, caractérisé en ce qu'on prépare par diazotation et échange du groupe diazo avec de l'anhydride sulfureux en présence d'un catalyseur à partir d'une aniline correspondante le chlorure de phénylsulfonyle et en ce qu'on transforme celui-ci par réaction avec une solution d'hydroxyde d'ammonium pour donner le sulfonamide.

37 - Agent herbicide et inhibiteur de la croissance des plantes, caractérisé en ce qu'il contient, outre des supports et autres additifs, comme matière active au moins une N-phénylsulfonyl-N'-triazinyl- ou -pyrimidinyl-urée de formule I de la revendication 1.

38 - Applicatiom des N-phénylsulfonyl-N'triazimyl- ou -pyrimidinyl-urées de formule I de la revendication 1 ou des agents qui les contiennent à la lutte contre la croissance végétale indésirable.

39 - Application des N-phénylsulfonyl-N'triazinyl- ou -pyrimidinyl-urées de formule I de la revendication 1 ou des agents qui les contiennent à l'inhibition de la croissance des plantes.

40 - Application des N-phénylsulfonyl-N'triazinyl- ou -pyrimidinyl-urées de formule I ou des agents qui les contiennent, selon la revendication 38, à la lutte sélective en pré- ou en post-levée contre les mauvaises herbes dans les cultures de plantes utiles.

41 - Application des N-phénylsulfonyl-N'triazinyl- ou -pyrimidinyl-urées de formule I ou de 39, agents qui les contiennent, selon la revendication 39, pour supprimer la croissance végétale au-delà du stade 2 feuilles, caracterisée en ce qu'on applique les matières actives en pré-levée.

42 - N-(2-Pentafluoréthoxy-phénylsulfonyl)-N, (4, 6-diméthoxy-1, 3, 5-triazin-2:y1)-urée selon la revendication 1.

43 - N-[2-(2-Chloréthoxy)-phénylsulfonyl]-N'-(4-méthoxy-6-méthyl-1, 3, 5-triazin-2-yl)-urée selon la revendication 1.

44 - N-[2-(2-Chloréthoxy)-phénylsulfonyl]-N'-(4, 6-diméthoxy-1, 3, 5-triazin-2-yl)-urée selon la revendication 1.

45 - N-(2-Pemtafluoréthoxy-phénylsulfonyl)-N'-(4-méthoxy-6-méthyl pyrimidin-2-yl)-urée selon la revendication 1.

46 - N-(2-Pentafluoréthoxy-phénylsulfonyl)
N -(4, 6-diméthyl-pyrimidin-2-yl)-urée selon la revendication 1.

**Revendications**

pour l'Etat contractant: AT

1 - Agent herbicide et inhibiteur de la croissance des plantes, caractérisé en ce qu'il contient, outre des supports et/ou autres additifs, comme matière active au moins une N-phénylsulfonyl-N'-pyrimidinylet -triazinyl-urée de formule générale

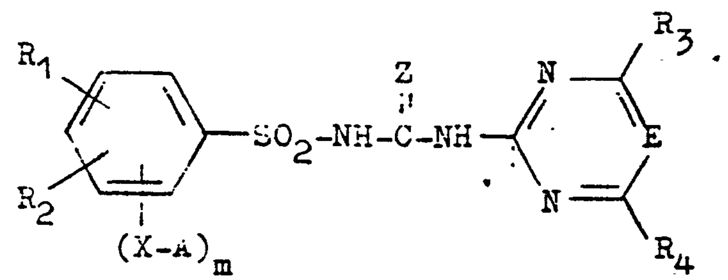

(I)

ou un de ses sels, où
A représente un radical halogénalcoyle en $C_1$ à $C_6$,
E représente le groupe néthine ou un azote,
X représente un oxygène, un soufre, un pont sulfinyle ou sulfonyle,
Z représente un oxygène ou un soufre,
m représente le nombre 1 ou 2,

$R_1$ représente un hydrogène, un alcoyle en $C_1$ à $C_5$, un alcényle en $C_2$ à $C_5$ ou un radical -Y-$R_5$,

$R_2$ représente un hydrogène, un halogène, un alcoyle en $C_1$ à $C_5$, un alcényle en $C_2$ à $C_5$, un halogénalcoyle en $C_1$ à $C_4$ ou un radical -Y-$R_5$, -$COOR_6$, -$NO_2$ ou -CO-$NR_7R_8$,

$R_3$ et $R_4$ représentent indépendamment l'un de l'autre un hydrogène, un alcoyle en $C_1$ à $C_4$, un alcoxy en $C_1$ à $C_4$, un alcoylthio en $C_1$ à $C_4$, un halogénalcoyle en $C_1$ à $C_4$, un halogène ou un alcoxyalcoyle avec au plus 4 atomes de carbone,

$R_5$ et $R_6$ représentent chacun un alcoyle en $C_1$ à $C_5$, un alcényle en $C_2$ à $C_5$ ou un alcynyle en $C_2$ à $C_5$,

$R_7$ et $R_8$ représentent indépendamment l'un de l'autre un hydrogène, un alcoyle en $C_1$ à $C_5$, un alcényle en $C_2$ à $C_5$ ou un alcynyle en $C_2$ à $C_6$ et

Y représente un oxygène, un soufre, un pont sulfinyle ou sulfonyle,

ainsi que les sels de ces composés, avec la précision que

m représente le nombre 2, lorsque A représente simultanément -$CHF_2$, -$CF_3$, -$CH_2$-$CF_3$, -$CF_2$-$CHF_2$, -$CF_2$-CHFCl, -$CF_2$-CHFBr ou -$CF_2$-CHF-$CF_3$;

$R_3$ représente -$OCH_3$ ou -$CH_3$;

$R_4$ représente -$OCH_3$, -$CH_3$, -$OC_2H_5$, -$CH_2$-O-$CH_3$ ou -$CH_2$-$CH_2$-$OCH_3$

et le noyau phényle n'est pas substitué ou est seulement substitué en position 2, 3, 5 ou 6 par rapport au groupe sulfonyle par un autre substituant du groupe F, Cl, Br ou -$CH_3$ ou lorsque simultanément X représente un oxygène. A un trifluorométhyle, $R_1$ un hydrogène, $R_2$ un alcényle en $C_2$ à $C_5$ en position 2, $R_3$ un hydrogène, un chlore, un brome, un méthyle, un éthyle, un alcoxy en $C_1$ à $C_3$, un trifluorométhyle, un méthylthio ou un méthoxyméthyle et $R_4$ représente un méthyle ou un méthoxy, ou lorsque simultanément X représente un oxygène, A un trifluorométhyle, $R_1$ un hydrogène, $R_2$ un chlore en position 2, $R_3$ un hydrogène, un chlore, un brome, un méthyle, un éthyle, un trifluorométhyle, un méthoxy, un méthoxyméthyle ou un méthoxyéthyle et le radical trifluorométhoxy prend la position 5 sur le noyau phényle.

2 - Agent selon la revendication 1, caractérisé en ce que Z représente un oxygène.

3 - Agent selon la revendication 1, caractérisé en ce que les radicaux $R_3$ et $R_4$ ensemble ne contiennent pas plus de 4 atomes de carbone.

4 - Agent selon la revendication 1 caractérisé en ce que Z représente un oxygène et m le nombre 1.

5 - Agent selon la revendication 1, caractérisé en ce que Z représente un oxygène et m le nombre 2.

6 - Agent selon la revendication 4, caractérisé en ce que le radical -X-A est en position 2 ou 3 par rapport au groupe sulfonyle.

7 - Agent selon la revendication 6, caractérisé en ce que le radical -X-A est en position 2.

8 - Agent selon la revendication 5, caractérisé en ce que les deux radicaux -X-A sont en position 2 et 5 par rapport au groupe sulfonyle.

9 - Agent selon la revendication 2, caractérisé en ce que seul un radical -X-A est présent en position 2 par rapport au radical sulfonyle et en ce que les radicaux $R_3$ et $R_4$ ensemble ne contiennent pas plus de 4 atomes de carbone.

10 - Agent selon la revendication 2, caractérisé en ce que deux radicaux -X-A- sont en position 2 et 5 par rapport au radical sulfonyle et en ce que les radicaux $R_3$ et $R_4$ ensemble ne contiennent pas plus de 4 atomes de carbone.

11 - Agent selon la revendication 10, caractérisé en ce qu A représente -$CHF_2$, -$CF_3$ ou -$CH_2$-$CH_2Cl$. 12 - Agent selon la revendication 9, caractérisé en ce que $R_1$ représente un hydrogène, et le radical $R_2$ est en position 5 ou 6 par rapport au groupe sulfonyle.

13 - Agent selon la revendication 12, caractérisé en ce que $R_2$ représente un hydrogène, un fluor, un nitro ou $COOR_6$.

14 - Agent selon la revendication 13, caractérisé en ce que X représente un oxygène ou un soufre.

15 - Agent selon la revendication 14, caractérisé en ce que $R_2$ représente un hydrogène, $R_3$ représente un chlore, un fluor, un méthyle, un chlorométhyle, un éthoxy, un i-propyloxy, un éthyle ou un méthoxy et $R_4$ représente un méthyle, un éthyle, un méthoxy ou un éthoxy.

16 - Agent selon la revendication 15, caractérisé en ce que X est un oxygène.

17 - Agent selon la revendication 15, caractérisé en ce que le radical A est incarné par -$CF_2G$, où G représente un hydrogène, F, Cl, Br, $CF_3$ ou $CH_3$.

18 - Agent selon la revendication 16, caractérisé en ce que G représente un chlore, un brome ou $CF_3$.

19 - Agent selon la revendication 15, caractérisé en ce que A représente -$CH_2F$, -$CH_2$-$CH_2F$ et -$CH_2$-$CH_2Cl$.

20 - Agent selon la revendication 1, caractérisé en ce que Z représente un soufre, X un oxygène ou un soufre et $R_3$ et $R_4$ représentent chacun indépendamment l'un de l'autre un alcoyle en $C_1$ à $C_3$, un alcoxy en $C_1$ à $C_3$ ou un alcoylthio en $C_1$ à $C_3$ avec ensemble au plus 4 atomes de carbone, A représente -$CHF_2$, -$CF_3$, -$C_2F_5$ et -$CF_3$, -$C_2F_5$ et -$CF_2$-$CHF_2$, le radical -X-A a la position 2 et m représente le nombre 1.

21 - Agent selon la revendication 1, caractérisé en ce qu'il contient la N-(2-difluorométhoxyphé-nyl-sulfonyl)-N'-(4-éthyl-6-méthoxy-1, 3, 5-triazin-2-yl)-urée.

22 - Agent selon la revendication 1, caractérisé en ce qu'il contient la N-(2-difluorométhoxyphé-nyl-sulfonyl)-N'-(4, 6-diéthoxy-1, 3, 5-triazin-2-yl)-urée.

23 - Agent selon la revendication 1, caractérisé en ce qu'il contient la N-(2-difluorométhoxyphényl-sulfonyl)-N-(4-chloro-6-méthyl-pyrimidin-2-yl)-urée.

24 - Agent selon la revendication 1, caractérisé en ce qu'il contient la N-(2-pentafluoréthoxyphé-nyl-sulfonyl)-N'-(4-méthoxy-6-méthyl-1, 3, 5-triazin-2-yl)-urée.

25 - Agent selon la revendication 1, caractérisé en ce qu'il contient la N-(2-difluorométhylthio-phénylsulfonyl)-N'-(4-chloro-6-méthoxy-pyrimidin-2-yl)-urée.

26 - Agent selon la revendication 1, caractérisé en ce qu'il contient la N-[2, 5-bis-(difluorométho-xy)-phénylsulfonyl]-N'-(4-méthoxy-6-méthyl-1, 3, 5-triazin-2-yl)-urée.

27 - Agent selon la revendication 1, caractérisé en ce qu'il contient la N-(2-difluorométhoxyphé-nyl-sulfonyl)-N'-(4-méthoxy-6-iso-propyloxy-1, 3, 5-tria-zin-2-yl)-urée.

28 - Agent selon la revendication 1, caractérisé en ce qu'il contient la N-(2-(difluorométhylthio-phénylsulfonyl)-N'-(4-éthyl-6-méthoxy-1, 3, 5-triazin-2-yl)-urée.

29 - Agent selon la revendication 1, caractérisé en ce qu'il contient la N-(2-difluorométhoxyphényl-sulfonyl)-N'-(4-chloro-6-méthoxy-pyrimidin-2-yl)-urée.

30 - Procédé de préparation des composés de formule I de la revendication 1, caractérisé en ce que a) ou bien on fait réagir un phénylsulfonamide de formule II

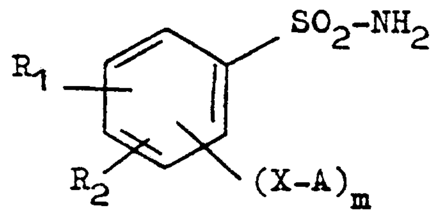

(II)

où A, $R_1$, $R_2$, X et m ont la signification donnée pour la formule I, en présence d'une base avec un N-pyrimidinyl- ou -triazinyl-carbamatede formule

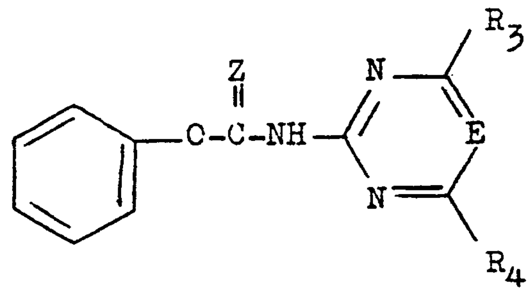

(III)

où E, $R_3$, $R_4$ et Z ont la siginfication donnée pour la formule I,

b) ou bien on fait réagir un phénylsulfonylisocyanate ou -isothiocyanate de formule IV

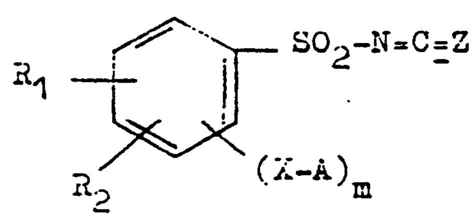

(IV)

où A, $R_1$, $R_2$, m, X et Z ont la signification donnée pour la formule I, éventuellement en présencé d'une base, avec une amine de formule V

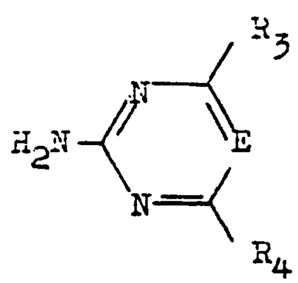

(V)

où E, $R_3$ et $R_4$ ont la signification donnée pour la formule X,

c) ou bien ou fait réagir des sulfonamides de formule II indiquée ci-dessus éventuellement en présence d'une base avec un isocyanate ou isothiocyanate de formule VI

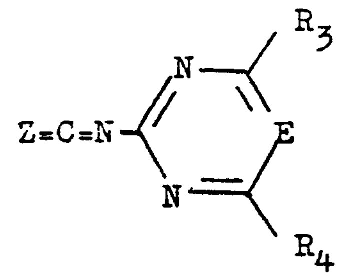

(VI)

où E, $R_3$, $R_4$ et Z ont la signification donnée pour la formule I,

d) ou bien on fait réagir un N-phénylsulfonylcarbamate de formule VII

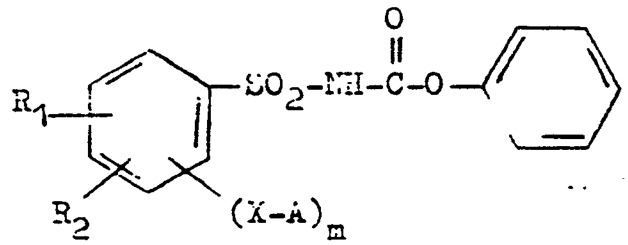

(VII)

où A, $R_1$, $R_2$, m et X ont la signification donnée pour la formule I, avec une amine de formule V donnée ci-dessus et éventuellement en ce qu'on le transforme en ses sels,

en faisant réagir une sulfonylurée de formule I avec une amine, un hydroxyde de métal alcalin ou de métal alcalino-terreux ou une base d'ammonium quaterinaire.

31 - Procédé de préparation des phénylsulfonamides de formule II

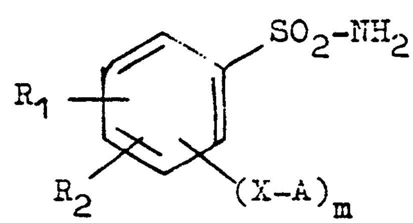

où A représente un radical halogénalcoyle en $C_1$ à $C_6$, à l'exception des radicaux $CHF_2$, $-CF_3$, $-CH_2-CF_3$, $-CF_2-CF_2H$, $-CF_2-CClFH$, $-CF_2-CClBrH$ et $-CF_2-CFH-CF_3$, et X, $R_1$ et $R_2$ ont les significations données pour la formule I, où A peut également représenter $-CHF_2$ ou $-CF_3$ lorsqu'on n'a pas simultanément $R_1$ représentant un hydrogène et $R_2$ un hydrogène, un alcényle en $C_2$ à $C_5$, un fluor, un chlore, un brome ou un méthyle, et caractérisé en ce qu'on prépare par diazotation et échange du groupe diazo avec de l'anhydride sulfureux en présence d'un catalyseur à partir d'une aniline correspondante le chlorure de phénylsulfonyle et en ce qu'on transforme celui-ci par réaction avec une solution d'hydroxyde d'ammonium pour donner le sulfonamide.

32 - Application des N-phénylsulfonyl-N'triazinyl- ou -pyrimidinyl-urées de formule I de la revendication 1 ou des agents qui les contiennent à la lutte contre la croissance végétale indésirable.

33 - Application des N-phénylsulfohyl-N'triaznyl- ou -pyrimidinyl-urées de formule I de la revendication 1 ou des agents qui les contiennent à l'inhibition de la croissance des plantes.

34 - Application des N-phénylsulfonyl-N'triazinyl- ou -pyrimidinyl-urées de formule I ou des agents qui les contiennent, selon la revendication 32, à la lutte sélective en pré ou en post-levée contre les mauvaises herbes dans les cultures de plantes utiles.

35 - Application des N-phénylsulfonyl-N'triazinyl- ou -pyrimidinyl-urées de formule I ou des agents qui les contiennent, selon la revendication 33, pour supprimer la croissance végétale au-delà du stade 2 feuilles, caractérisée en ce qu'on applique les matières actives en pré-levée.

36 - Agent selon la revendication 1, caractérisé en ce qu'il contient comme matière active la N-(2-pentafluoréthoxy-phénylsulfonyl)-N'-(4, 6-diméthoxy-1, 3, 5-triazin-2-yl)-urée.

37 - Agent selon la revendication 1, caractérisé en ce qu'il contient la N-[2-(2-chloréthoxy)-phénylsulfonyl]-N'-(4-méthoxy-6-méthyl-1, 3, 5-triazin-2-yl)-urée.

38 - Agent selon la revendication 1, caractérisé en ce qu'il contient la N-[2-(2-chloréthoxy)-phénylsulfonyl]-N'-(4, 6-diméthoxy-1, 3, 5-triazin-2-yl)-urée.

39 - Agent selon la revendication 1, caractérisé en ce qu'il contient la N-(2-pentafluoréthoxy-phénylsulfonyl)-N'-(4-méthoxy-6-méthyl-pyrimidin-2-yl)-urée.

40 - Agent selon la revendication 1, caractérisé en ce qu'il contient la N-(2-pentafluoréthoxy-phénylsulfonyl)-N'-(4, 6-diméthyl-pyrimidin-2-yl)-urée.